# EUROPÄISCHE PATENTANMELDUNG

(11) **EP 0 892 047 A2**
(43) Veröffentlichungstag der Anmeldung: **20.01.1999**
(21) Anmeldenummer: 98112470.4
(22) Anmeldetag: 06.07.1998
(51) Int. Cl.: C12N 15/12, C07K 14/47, A61K 48/00, G01N 33/50

(54) **Humanes und murines Semaphorin L**

(30) Priorität: 09.07.1997 DE 19729211; 11.02.1998 DE 19805371
(71) Anmelder: Hoechst Marion Roussel Deutschland GmbH, 65929 Frankfurt am Main (DE)
(72) Erfinder: Fleckenstein, Bernhard, Prof. Dr., 91369 Wiesenthau (DE); Ensser, Armin, Dr., 90419 Nürnberg (DE)

(57) **Zusammenfassung**

Humanes Semaphorin L(H-SemaL) und korrespondierende Semaphorine in anderen Spezies.

Gegenstand der Erfindung sind neuen Semaphorine, die sich durch eine besondere Domänenstruktur auszeichnen und deren Derivate, Nukleinsäuren (DNA, RNA, cDNA), die für diese Semaphorine kodieren und deren Derivate sowie die Verwendung derselben.

Gegenstand der vorliegenden Erfindung sind Semaphorine mit einer neuen, bisher nicht bekannten und nicht zu erwartenden Domänenstruktur, denen eine biochemische Funktion im Immunsystem zukommt (immunmodulierende Semaphorine). Die erfindungsgemäßen Semaphorine werden als Semaphorine vom Typ L (SemaL) bezeichnet. Sie enthalten ein N-terminales Signalpeptid, eine charakteristische Sema-Domäne und im C-terminalen Bereich des Proteins eine Immunglobulin-ähnliche Domäne und eine hydrophobe Domäne, die eine potentielle Transmembrandomäne darstellt.

## Beschreibung

Humanes Semaphorin L(H-SemaL) und korrespondierende Semaphorine in anderen Spezies.

Gegenstand der Erfindung sind neue Semaphorine, die sich durch eine besondere Domänenstruktur auszeichnen und deren Derivate, Nukleinsäuren (DNA, RNA, cDNA), die für diese Semaphorine kodieren und deren Derivate sowie die Herstellung und Verwendung derselben.

Semaphorine wurden erstmals von Koldokin {Kolodkin et al. (1993) Cell 75:1389-1399} als Mitglieder einer konservierten Genfamilie beschrieben.

Inzwischen wurden die Gene bzw. Teile der Gene weiterer Semaphorine kloniert und teilweise charakterisiert. Bisher waren insgesamt 5 humane (H-Sema III, H-Sema V, H-Sema IV, H-SemaB und H-SemaE) {Koldokin et al. (1993); Roche et al. (1996) Onkogene 12:1289-1297; Sekido et al. (1996) Proc. Natl. Acad. Sci. USA 93:4120-4125; Xiang et al. (1996) Genomics 32:39-48; Hall et al. (1996) Proc. Natl. Acad. Sci. USA 39:11780-11785; Yamada et al. (1997) (GenBank Zuordnungs-Nr. AB000220)}, 8 rnurine (Gene der Maus; M-Sema A bis M-SemaH) {Püschel et al. (1995) Neuron 14:941-948; Messerschmidt et al. (1995) Neuron 14:949-959; Inigaki et al. (1995) FEBS Letters 370:269-272; Adams et al. (1996) Mech. Dev. 57:33-45; Christensen et al. (1996) (Genbank Zuordnungs-Nr. Z80941, Z93948)}, 5 galline (Huhn) (Collapsin-1 bis -5) {Luo et al. (1993); Luo et al. (1995) Neuron 14:1131-1140}, und Gene von Ratte (R-Sema III) {Giger et al. (1996) J. Comp. Neurol. 375:378-392}, Zebrafisch, Insekten (Fruchtfliege (Drosophila melanogaster: D-Sema I und D-Sema II), Käfer (Tribolium confusum: T-Sema I), Grasshüpfer (Schistocerca americana: G-Sema I)) {Kolodkin et al. (1993)}, und Nematoden (C.elegans: Ce-Sema) {Roy et al. (1994) (GenBank Zuordnungs-Nr. U15667)} bekannt. Weiterhin besitzen zwei Poxviren (Vaccinia (ORF-A39) und Variola (ORFA39-homolog)) {Kolodkin et al. (1993)} sowie der Alcelaphine Herpesvirus Typ 1 (AHV-1) (AHV-Sema) {Ensser und Fleckenstein (1995) Gen. Virol. 76:1063-1067} zu Semaphorinen homologe Gene.

Einen Überblick über die bisher identifizierten Semaphorine in verschiedenen Spezies gibt Tabelle 1. In Tabelle 1 sind die Namen der Semaphorine (Spalte 1), die verwendeten Synonyme (Spalte 2), die Spezies aus der das jeweilige Semaphorin isoliert wurde (Sparte 3) sowie, soweit bekannt, Daten zur Domänenstruktur des kodierten Proteins und zur chromosomalen Lokalisation (Spalte 4 in Tabelle 1), die Zuordnungsnummer unter der die Sequenz des Gens in Gendatenbanken, z.B. in einer EST (expressed sequence tags) Datenbank, EMBL (European Molecular Biology Laboratory, Heidelberg) oder NCBI (National Center for Biotechnology Information, Maryland, USA) gespeichert ist und die entsprechende Referenz unter der diese Daten publiziert wurden (Spalte 5 in Tabelle 1), angegeben.

Alle Genprodukte (kodierte Semaphorine) der bisher bekannten Semaphorin Gene weisen ein N-terminales Signalpeptid auf, an dessen C-terminalem Ende sich eine charakteristische Sema-Domäne mit einer Länge von etwa 450 bis 500 Aminosäuren befindet Innerhalb der Sema-Domäne finden sich stark konservierte Aminosäuremotive und eine Anzahl hochkonservierter Cysteinreste. Die Genprodukte (Semaphorine) unterscheiden sich in den auf die Sema-Domäne folgenden C-terminalen Sequenzen, die aus einer oder mehreren Domänen aufgebaut sind. Sie weisen beispielsweise in diesen C-terminalen Aminosäuresequenzen Transmembrandomänen (TM), Immunoglobulin-ähnliche Domänen (Ig) (konstanter Teil des Immunoglobulins), zytoplasmatische Sequenzen (CP), Prozessierungssignale (P) (beispielsweise mit der Konsensussequenz (RXR), wobei R für die Aminosäure Arginin und X für eine beliebige Aminosäure steht) und/oder hydrophile C-Termini (HPC) auf. Auf der Basis der unterschiedlichen Domänenstruktur im C-Terminus lassen sich die bisher bekannten Semaphorine in 5 verschiedene Untergruppen einteilen (I bis V):
- I: Sezerniert, ohne weitere Domäne (z.B. ORF-A49)
- II Ig: Sezerniert (ohne Transmembrandomäne) (z.B. AHV-Sema)
- III Ig, TM, CP: Membranverankert mit zytoplasmatischer Sequenz (z.B. CD100)
- IV Ig, (P), HPC: Sezerniert mit hydrophilem C-Terminus (z.B. H-Sema III, M-SemaD, Collapsin-1)
- V Ig, TM, CP: Membranverankert mit C-terminalem 7 Thrombospondin-Motiv (z.B. M-SemaF und G)

Ein Rezeptor oder extrazellulärer Ligand für Semaphorine wurde bisher nicht beschrieben. Im Zusamrnenhang mit Semaphorin-vermittelten Effekten wurden intrazelluläre, heterotrimere GTP-bindende Proteinkomplexe beschrieben. Als ein Bestandteil dieser Proteinkomplexe wurden bei Hühnern sogenannte CRMP-Proteine (Collapsin response mediator protein) identifiziert, welche vermutlich Bestandteil der Semaphorin-induzierten intrazellulären Signalkaskade sind (Goshima et al. (1995) Nalure 376: 509-514). Das CRMP62 beispielsweise besitz: Homologie zu unc-33, einem für das gerichtete Axonwachstum essentiellen Nemaloden-Protein. Ein humanes Protein mit 98% Aminosäure-Identität zu CRMP62 ist ebenfalls bekannt (Hamajima et al. (1996) Gene 180: 157-163. In Ratten wurden ebenfalls mehrere CRMP-verwandte Gene beschrieben (Wang et al. (1996) Neurosci. 16: 6197-6207.

Die sezernierten oder transmembrahen Semaphorine vermitteln repulsive Signale für wachsende Nervenknospen. Sie spielen eine Rolle bei der Entwicklung des zentralen Nervensystems (ZNS) und werden vor allem in Muskel- und Nervengewebe exprimiert (Koldokin et al. (1993); Luo et al. (1993) Cell 75:217-227.

Außer im ZNS konnte eine deutliche Expression von M-SemaG auch auf Zellen des lymphatischen und hämatopoetischen Systems beobachtet werden, im Gegensatz zum nahe verwandten M-SemaF {Furuyima et al. (1996) J. Biol. Chem. 271: 33376-33381}.

Kürzlich wurden zwei weitere humane Semaphorine identifiziert, H-Sema IV und H-Sema V und zwar in einer Region auf Chromosom 3p21.3, deren Deletion mit verschiedenen Formen von Bronchialkarzinomen assoziiert ist H-Sema IV {Roche et al. (1996), Xiang et al. (1996), Sekido et al. (1996)} ist auf Aminosäureebene etwa zu 50% identisch mit M-SemaE, während H-Sema V {Sekido et al. (1996)} das direkte Homolog zu M-Sema-A ist (86% Aminosäureidentität). Da diese Gene (H-Sema IV und V) im Rahmen von DNA-Sequenzierungs-Projekten der deletierten 3p21.3 Loci gefunden wurden, ist die komplexe Intron-Exon-Struktur dieser beiden Gene bekannt. Die beiden Gene werden in verschiedenen neuronalen und nicht neuronalen Geweben exprimiert.

Ebenfalls erst vor kurzem wurde das zelluläre Oberflächenmolekül CD100 (human), exprimiert und induziert auf aktivierten T-Zellen, als Semaphorin identifiziert (ebenfalls in Tabelle 1 aufgeführt). Es unterstützt die Interaktion mit B-Zellen über den Rezeptor CD40 und den entsprechenden Liganden CD40L. CD100 ist ein membranverankertes Glykoprotein-Dimer von 150kd (Kilodalton). Es wurde eine Assoziation des intrazytoplasmatischen C-Terminus von CD100 mit einer noch unbekannten Kinase beschrieben {Hall et al. (1996)}. Damit ist CD100 das erste und bisher einzige Semaphorin, dessen Expression in Zellen des Immunsystems nachgewiesen werden konnte.

Unter der Fragestellung "Transformierende Gene von Rhadinoviren" wurde das komplette Genom des Alcelaphinen Herpesvirus Typ 1 (AHV-1) kloniert und sequenziert {Ensser et al. (1995)}. AHV-1 ist der Erreger des bösartigen Katarrhalfiebers, einer mit einem lymphoproliferativen Syndrom einhergehenden, meist fatalen Erkrankung verschiedener Wiederkäuer. Bei der Analyse wurde an einem Ende des viralen Genoms ein offener Leserahmen mit entfernter, aber signifikanter Homologie zu einem Gen von Vacciniavirus (ORF-A39 entspricht VAC-A39 in Ensser et al. (1995)) J. Gen. Virol. 76:1063-1067), welches der Genfamilie der Semaphorine zugerechnet wurde, gefunden. Während das AHV-1 Semaphorin (AHV-Sema) eine gut konservierte Semaphorin-Struktur besitzt, sind die Poxvirus-Gene (ORF-A39 und ORF-A39-homolog, siehe Tabelle 1) C-terminal verkürzt, d.h. bei ihnen ist die konservierte Sema-Domäne nur unvollständig vorhanden.

Ein Datenbankvergleich des gefundenen AHV-Sema mit dbEST (EST (expressed sequence tags)-Datenbank (db)) lieferte jeweils 2 EST-Sequenzen von 2 unabhängigen cDNA-Klonen aus humaner Plazenta (Zuordnungsnummern H02902, H03806 (Klon 151129), Zuordnungsnummern R33439 und R33537 (Klon 135941)). Diese wiesen deutlich höhere Homologie zum AHV-1 Semaphorin auf, als zu den bis dahin beschriebenen neuronalen Semaphorinen.

Gegenstand der vorliegenden Erfindung sind Semaphorine mit einer neuen, bisher nicht bekannten und nicht zu erwartenden Domänenstruktur, denen eine biochemische Funktion im Immunsystem zukommt (immunmodulierende Semaphorine). Die erfindungsgemäßen Semaphorine werden als Semaphorine vom Typ L (SemaL) bezeichnet. Sie enthalten ein N-terminales Signalpeptid, eine charakteristische Sema-Domäne und im C-terminalen Bereich des Proteins eine Immunglobulinähnliche Domäne und eine hydrophobe Domäne, die eine potentielle Transmembrandomäne darstellt

Die Aminosäure-Sequenz des Signalpeptids kann weniger als 70, vorzugsweise weniger als 60 Aminosäuren und mehr als 20, vorzugsweise mehr als 30 Aminosäuren aufweisen, besonders bevorzugt ist eine Länge von etwa 40 bis 50 Aminosäuren. In einer speziellen Ausführungsform der Erfindung hat das Signalpeptid eine Länge von 44 Aminosäuren, d.h. zwischen den Aminosäuren 44 und 45 befindet sich eine Spaltstelle für eine Signalpeptidase.

Die Sema-Domäne kann eine Länge von 300 bis 700 oder mehr, vorzugsweise von etwa 400 bis 600 Aminosäuren aufweisen. Bevorzugt sind Sema-Domänen mit einer Länge von 450 bis 550 Aminosäuren, vorzugsweise von etwa 500 Aminosäuren. In einer besonderen Ausführungsform der Erfindung schließt sich die Sema-Domäne an das Signalpeptid an, wobei sich die Sema-Domäne vorzugsweise bis zur Aminosäure 545 erstreckt.

Die Immunglobulin-ähnliche Domäne kann eine Länge von etwa 30 bis 110 oder mehr Aminosäuren aufweisen, bevorzugt sind Längen zwischen 50 und 90, besonders bevorzugt etwa 70 Aminosäuren.

Die Transrnembrandomäne kann eine Länge von etwa 10 bis 35, vorzugsweise von etwa 15 bis 30, besonders bevorzugt von etwa 20 bis 25 Aminosäuren aufweisen.

Gegenstand der Erfindung sind Semaphorine vom Typ L aus verschieden Spezies, insbesondere aus Wirbeltieren, beispielsweise aus Vögeln und/oder Fischen, vorzugsweise aus Säugetieren, beispielsweise aus Primaten, Ratte, Kanninchen, Hund, Katze, Schaf, Ziege, Kuh, Pferd, Schwein, besonders bevorzugt aus Mensch und Maus. Gegenstand der Erfindung sind auch entsprechende Semaphorine aus Mikroorganismen, insbesondere aus pathogenen Mikroorganismen, beispielsweise aus Bakterien, Hefen und/oder Viren, z.B. aus Retroviren, insbesondere aus humanpathogenen Mikroorganismen.

Eine Ausführungsform der Erfindung ist ein entsprechendes humanes Semaphorin (H-SemaL), das ein Signalpeptid, eine Sema-Domäne, eine Immunglobulin-ähnliche Domäne und eine Transmembrandomäne aufweist Eine spezielle Ausführungsform ist das Semaphorin, das durch die Aminosäuresequenz gemäß Tabelle 4 gegeben ist.

Eine weitere Ausführungsform der Erfindung sind korrespondierende Semaphorine in anderen Spezies, die im Bereich der Sema-Domäne eine Aminosäureidentität größer als 40 %, vorzugsweise größer 50 %, besonders bevorzugt größer 60 % im Bezug auf die Sema-Domäne von H-SemaL (Aminosäuren 45 bis 545 der Sequenz in Tabelle 4) aufweisen. Aus näher verwandten Spezies (z.B. Primaten, Maus) können die korrespondierenden Semaphorine durchaus Aminosäureidentitäten größer als 70%, vorzugsweise größer als 80 %, besonders bevorzugt größer als 90 % aufweisen. Prozentuale Homologien können beispielsweise mit dem Programm GAP (GCG Programm-Paket, Genetic Computer Group (1991)) bestimmt bzw. berechnet werden.

Eine derartige Ausführungsform der Erfindung ist ein korrespondierendes Semaphorin der Maus (murines Semaphorin (M-SemaL)). Beispielsweise enthält dieses die partielle Aminosäuresequenz gemäß Tabelle 5 (murines Semaphorin (M-SemaL)).

Die Erfindung betrifft auch korrespondierende Semaphorine, die eine Aminosäureidentität (über die Gesamtlänge der Aminosäuresequenz des Protein betrachtet) von nur etwa 15 bis 20% bei wenig verwandten Spezies (phylogenetisch weit voneinander entfernt), vorzugsweise 25 bis 30%, besonders bevorzugt 35 bis 40 % oder eine höhere Identität im Bezug auf die gesamte Aminosäuresequenz von H-SemaL gemäß Tabelle 4 aufweisen.

Die Gene, die für Semaphorine von Typ L kodieren, weisen eine komplexe Exon-Intron-Struktur auf. Diese Gene können beispielsweise zwischen 10 und 20 Exons, vorzugsweise etwa 11 bis 18, besonders bevorzugt 12 bis 16 Exons und eine entsprechende Anzahl von Introns aufweisen. Sie können aber auch die gleiche Anzahl Exons und Introns aufweisen wie das Gen von H-SemaL (13 oder 15 Exons, vorzugsweise 14 Exons). Eine besondere Ausführungsform der Erfindung betrifft das Gen von H-SemaL. Dieses Gen hat vorzugsweise eine Länge von 8888 bis 10000 oder mehr Nukleotiden. Das humane Semaphorin-Gen enthält vorzugsweise die Nukleotid-Sequenz, die in Tabelle 14 gegeben ist oder die Nukleotidsequenz, die in der Datenbank GenBank® unter der Zugangsnummer AF030697 hinterlegt wurde. Diese Nukleotidsequenzen enthalten mindestens 13 Introns. Darüber hinaus weist das humane Semaphorin-Gen am 5'-Ende einen zusätzlichen Sequenzbereich auf. Dieser Bereich enthält gegebenfalls weitere kodierende und nichkodierende Sequenzen, z.B. ein oder zwei weitere Introns bzw. Exons..

Die Versuche zur chromosomalen Lokalisation des humanen Semaphorins vom Typ L ergaben, daß das entsprechende Gen an Position 15q22.3-23 lokalisiert ist Entsprechend wurde das Gen für M-SemaL an Position 9A3.3-B lokalisiert.

Als Folge der komplexen Intron-Exon Struktur kann das Primärtranskript der Semaphorin mRNA unterschiedlich gespliced werden, wodurch unterschiedliche Splicevarianten der Semaphorine entstehen. Die aus diesen Splicevarianten translatierten Proteine sind Derivate der erfindungsgemäßen Semaphorine. Sie entsprechen in ihrer Aminosäuresequenz und auch weitgehend in ihrer Domänenstruktur den beschriebenen, erfindungsgemäßen Semaphorinen vom Typ L, sind jedoch gegebenenfalls gegenüber diesen verkürzt. Beispielsweise können Splicevarianten, denen die Transmembrandomäne ganz oder teilweise fehlt, gebildet werden. Ein Semaphorin-Derivat, welches keine oder keine vollständige Transmembrandomäne, aber ein Signalpeptid enthält, kann sezerniert werden und auf diese Weise außerhalb der Zelle lokal oder auch über größere Entfernungen wirken, beispielsweise auf andere Zellen. Eine andere Splicevariante kann beispielsweise keine Sequenz mehr enthalten, die für ein Signalpeptid kodiert und gegebenfalls auch keine Sequenz, die für eine für hydrophobe Aminosäuresequenz kodiert, die eine potentielle Transmembrandomäne darstellt. Eine Folge wäre, daß dieses Semaphorin-Derivat weder in die Membran eingebaut, noch sezerniert wird (es sei denn über sekretorische Vesikel). Ein solches Semaphorin-Derivat kann an intrazellulären Prozessen, beispielsweise an Signaltransduktionsprozessen beteiligt sein. Auf diese Weise können mit dem gleichen Grundmoleküle (Semaphorine vom Typ L) und den davon abgeleiteten Derivaten (beispielsweise Splicevarianten) vielfälltige intra- und extrazelluläre Prozesse reguliert und/oder aufeinander abgestimmt werden.

Eine besondere Ausführungsform der Erfindung betrifft Semaphorin-Derivate, die sich von den erfindungsgemäßen Semaphorinen vom Typ L ableiten, die aber keine oder keine vollständige Transmembrandomäne enthalten.

Eine weitere Ausführungsform der Erfindung betrifft Semaphorin-Derivate, die sich von den erfindungsgemäßen Semaphorinen vom Typ L ableiten, die aber kein Signalpeptid enthalten.

Das Signalpeptid kann auch posttranslational abgespalten werden. Dadurch wird ein membranständiges (mit TM-Domäne) oder ein sezerniertes (Splicevariante ohne TM-Domäne) Semaphorin-Derivat mit verkürzter Domänenstruktur gebildet. Ein auf diese Weise posttranslational prozessiertes Semaphorin-Derivat enthält nur noch Sema-Domäne, Ig-Domäne und gegebenfalls Transmembrandomäne. Eine Signalpeptidschnittstelle kann beispielsweise direkt am Ende des Signalpeptids liegen, sie kann z.B. 40 bis 50 Aminosäuren oder weiter vom Aminoterminus entfernt lokalisiert sein.

Ein "verkürztes" (d.h. weniger Domänen enthaltendes) Semaphorin L-Derivat ist von anderen Semaphorinen, die sich nicht von den Semaphorinen vom Typ L ableiten, dadurch zu unterscheiden, daß es eine sehr große (> 90 %) Aminosäureidentität oder eine identische Aminosäuresequenz mit den Semaphorinen vom Typ L in den vorhandenen Domänen aufweist.

Die erfindungsgemäßen Semaphorine können auch in anderer Weise posttranslational modifiziert sein. Beispielsweise können sie ein-, zwei-, drei-, vier- fünf, sechs-, sieben-, acht-, neun- zehn- oder mehrfach glykosyliert (N- und/oder O-glykosyliert) vorliegen. Die Aminosäuresequenzen der Semaphorine können dann ebenso viele oder mehr Konsensussequenzen für potentielle Glykosylierungsstellen aufweisen, vorzugsweise fünf derartige Stellen. Eine Ausführungsform der Erfindung betrifft Semaphorine, bei denen die Glykosylierungsstellen an Positionen lokalisiert sind, die den Positionen 105, 157, 258, 330 und 602 der H-SemaL Aminosäuresequenz entsprechen (Tabelle 4).

Darüber hinaus können die Semaphorine in Form ihrer phosphorylierten Derivate vorliegen. Semaphorine können die Substrate unterschiedlicher Kinasen sein, beispielsweise können die Aminosäuresequenzen Konsensussequenzen für Protein Kinase C, Tyrosin Kinase und/oder Kreatin Kinasen aufweisen. Weiterhin können die Aminosäuresequenzen der Semaphorine Konsensussequenzen für potentielle Myristylierungsstellen aufweisen. An diesen Stellen können entsprechende Semaphorin-Derivate mit Myristinsäure verestert sein.

Die erfindungsgemäßen Semaphorine vom Typ L und deren Derivate können in Form von Monomeren, Dimeren und/oder Multimeren vorliegen, beispielsweise können zwei oder mehr Semaphorine bzw. deren Derivate über intermolekulare Disulfidbrücken miteinander verbunden sein. Darüber hinaus können sich intramolekulare Disulfidbrücken ausbilden.

Derivate der erfindungsgemäßen Semaphorine sind weiterhin Fusionsproteine. Ein solches Fusionsprotein enthält einerseits ein Semaphorin vom Typ L oder Teile desselben und darüber hinaus ein weiteres Peptid oder Protein bzw. ein Teil desselben. Peptide oder Proteine bzw. Teile derselben können z.B. Epitope-Tags (z.B. His-Tag (6xHistidin), Myc-Tag, flu-Tag), die z.B. zur Aufreinigung der Fusionsproteine verwendet werden können, oder solche, die zur Markierung der Fusionsproteine verwendet werden können, z.B. GFP (green fluorescent protein), sein. Beispiele für Derivate der Semaphorine vom Typ L sind z. B. durch die in den Beispielen beschriebenen Konstrukte gegeben. Die Sequenzen dieser Konstrukte können aus den Tabellen 7 bis 15 gegebenenfalls unter Berücksichtigung der Annotationen zu den Plasmiden entnommen werden.

Weiterer Gegenstand der Erfindung sind Nukleinsäure-Sequenzen, vorzugsweise DNA- und RNA-Sequenzen, die für die erfindungsgemäßen Semaphorine vom Typ L und/oder deren Derivate kodieren, beispielsweise die entsprechenden Gene, die unterschiedlichen Splicevarianten der mRNA, die dazu korrespondierenden cDNAs sowie deren Derivate, z.B. Salze der DNA bzw. RNA Derivate im Sinne der Erfindungen sind Sequenzen oder Teile davon, die z.B. mit molekularbiologischen Methoden verändert und an die jeweiligen Anforderungen angepaßt werden, beispielsweise verkürzte Gene oder Teile der Gene (z. B. Promotorsequenzen, Terminatorsequenzen), cDNAs oder Chimäre derselben, Konstrukte für Expressionen und Klonierungen und deren Salze.

Eine Ausführungsforrn betrifft die genomischen Sequenzen (Gene) der Semaphorine vom Typ L. Die Erfindung betrifft die Intron- und Exon-Sequenzen und genregulatorische Sequenzen, beispielsweise Promotor-, Enhancer- und Silencer-Sequenzen.

Ein Gegenstand dieser Ausführungsform ist das Gen von H-SemaL bzw. dessen Derivate. Ein Gegenstand der Erfindung ist ein Gen, das die Nukleotidsequenz, die in Tabelle 14 gegeben ist, enthält. Ein weiterer Gegenstand der Erfindung ist das Gen, das die Nukleotidsequenz, die in der Datenbank GenBank® unter der Zugangsnummer AF030697 hinterlegt ist, enthält.

Ein weiterer Gegenstand dieser Ausführungsform ist das Gen von M-SemaL bzw. dessen Derivate.

Ein weiterer Gegenstand der Erfindung ist die cDNA von H-SemaL oder deren Derivate (z. B. Teile der cDNA). Eine besondere Ausführungsform ist die cDNA von H-SemaL gemäß der Nukleotidsequenz in Tabelle 2. Ein weiterer Gegenstand der Erfindung ist die cDNA von H-SemaL, die in der Datenbank GenBank® unter der Zugangsnummer AF030698 hinterlegt ist Gegenstand der Erfindung sind auch die zu diesen cDNAs korrespondierenden mRNAs bzw. Teile derselben.

Ein weiterer Gegenstand der Erfindung ist die cDNA von M-SemaL oder deren Derivate (z. B. Teile der cDNA). Eine besondere Ausführungsform ist die partielle cDNA-Sequenz von M-SemaL gemäß Tabelle 3 sowie cDNA-Sequenzen, die diese partielle cDNA-Sequenz enthalten. Eine weitere Ausführungsform der Erfindung betrifft die cDNA von M-SemaL, die in der Datenbank GenBank unter der Zugangsnummer AF030699 hinterlegt ist. Gegenstand der Erfindung sind auch die zu diesen cDNAs korrespondierenden mRNAs bzw. Teile derselben.

Die Erfindung beinhaltet auch Allele und/oder individuelle Ausprägungsformen der Gene/mRNAs/cDNAs, die sich nur geringfügig von den hier beschriebenen Semaphorin-Sequenzen unterscheiden und für ein identisches oder nur geringfügig verändertes Protein (Abweichung in der Aminosäure-Sequenz kleiner oder gleich 10%) kodieren (weiteres Beispiel für Derivate). Weitere Beispiele für die Derivate sind durch die in den Beispielen angegebenen Konstrukte gegeben. Die Sequenzen dieser Konstrukte sind in den Tabellen 7 bis 14 dargestellt und können unter Berücksichtigung der Annotation für Plasmide interpretiert werden.

Ein weiterer Gegenstand der Erfindung sind Plasmide, die DNA, die für die Semaphorine des Typs L bzw. deren Derivate kodiert, enthalten. Solche Plasmide können beispielsweise Plasmide mit hohen Replikationsraten sein, die für eine Amplifikation der DNA, z.B. in E. coli geeignet sind.

Eine spezielle Ausführungsform sind Expressionsplasmide, mit denen die Semaphorine bzw. Teile davon oder deren Derivate in prokaryoten und/oder eukaryoten Expressions-Systemen exprimiert werden können. Es sind sowohl Expressionsplasmide die konstitutive als auch solche, die induzierbare Promotoren enthalten, geeignet.

Gegenstand der Erfindung sind auch Verfahren zur Herstellung von Nukleinsäuren₁ die für Semaphorine vom Typ L oder Derivate derselben kodieren.
Beispielsweise können diese Nukleinsäuren, z B. DNA oder RNA auf chemischem Weg synthetisiert werden. Insbesondere können diese Nukleinsäuren, z. B. die entsprechenden Gene oder cDNAs bzw. Teile derselben mit der PCR unter Verwendung von spezifischen Primern und geeignetem Ausgangsmaterial als Template (z. B. cDNA aus einem geeigneten Gewebe oder genomische DNA) amplifiziert werden.
Ein konkretes Verfahren zur Herstellung von Semophorin L cDNA bzw. des H SemaL Gens ist in den Beispielen beschrieben.

Die Erfindung betrifft auch Verfahren zur Herstellung der Semaphorine vom Typ L. Beispielsweise kann ein Semaphorin L oder ein Derivat desselben dadurch hergestellt werden, daß eine entsprechende Nukleinsäure-Sequenz, die für ein Semaphorin vom Typ L oder ein Derivat desselben kodiert, in einen Expressionsvektor kloniert und mit diesem rekombinanten Vektor eine geeignete Zelle transformiert wird. Es können beispielsweise prokaryote oder eukaryote Zellen verwendet werden. Die Semaphorine vom Typ L oder deren Derivate können gegebenenfalls auch auf chemischem Weg hergestellt werden.

Darüber hinaus können die Semaphorine vom Typ L bzw. deren Derivate als Fusionsproteine exprimiert werden, beispielsweise mit Proteinen oder Peptiden, die einen Nachweis des exprimierten Fusionsproteins erlauben, z.B. als Fusionsprotein mit GFP (green fluorescent protein). Die Semaphorine können auch als Fusionsproteine mit einem, zwei, drei oder mehreren Epitop-Tags, beispielsweise mit Myc- und/oder His-(6xHistidin) und/oder flu-Tags exprimiert werden. Entsprechend können Plasmide verwendet oder hergestellt werden, die DNA-Sequenzen enthalten, die für diese Fusionsproteine kodieren. Beispielsweise können Semaphorin kodierende Sequenzen in Plasmide kloniert werden, die DNA-Sequenzen enthalten, die für GFP und/oder Epitop-Tags, z.B. Myc-Tag, His-Tag, flu-Tag kodieren. Konkrete Beispiele dafür sind durch die Beispiele und die in den Tabellen aufgeführten Sequenzen ggfs. unter Zuhilfenahme der Annotation zu den Plasmiden gegeben.

Ein weiterer Gegenstand der Erfindung sind Antikörper, die spezifisch die Semaphorine vom Typ L, deren Derivate oder Teile davon binden bzw. erkennen. Dies können beispielsweise polyklonale oder monoklonale Antikörper, die z.B. in Maus, Kaninchen, Ziege, Schaf, Huhn usw. hergestellt werden können, sein.

Eine besondere Ausführungsforrn dieses Gegenstandes der Erfindung sind Antikörper, die gegen die Epitope die den Aminosäuresequenzen von Position 179 bis 378 bzw. 480 bis 666 der H-SemaL Sequenz gemäß Tabelle 4 entsprechen, gerichtet sind. Die Erfindung betrifft auch ein Verfahren zur Herstellung von spezifischen Anti-Semaphorin L Antikörpern, wobei für die Herstellung Antigene verwendet werden, die genannten Epitope enthalten.

Die Erfindung betrifft auch Verfahren zur Herstellung der Antikörper, vorzugsweise wird dazu ein Fusionsprotein, bestehend aus einem charakteristischen Semaphorin Epitop und einem Epilop-Tag, welches für die anschließende Aufreinigung des rekombinanten Fusionsproteins verwendet werden kann. Das aufgereinigte Fusionsprotein kann anschließend für die Immunisierung verwendet werden. Zur Herstellung des rekombinanten Fusionsproteins wird ein entsprechender rekombinanter Expressionsvektor hergestellt und mit diesem eine geeignete Zelle transformiert. Aus dieser Zelle kann das rekombinante Fusionsprotein isoliert werden. Die Durchführung kann beispielsweise wie in Beispiel 8 beschrieben erfolgen.

Diese Antikörper können beispielsweise zur Aufreinigung der entsprechenden Semaphorine, z.B. von H-SemaL und dessen Derivaten z.B. über Affinitätssäulen oder zum immunologischen Nachweis der Proteine, z.B. im ELISA, im Western-Blot und/oder in der Immunhistochemie verwendet werden. Die Antikörper können auch zur Analyse der Expression von H-SemaL, z. B. in verschiedenen Zelltypen bzw. Zellinien verwendet werden.

Die cDNA von H-SemaL hat eine Länge von 2636 Nukleotiden (Tabelle 2). Das Genprodukt der H-SemaL-cDNA hat eine Länge von etwa 666 Aminosäuren (Tabelle 4) und weist die typische Domänenstruktur eines Semaphorins vom Typ L auf. Das Genprodukt weist ein N-terminales Signalpeptid (Aminosäuren 1 bis 44), Sema-Domäne (Aminosäure 45 bis ungefähr Aminosäure 545) und Ig (Immunglobulin)-Domäne (etwa Aminosäuren 550 bis 620) sowie am C-terminalen Ende eine hydrophobe Aminosäuresequenz auf, die eine potentielle Transmembrandomäne darstellt. Diese Domänen-Struktur wurde bisher für Semaphorine noch nie beschrieben. Es handelt sich um ein membranassoziiertes, wahrscheinlich an der Zelloberfläche lokalisiertes Glykoprotein einer neuen Untergruppe. Aufgrund dieser bisher nicht bekannten Domänenstruktur können die Semaphorine nun in VI Untergruppen eingeteilt werden:
- I: Sezerniert, ohne weitere Domäne (z.B. ORF-A49)
- II Ig: Sezerniert (ohne Transmembrandomäne) (z.B. AHV-Sema)
- III Ig, TM, CP: Membranverankert mit zytoplasmatischer Sequenz (z.B. CD100)
- IV Ig, (P), HPC: Sezerniert mit hydrophilem C-Terminus (z.B. H-Sema III, M-SemaD, Collapsin-1)
- V Ig, TM, CP: Membranverankert mit C-terminalem 7 Thrombospondin-Motiv (z.B. M-Sema-F und G)
- VI Ig,: TM Membranverankert (z.B. H-SemaL, M-SemaL)

Die nichtglykosylierte, nichtprozessierte Form von H-SemaL hat ein errechnetes Molekulargwicht von etwa 74,8 kD (74823 Dalton) (berechnet mit PeptideSort, GCG-Programm-Paket). Der Isoelektrische Punkt berechnet sich zu pH= 7.56.
Eine mögliche Signalpeptid-Schnittstelle liegt zwischen den Aminosäuren 44 und 45 (Tabelle 3; berechnet mit SignalP (http.//www.cbs.dtu.dk/services/Signal P), einem auf neuronalen Netzwerken basierenden Programm zur Analyse von Signalsequenzen {Nielsen H. et al. (1997) Protein Engineering 10:1-6}). Dies ergibt für das prozessierte Protein (ohne Signalpeptid) ein Molekulargewicht (MW) von 70,3 kD (70323 Dalton) und einen Isoelektrischen Punkt von pH=7.01.

Die genomische Struktur ist ebenfalls weitgehend geklärt. Das H-SemaL-Gen weist 13 oder 15 oder mehr Exons, vorzugsweise 14 Exons und 12 oder 14 Introns, vorzugsweise 13 Introns auf. Aufgrund dieser komplexen Exon-Intron-Struktur sind unterschiedliche Splice-Varianten möglich. Die mRNA des transkribierten H-SemaL-Gens findet sich im Northern-Blot vor allem in Placenta, Keimdrüsen, Thymus und Milz. Es wurde keine mRNA in neuronalem Gewebe oder in Muskelgewebe nachgewiesen. Ein Hinweis auf eine spezifisch regulierte Expression in Endothelzellen liegt vor.

Durch alternatives Splicing können auch Formen von H-SemaL mit intrazytoplasmatischen Sequenzen entstehen, die eine Rolle in der intrazellulären Signaltransduktion spielen, ähnlich wie z.B. bei CD100.
Ebenfalls möglich wären durch alternatives Splicing entstehende, sezernierte Formen von H-SemaL, analog zum viralen AHV-Sema.
Nukleotid- und Aminosäuresequenzanalysen wurden mit Hilfe des GCG Programm-Pakets (Genetics Computer Group (1991) Program manual for the GCG package, version 7, 575 Science Drive, Wisconsin, USA 53711), FASTA (Pearson und Lipman (1988) Proc. Natl. Acad. Sci. 85, 2444-2448) und BLAST-Program (Gish und States (1993) Nat. Genet.3, 266-272; Altschul et al. (1990) J. Mol. Biol. 215, 403-410) durchgeführt. Diese Programme wurden auch für Sequenzvergleiche mit GenBank (Version 102.0) und Swiss Prof (Version 34.0) verwendet.

Posttranslationale Modifikationen wie Glykosylierung und Myristylierung von H-SemaL sind ebenfalls möglich. Konsensus-Sequenzen für N-Glykosylierungsstellen wurden mit Hilfe des Programms Prosite (GCG Programm-Paket) an den Positionen 105, 157, 258, 330, 602 der Aminosäuresequenz von H-SemaL (gemäß Tabelle 4) gefunden, solche für Myristylierung an den Positionen 114, 139, 271, 498, 499, 502, 654 (Konsensus-Sequenz: G∼(E, D, R, K, H, P, F, Y, W) x (S, T, A,G, C, N)∼(P)). Darüber hinaus enthält die Aminosäuresequenz von H-SemaL mehrere Konsensus-Sequenzen für potentielle Phosphorylierungsstellen durch unterschiedliche Kinasen. Deshalb kann davon ausgegangen werden, daß H-SemaL das Substrat unterschiedlicher Kinasen sein kann, z.B. Phosphorylierungsstellen für Kreatin-Kinase 2, Protein-Kinase C und Tyrosin-Kinase.

Vorausgesagte Kreatin-Kinase 2-Phosphorylierungs-Stellen (Konsensussequenz Ck2: (S,T)x2(D,E)) (Prosite, GCG) an den Positionen 119, 131, 173, 338, 419, 481 der Aminosäuresequenz. Vorausgesagte Protein-Kinase-C-Phosphorylierungs-Stellen (Konsensussequenz PkC: (S,T)x(R,K)) (Prosite, GCG) an den Positionen 107, 115, 190, 296, 350, 431, 524, 576 der Aminosäuresequenz. Vorausgesagte Tyrosin-Kinase-Phosphorylierungs-Stelle (Konsensussequenz: (R,K)x{2,3}(D,E)x{2,3}Y) (Prosite GCG) an Position 205 der Aminosäuresequenz.
Die Konsensussequenzen sind im Einbuchstabencode für Aminosäuren angegeben.

Ein für Integrine charakteristisches "RGD"-Motiv (Arginin-Glycin-Asparaginsäure) findet sich an Position 267.
Die Glykosylierungsstellen sind gut konserviert zwischen viralem AHV-Sema, H-SemaL und (soweit bekannt) M-SemaL.

Eine Di- oder Multimerisierung von H-SemaL ist möglich und wurde bei anderen Semaphorinen wie CD100 beschrieben {Hall et al. (1996)}. Das CD100 Molekül ist ebenfalls ein membranverankertes Glykoprotein-Dimer von 150kd. CD100 ist jedoch nicht nahe verwandt mit dem erfindungsgemäßen humanen Semaphorin (H-SemaL).

Die partielle cDNA-Sequenz von M-SemaL hat eine Länge von 1195 Nukleotiden. Diese Sequenz kodiert für ein Protein mit 394 Aminosäuren. Diese 394 Aminosäuren entsprechen den Aminosäuren 1 bis 396 von H-SemaL. Das Signalpeptid im M-SemaL ersteckt sich über die Aminosäuren 1 bis 44 (genau wie im H-SemaL). Die Sema-Domäne beginnt bei der Aminosäure 45 und erstreckt sich bis zum Ende bzw. wahrscheinlich über das Ende der Sequenz gemäß Tabelle 4 hinaus.

Multiple Alignments wurden mit Hilfe des Programms Clustal W (Thompson et al. (1994)) durchgeführt Diese Alignments wurden manuell weiterbearbeitet mit Hilfe von SEAVIEW (Galtier et al. (1996) Comput Appl. Biosci 12, 543-548). Die phylogenetischen Entfernungen wurden mit Clustal W (Thompson et al. (1994)) bestimmt.

Ein Vergleich der Proteinsequenzen der bekannten und der neuen Semaphorine und eine phylogenetische Analyse dieser Sequenzen zeigt, daß sich die Gene entsprechend ihrer phylogenetischen Verwandschalt einteilen lassen. Hier fließt natürlich die C-terminale Domänenstruktur der entsprechenden Semaphorin-Subtypen als entscheidender Faktor mit ein, weshalb Semaphorine der gleichen Untergruppen in der Regel auch phylogenetisch näher verwandt sind, als Semaphorine unterschiedlicher Untergruppen. Einfluß hat auch, aus welcher Spezies das Semaphorin isoliert wurde, d.h. ob die entsprechenden Spezies phylogenetisch nahe miteinander verwandt sind oder nicht.

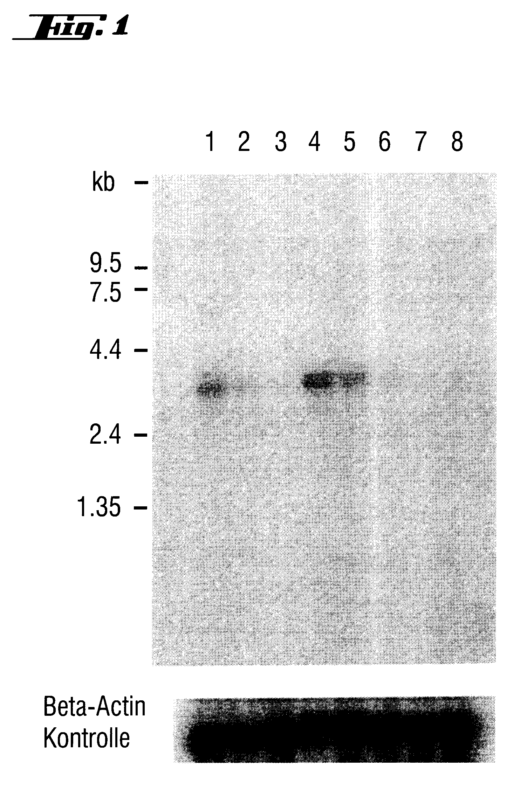

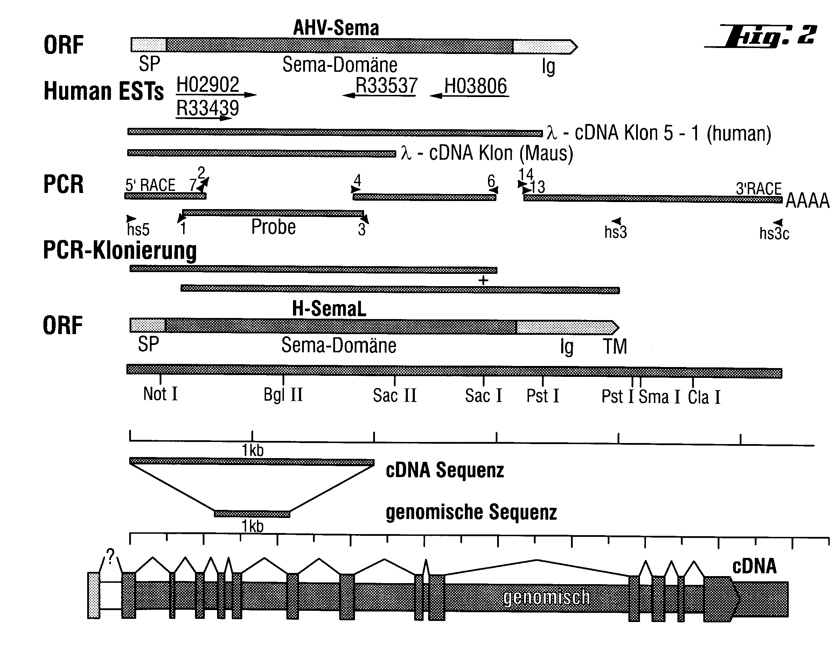

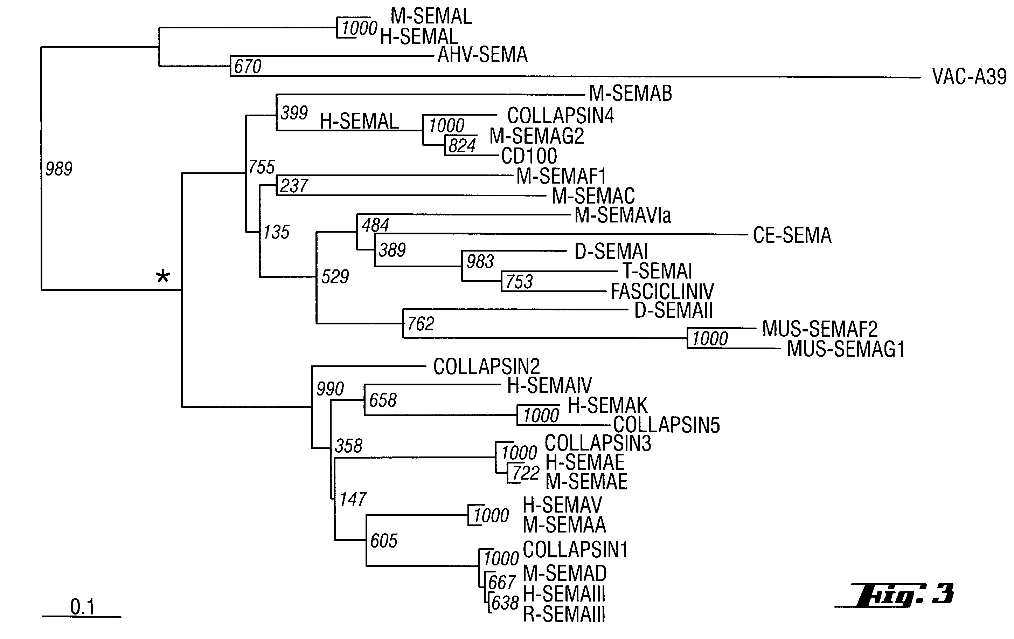

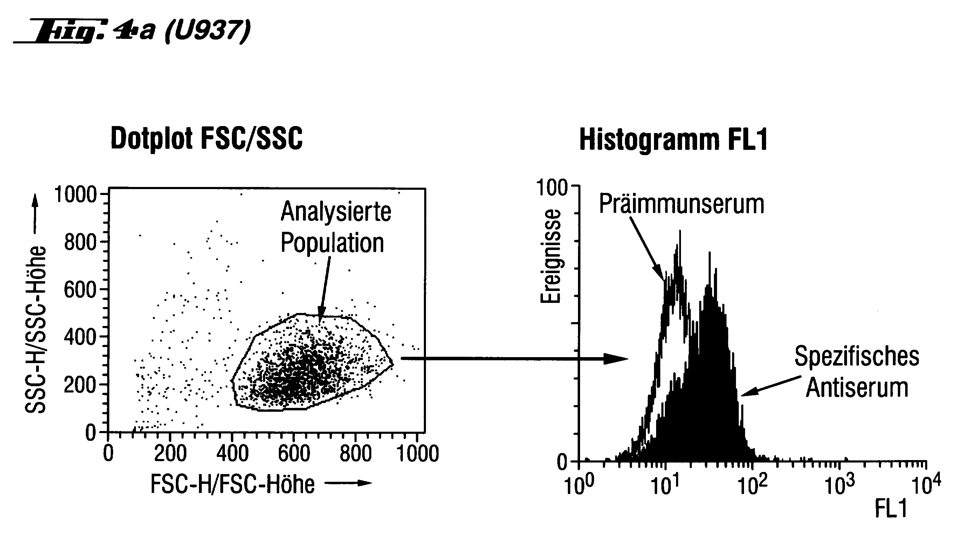

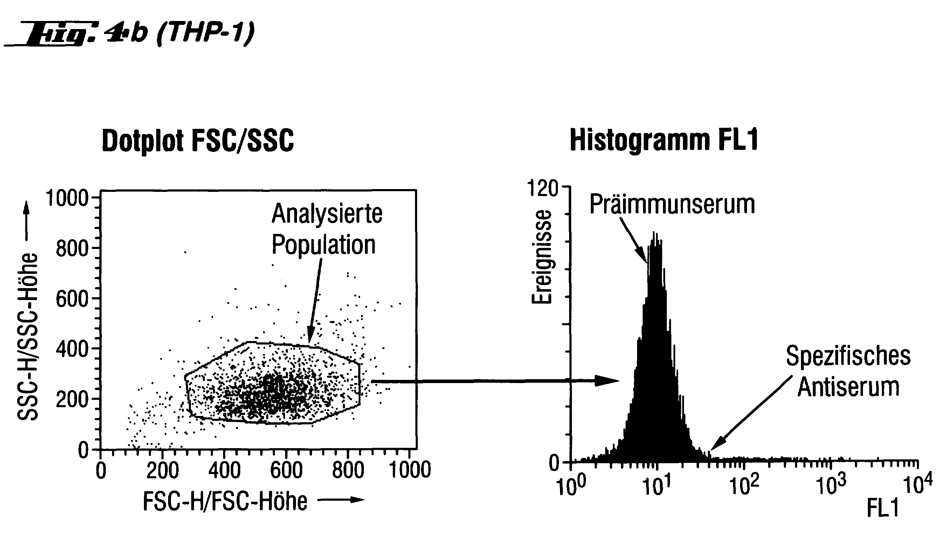

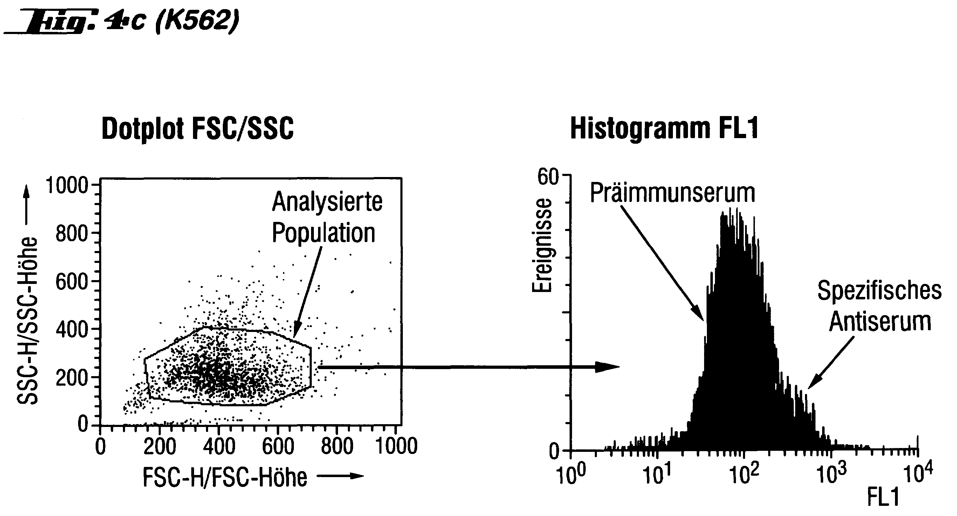

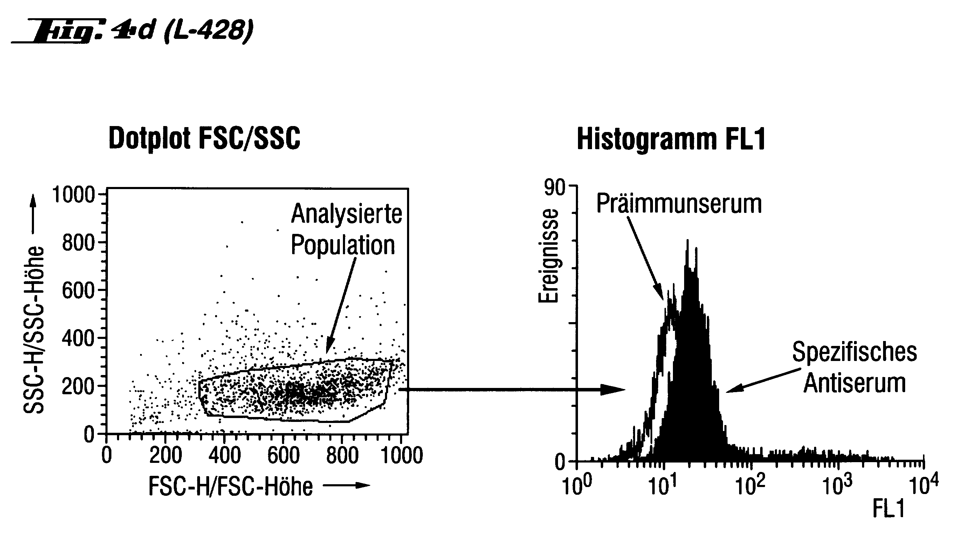

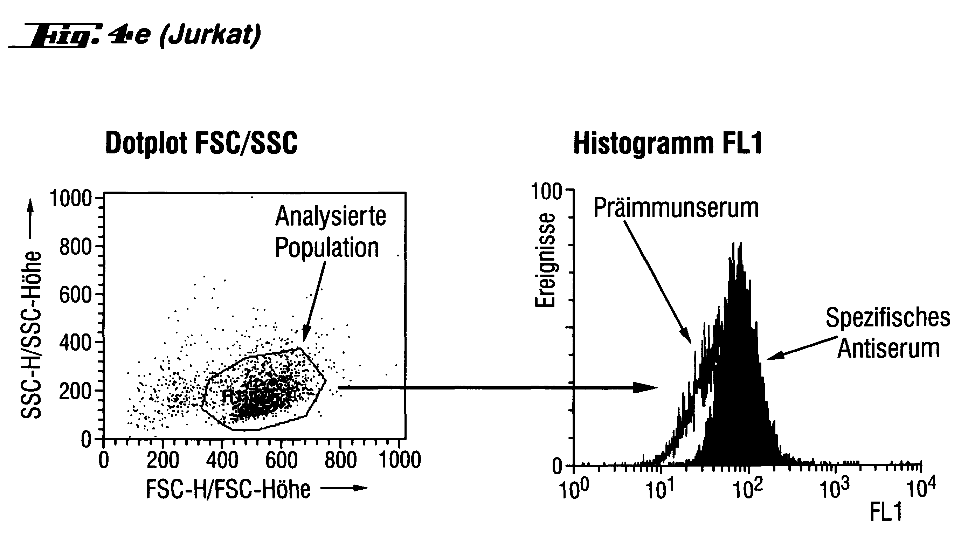

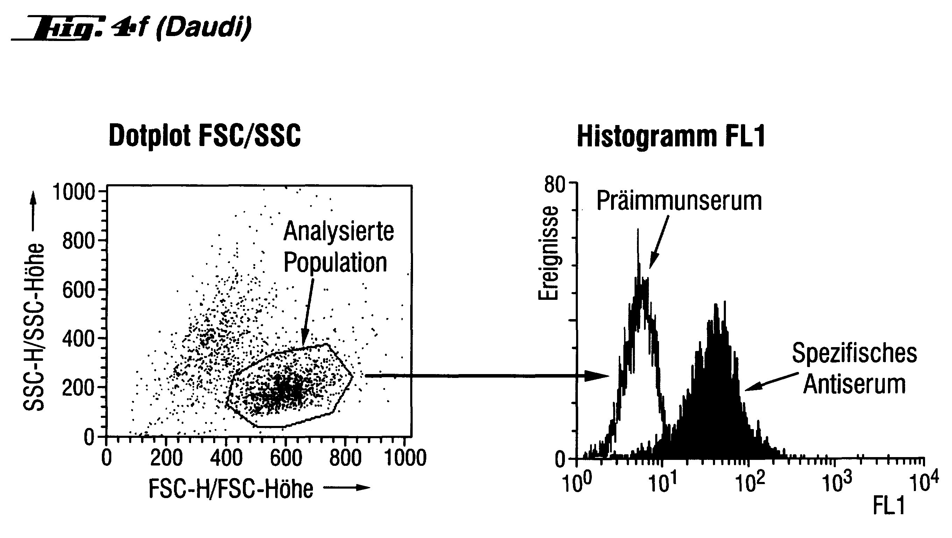

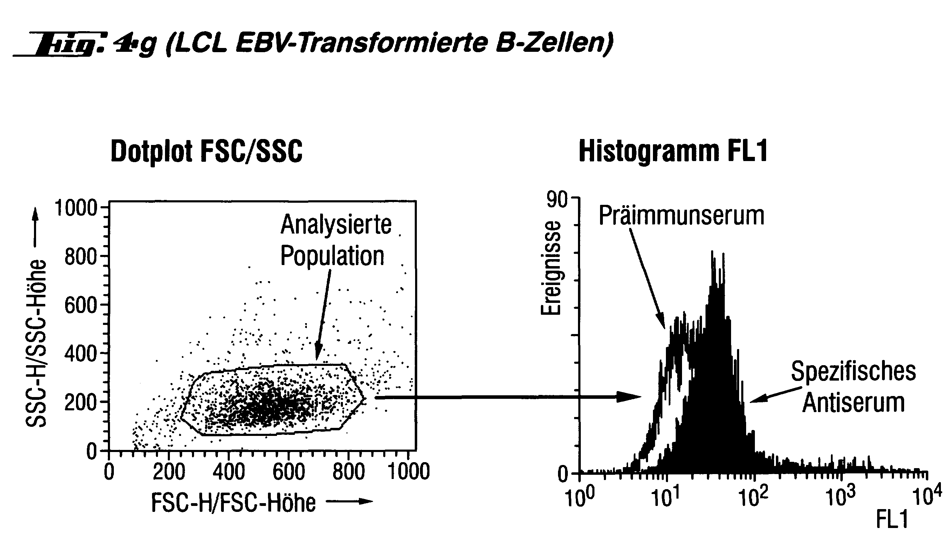

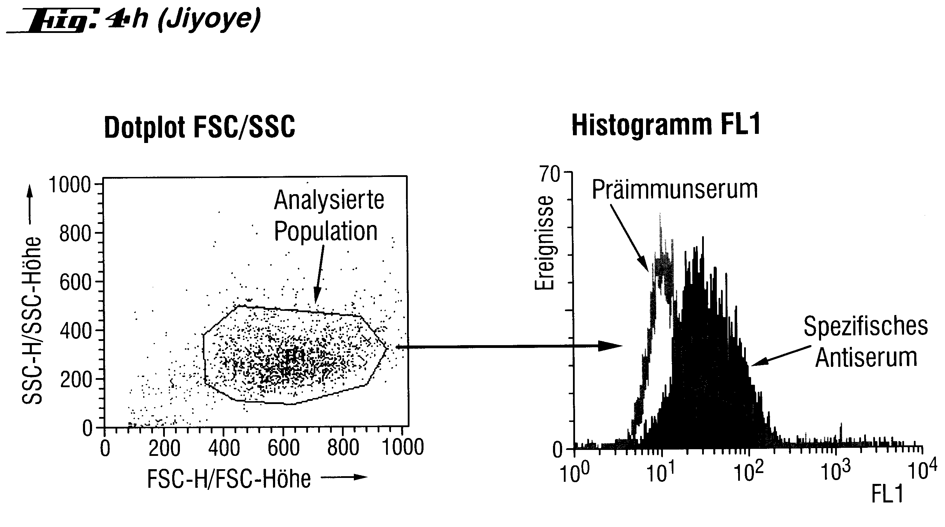

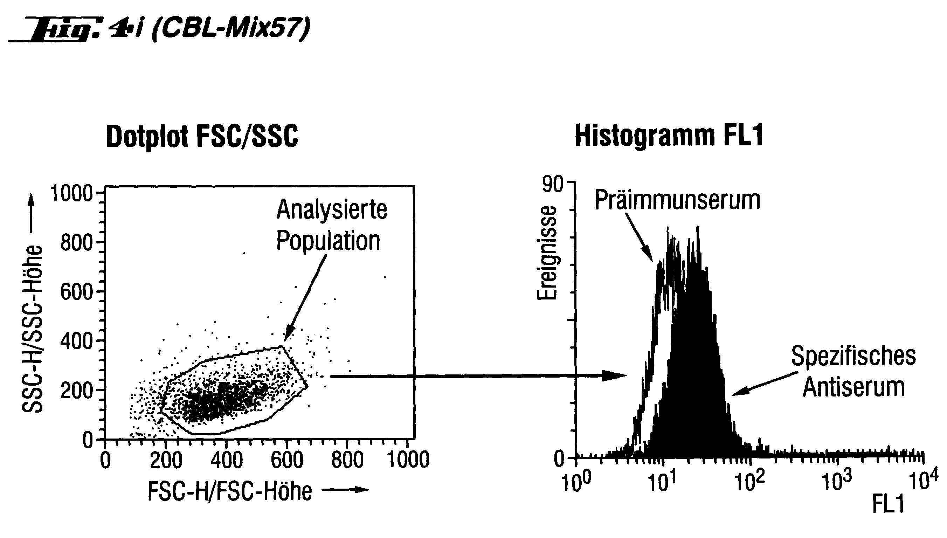

Eine phylogenetische Analyse (vergl. Figur 3) der bekannten Semaphorin Aminosäuresequenzen (vollständige Sequenzen und/oder Teilsequenzen, wobei die Aminosäuresequenzen für H-SemaL und M-SemaL gemäß den Tabellen 4 und 5 verwendet wurden, für alle anderen Sequenzen, die unter den Zugangsnummern gespeicherten Sequenzen bzw. die von diesen Sequenzen abgeleiteten Aminosäuresequenzen)) mittels des Programms CLUSTALW {Thompson J.D. et al. (1994) Nucleic Acids Res. 22:4673-4680} zeigt, daß die Aminosäuresequenzen von H-SemaL und M-SemaL phylogenetisch nahe miteinander verwandt sind und eine eigene phylogenetische Gruppe bilden. H-SemaL und M-SemaL wiederum sind phylogenetisch am nächsten verwandt mit AHV-Sema und Vac-A39. Sie sind untereinander deutlich näher verwandt, als mit irgendeinem anderen bisher bekannten Semaphorin. Die Analyse zeigt weiterhin, daß auch andere Semaphorine phylogenetisch nahe miteinander verwandt sind und eigene Gruppen innerhalb der Semaphorine bilden. Beispielsweise fallen die Semaphorine, die sezerniert werden, z.B. H-Sema III, IV, V, und E in eine phylogenetische Gruppe. Zu dieser Subfamilie gehören auch deren Homologe in anderen Spezies, während das humane (transmembrane) CD100 mit dem entsprechenden Maus-Homologen (M-Sema G2) und mit Collapsin-4 in eine phylogenetische Gruppe fällt.

Im Bezug auf die gesamten Aminosäuresequenzen liegen die beobachteten Homologien innerhalb der phylogenetischen Gruppen zwischen etwa 90% und 80% Aminosäureidentität im Bezug auf sehr nahe verwandte Gene wie z.B. H- und M-SemaE oder -III/D und etwas weniger als 40% bei wenig verwandten Genen der Semaphorine. Innerhalb der Sema-Domäne liegt die beobachtete Aminosäureidentität um einige Prozent höher, und durch ihren großen Anteil am Gesamtprotein (50-80% des Proteins gehören zur Sema-Domäne) der Aminosäuresequenz beinflußt diese wesentlich die Gesamtidentität.
H-SemaL ist, über das Gesamtprotein berechnet, zu 46% identisch mit AHV-Sema, wird dagegen die Sema-Domäne allein betrachtet, dann beträgt die Aminosäureidentität 53%. Dies ist höher als z.B. zwischen den verwandten M-SemaB und -C (37% Identität im Bezug auf das Gesamtprotein, 43% Identität im Bezug auf die Sema-Domäne), ähnlich wie M-SemaA und -E (43% Gesamtprotein, 53% Sema-Domäne). Die Aminosäureidentität zwischen der partiellen M-SemaL Sequenz (Tabelle 6) und H-SemaL (Tabelle 5) liegt im Bereich der Sema-Domäne bei 93%, so daß davon ausgegangen werden kann, daß es sich um das entsprechend homologe Gen der Maus handelt.
Korrespondierende Semaphorine zu H-SemaL und M-SemaL in anderen Spezies können innerhalb der Sema-Domäne eine Aminosäureidentität größer als 40% im Bezug auf H-SemaL aufweisen. Bei den nahe verwandten Wirbeltieren (Säuger, Vögel) können sogar Aminosäureidentitäten über 70% angetroffen werden.

Es handelt sich um eine neue Subfamilie von Semaphorinen mit größerer Aminosäureidentität zu dem viralen AHV-Sema als zu den bisher bekannten humanen bzw. murinen Semaphorinen, und mit einer für humane Semaphorine bisher nicht bekannten C-terminalen Struktur. Diese neuen Semaphorine (Mitglieder der Subfamilie) zeichnen sich dadurch aus, daß sie aufgrund ihrer Domänen-struktur in die Untergruppe IV fallen und/oder die gleiche phylogenetische Gruppe fallen wie H-SemaL und M-SemaL und/oder im Bezug auf die gesamte Aminosäuresequenz zu H-SemaL eine Aminosäureidentität von mindestens 30 bis 40 %, vorzugsweise 50 bis 60 %, besonders bevorzugt 70 bis 80 % oder eine größere Identität aufweisen und/oder im Bezug auf die Sema-Domäne eine Aminosäureidentität mit H-SemaL von mindestens 70 %, vorzugsweise größer 80 %, besonders bevorzugt größer 90 % aufweisen.

Den Semaphorinen vom Typ L kommt auch eine andersartige biochemische Funktion zu. Eine neue Funktion dieser Semaphorine liegt in der Modulation des Immunsystems.

Das nächste Verwandte von H-SemaL ist das virale AHV-Semaphorin (AHV-Sema). Dieses ist von ähnlicher Größe, besitzt aber im Gegensatz zum H-SemaL keine Transmembrandomäne. Das AHV-Sema wird vermutlich von virusinfizierten Zellen sezerniert, um den H-SemaL äquivalenten Rezeptor (Semaphorin von Typ L im Streifengnu) im natürlichen Wirt (Streifengnu) zu blockieren, und so dem Angriff des Immunsystems zu entgehen. Ferner ist eine Funktion als repulsives Agens (Chemorepellent) für Zellen des Immunsystems denkbar.

Die biochemische Funktion der neuen Semaphorine vom Typ L und deren Derivate ist als generell immunmodulierend und/oder entzündungsmodulierend anzusehen. Einerseits können sie
A) als die Immunantwort hemmende Moleküle entweder lokal, z.B. als Transmembranprotein an der Oberfläche von Zellen oder auch über größere Entfernungen, z.B. wenn sie durch Prozessierung (z.B. Proteasen) oder alternatives Splicing sezerniert werden, z.B. durch Diffusion im Gewebe, ihre Wirkung als Chemorepellent und/oder Immunsuppressivum entfalten.
   Beispielsweise kann die Expression dieser neuen Semaphorine vom Typ L z.B. an der Oberfläche der Zellen der Gefäßendothelien das Leukozyten-Attachment und deren Migration durch die Gefäßwand verhindern. Den neuen Semaphorinen kann eine Rolle bei der Aufrechterhaltung von Schrankenwirkungen, z.B. zur Verhindung von Infektionen in besonders "wichtigen" oder exponierten Organen, beispielsweise zur Aufrechterhaltung der Blut-Hirn-Schranke, des Plazentarkreislaufs und/oder anderen immunologisch priviligierten Orten (z.B. Pancreas-Inseln) und/oder beim Schutz vor Autoimmunerkrankungen zukommen. Darüber hinaus können die neuen Semaphorine und/oder ihre Derivate in verschiedenen Geweben auch an repulsiven Signalen, beispielsweise für Zellen des Immunsystems (z.B. Leukozyten) als Schutz gegen versehentliche Aktivierung von Abwehrmechanismen beteiligt sein.
B) Weiterhin können den neuen Semaphorinen und/oder deren Derivaten Funktionen als akzessorische Moleküle zukommen. An der Zelloberfläche exprimiert können sie beispielsweise an der Interaktion mit Zellen des Immunsystems im Rahmen der Aktivierung von Abwehrmechanismen z. B. bei Virusinfektionen beteiligt sein.

Dadurch ergeben sich mehrere Verwendungsmöglichkeiten für die neuen Sempahorine vom Typ L und deren Derivaten sowie den für diese Proteine kodierenden Nukleinsäuren.

Funktion A): Es handelt sich um ein immunsuppressives und/oder entzündungshemmendes Prinzip: Zahlreiche potentielle Anwendungsmöglichkeilen liegen in den Bereichen Organtransplantation, Entzündungstherapie, Immuntherapie und Gentherapie.

Beispielsweise können mit Hilfe der Semaphorin-kodierenden DNA oder Derivaten derselben nichthumane, transgene Tiere hergestellt werden.
Eine Anwendungsmöglichkeit dieser Tiere liegt in der Hemmung der Transplantatabstoßung in transgenen Modellen für Organtransplantationen. Beispielsweise können transgene, gegen Abstoßung geschützte tierische Organe für Xenotransplantationen hergestellt werden. Dies sollte z.B. auch zusammen mit anderen Transgenen (z.B. Komplementregulatoren wie DAF oder CD59) möglich sein.
Eine weitere Anwendung liegt in der Herstellung von nicht-humanen Knock-out Tieren, beispielsweise von Knock-out Mäusen( 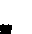 Laboratory Protocols for Gene-Targeting", Torres and Kühn (1997) Oxford University Press, ISBN 0-19-963677-X): Durch Knock-out des Mausgens M-SemaL können z.B. weitere Funktionen des Gens aufgefunden werden. Sie stellen auch potentielle Modellsysteme für entzündliche Erkrankungen dar, falls die Mäuse ohne Semaphorin-Gen lebensfähig sind. Sollte M-SemaL für die Immunmodulation wichtig sein, so sind vermehrt solche Mäuse zu erwarten.
Weiterhin können nicht-humane Knock-in-Tiere, beispielsweise Mäuse, hergestellt werden. Dabei wird z.B. M-SemaL durch normales/verändertes H-SemaL oder verändertes M-SemaL (z.B. Integration der neuen Semaphorin-Subtypen unter der Kontrolle von konstitutiven und/oder induzierbaren Promotoren) ersetzt. Solche Tiere können z.B. für die Suche nach weiteren Funktionen der neuen Semaphorine, z.B. Funktionen des humanen Gens oder Derivaten dieser Gene dienen oder zur Identifizierung und Charakterisierung von immunmodulierenden Wirkstoffen benutzt werden.

Verwendung von z. B. Nukleinsäuren, die für Semaphorine vom Typ L oder Derivate derselben kodieren, zur Herstellung von z.B. rekombinanten Immunsuppressiven, anderen löslichen Proteinen oder Peptiden die sich von der Aminosäuresequenz der Semaphorine vom Typ L, z.B. Von H-SemaL oder den entsprechenden Nukleinsäuren, z.B. Genen ableiten. In ähnlicher Weise können auch Agonisten mit struktureller Ähnlichkeit hergestellt werden. Diese immunsuppressiven Wirkstoffe/Agonisten können bei Autoimmunerkrankungen und entzündlichen Erkrankungen und/oder Organtransplantationen eingesetzt werden.

Gentherapie mit Semaphorinen vom Typ L, z.B. mit Nukleinsäuren, die für H-SemaL oder deren Derivate kodieren, z. B. mittels viraler oder nichtviraler Methoden. Einsatz bei Autoimmunerkrankungen und entzündlichen Erkrankungen, der Transduktion von Organen sowie vor/während/nach Transplantationen zur Verhinderung der Transplantatabstoßung.

Insbesondere können die neuen Semaphorine und/oder die für diese Semaphorine kodierenden Nukleinsäuren und Derivate derselben, insbesondere H-SemaL, für H-SemaL kodierende DNA und Derivate derselben in einem Verfahren zum Screening von Wirkstoffen, insbesondere zur Identifizierung und Charakterisierung von immunnodulierenden Wirkstoffen, eingesetzt werden.

Funktion B): H-SemaL ist ein akzessorisches Molekül, das an der Zelloberfläche exprimiert wird und an der Interaktion mit Zellen, z.B. des Immunsystems, z.B. als akzessorisches Molekül in der Aktivierung von Signalwegen, beteiligt ist Ein virales Gen bzw. das Genprodukt eines viralen oder anderen pathogenen Gens z.B. mikrobilogischen Ursprunges könnte z.B. als kompetitiver Inhibitor dieses akzessorischen Moleküls wirken. Eine Anwendung für die neuen Semaphorine liegt bei dieser Funktion ebenfalls im Bereich der Organtransplantation, Entzündungstherapie, Irnmuntherapie und/oder Gentherapie.
Beispielsweise können die neuen Semaphorine in einem Verfahren zum Screening von antagonistischen Wirkstoffen bzw. Inhibitoren verwendet werden. Auf diese Weise identifizierte Wirkstoffe können dann z.B. zur Blockade des Semaphorin-Rezeptors eingesetzt werden. Lösliche und/oder sezernierte H-SemaL Antagonisten bzw. Inhibitoren können beispielsweise chemische Substanzen oder die neuen Semaphorine bzw. Derivate derselben selbst sein (z.B. Teile/verkürzte Formen derselben, beispielsweise ohne Membrandomäne oder als Ig-Fusionsproteine oder von diesen abgeleitete Peptide, die geeignet sind, den korrespondierenden Rezeptor zu blockieren). Auf diese Weise identifizierte, spezifische Antagonisten und/oder Inhibitoren können beispielsweise kompetitiv wirken und bei der Hemmung der Abstoßung z.B. in transgenen Modellen für Organtransplantationen und bei Autoimmunerkrankungen, entzündlichen Erkrankungen und Organtransplantationen eingesetzt werden. Nukleinsäuren, z. B. DNA, die für die neuen Semaphorine kodieren bzw. deren mit Hilfe von molekularbiologischen Methoden erzeugte Derivate können beispielsweise für die Herstellung nichthumaner, transgener Tiere verwendet werden. Eine Überexpression von H-SemaL kann in diesen transgenen Tieren zu vermehrter Anfälligkeit für Autoimmunerkrankungen und/oder entzündlichen Erkrankungen führen. Solche transgenen Tiere eignen sich damit zum Screening von neuen, spezifischen, immunmodulierenden Wirkstoffen.
Solche Nukleinsäuren können ebenso für die Herstellung von nicht-humanen Knock-out Tieren, beispielsweise Knock-out Mäusen, bei denen das Mausgen M-SemaL ausgeschaltet wird, verwendet werden. Solche Knock-out Tiere können für die Suche nach weiteren biochemischen Funktionen des Gens eingesetzt werden. Sie stellen auch potentielle Modellsysteme für entzündliche Erkrankungen dar, falls die Mäuse ohne das M-SemaL Gen lebensfähig sind.
Diese DNA kann ebenso zur Herstellung von nicht-humanen Knock-in Tieren, beispielsweise Mäusen verwendet werden. Dabei wird das M-SemaL-Gen durch ein verändertes M-SemaL Gen/cDNA oder ein gegebenfalls verändertes, z.B. mutiertes Semaphorin Typ L Gen/cDNA einer anderen Spezies, z.B. von H-SemaL ersetzt, Solche transgenen Tiere können für die Suche nach weiteren Funktionen der erfindungsgemäßen Semaphorine verwendet werden.

Die Erfindung betrifft auch die Verwendung der Semaphorine vom Typ L und deren Derivate, sowie der für diese Proteine kodierenden Nukleinsäuren, z. B. Gene/cDNAs und deren Derivate und/oder mit Hilfe dieser Semaphorine identifizierter Wirkstoffe zur Herstellung von Arzneimitteln. Beispielsweise können Arzneimittel hergestellt werden, die in der Gentherapie angewendet werden können und die Agonisten und/oder Antagonisten der Expression der Semaphorine vom Typ L, beispielsweise von H-SemaL, enthalten. Dazu können z. B. virale und/oder nichtvirale Methoden verwendet werden. Diese Arzneimittel können z.B. bei Autoimmunerkrankungen und entzündliche Erkrankungen, Organtransplantationen vor und/oder während und/oder nach der Transplantation zur Verhinderung der Abstoßung eingesetzt werden.
Die für die neuen Semaphorine kodierenden Nukleinsäuren, z. B. Gene, cDNAs und deren Derivate können auch als Hilfsmittel in der Molekularbiologie eingesetzt werden.
Darüberhinaus können die neuen Semaphorine, insbesondere H-SemaL und Nukleinsäuren, z. B. Gene/cDNAs derselben in Verfahren zum Screening neuer Wirkstoffe eingesetzt werden. Modifizierte Proteine und/oder Peptide, die sich z. B. von H-SemaL und/oder M-SemaL ableiten, können zur Suche nach dem entsprechenden Rezeptor und/oder dessen Antagonisten bzw. Agonist in funktionellen Tests, beispielsweise mittels Expressionskonstrukten von H-SemaL und Homologen eingesetzt werden.

Die Erfindung betrifft auch die Verwendung eines Semaphorins vom Typ L oder einer Nukleinsäure-Sequenz, die für ein Semaphorin vom Typ L kodiert in einem Verfahren zur Identifizierung von pharmakologischen Wirkstoffen, insbesondere von immunmodulierenden Wirkstoffen.

Die Erfindung betrifft auch Verfahren zur Identifizierung von Wirkstofen, wobei ein Semaphorin vomTyp L oder ein Derivat derselben bzw. eine Nukleinsäure-Sequenz, die für ein Semaphorin vom Typ L kodiert oder ein Derivat derselben eingesetzt wird, um pharmakologische Wirkstoffe, z. B. immunmodulierende Wirkstoffe zu identifizieren. Beispielsweise betrifft die Erfindung ein Verfahren, bei dem ein Semaphorin vom Typ L unter definierten Bedingungen mit einem zu untersuchenden Wirkstoff inkubiert wird und parallel ein zweiter Ansatz ohne den zu untersuchenden Wirkstoff, aber unter ansonsten gleichen Bedingungen durchgeführt wird und dann die inhibierende bzw. aktivierende Wirkung des zu untersuchenden Wirkstoffs bestimmt wird.
Beispielsweise betrifft die Erfindung auch Verfahren zur Identifizierung von Wirkstoffen, wobei eine Nukleinsäure-Sequenz, die für ein Semaphorin vom Typ L kodiert oder ein Derivat derselben unter definierten Bedingungen in Gegenwart eines zu untersuchenden Wirkstoffs exprimiert wird und das Ausmaß der Expression bestimmt wird. Gegebenenfalls können auch in einem solchen Verfahren zwei oder mehrere Ansätze parallel unter gleichen Bedingungen durchgeführt werden, wobei die Ansätze aber unterschiedliche Mengen des zu untersuchenden Wirkstoffs enthalten.
Beispielsweise kann der zu untersuchende Wirkstoff die Transkription und/oder die Translation inhibieren oder aktivieren.
Das Semaphorin vom Typ L kann wie seine viralen Homologen an das neu beschriebene Rezeptormolekül VESPR (Comeau et al, (1998) Immunity, Vol. 8, 473-482) binden und kann vermutlich in Monozyten eine Induktion von Zell-Adhäsionsmolekülen wie ICAM-1 und Zytokinen wie Interleukin-6 und Interleukin-8 bewirken. Dies kann zu deren Aktivierung und zur Zellaggregation führen. Das Expressionsmuster des VESPR-Rezeptors zeigt teilweise interessante Paralleln zu H-Senal, z.B. eine starke Expression in Placenta und eine deutliche Expression in Milzgewebe. Interaktionen mit weiteren, noch unbekannten Rezeptoren der Plexin-Familie, oder anderen Rezeptoren sind möglich. Auch eine Interaktion mit sich selbst oder anderen semaphorinähnlichen Molekülen ist möglich. Eine Interaktion der Semaphorine vom Typ L kann insbesondere Ober eine konservierte Domäne im C-terminalen Bereich der Sema-Domäne stattfinden.

### Zu den Annotation Plasmiden:

pMelBacA-H-SemaL (6622bp) in pMelBacA (Invitrogen, De Schelp, NL)(SEQ ID NO.42). Nukleotid 96-98 ATG - Startkodon, Nukleotid 96-168 Mellitin Signal-Sequenz, Nukleotid 168-173 BamHI Schnittstelle (PCR/Klonierung), Nukleotid
   171-1998 Leserahmen SemaL Aminosäuren 42-649 (ohne eigene Signal-Sequenz und ohne Transmembransequenz), Nukleotid 1993-1998 EcoRI Schnittstelle (PCR/Klonierung) und Nukleotid 1992-1994 Stop Codon
Plasmid pCDNA3.1-H-SemaL-MychisA (7475 bp) (SEQ ID NO. 35):
   Nukleotid 954-959 BamHI Schnittstelle (Klonierung),
   Nukleotid 968-970 ATG SemaL, Nukleotid 968-2965 Leserahmen SemaL, Nukleotid 2963-2968 Pml I Schnittstelle, Nukleotid 2969-2974 HindIII Schnittstelle,
   Nukleotid 2981-3013 Myc-Tag, Nukleotid 3026-3033 6xHis-Tag, Nukleotid 3034-3036 Stop Codon,
Plasmid pCDNA3.1-H-SemaL-EGFP-MychisA (8192 bp): (SEQ ID NO. 36):
   Nukleotid 954-959 BamHI Schnittstelle (Klonierung), Nukleotid 968-970 ATG SemaL, Nukleotid 968-2965 Leserahmen SemaL, Nukleotid 2963-2965 halbe Pml I Schnittstelle, Nukleotid 2966-3682 Leserahmen EGFP (in Pml I Kloniert), Nukleotid 3683-3685 halbe Pml I Schnittstelle, Nukleotid 3685-3691 HindIII, Nukleotid 3698-3730 Myc-Tag, Nukleotid 3743-3760 6xHis-Tag, und Nukleotid 3761-3763 Stop Codon
Plasmid plND-H-SemaL-EA (7108 bp) in Vektor plND (Invitrogen, De Schelp, NL) (SEQ ID No. 38):
   Nukleotid 533-538 BamHI Schnittstelle (Klonierung), Nukleotid 546-548 ATG SemaL, Nukleotid 546-Leserahmen SEMAL, Nukleotid 2542-2547 Pml I Schnittstelle, Nukleotid 2546-2553 HindIII Schnittstelle und Nukleotid 2563-2565 Stop Codon.
Plasmid plND-H-SemaL-EE (Gesamtlänge 7102 bp) in Vektor plND (Invitrogen, De Schelp, NL) (SEQ ID No. 37):
   Nukleotid 533-538 BamHI Schnittstelle (Klonierung), Nukleotid 546-548 ATG SemaL, Nukleotid 546-Leserahmen SemaL, Nukleotid 2542-2547 Pml I Schnittstelle, Nukleotid 2548-2553 HindIII Schnittstelle, Nukleotid 2560-2592 Myc-Tag, Nukleotid 2605-2622 6xHis-Tag und Nukleotid 2623-2625 Stop Codon.
Plasmid pQE30-H-SemaL-179-378.seq (4019 bp) in Vektor pQE30 (Qiagen, Hilden) entspricht pQE30-H-SemaLBH (SEQ ID No. 39):
   Nukleotid 115-117 ATG, Nukleotid 127-144 6xHis-Tag, Nukleotid 145-750 BamHI-HindIII PCR-Fragment SemaL Aminosäuren (aa) 179-378 und Nukleotid 758-760 Stop Codon.
Plasmid pQE31-H-SemaL- (SH (3999 bp) in Vektor pQE31 (Qiagen, Hilden) (SEQ ID No. 40):
   Nukleotid 115-117 ATG, Nukleotid 127-144 6xHis-Tag, Nukleotid 147-152 BamHI, Nukleotid 159-729 SacI-HindIII Fragment SemaL (C-terminal) aa480-666 und Nukleotid 734-736 Stop Codon.

### Beispiele:

Versuchsbedingungen, die in den Beispielen Anwendung finden:

### Verwendete PCR-Programme:

Taq52-60 (mit Ampli-Taq^{R}-Polymerase, Perkin Elmer, Weil der Stadt, Deutschland)
   - 96°C/60s: 1 Zyklus
   - 96°C/15s-52°C/20s-70°C/60s: 40 Zyklen
   - 70°C/60s: 1 Zyklus
Taq60-30
   - 96°C/60s: 1 Zyklus
   - 96°C/15s-60°C/20s-70°C/30s: 35 Zyklen
   - 70°C/60s: 1 Zyklus
Taq60-60
   - 96°C/60s: 1 Zyklus
   - 96°C/15s-60°C/20s-70°C/60s: 35 Zyklen
   - 70°C/60s: 1 Zyklus
Taq62-40
   - 96°C/60s: 1 Zyklus
   - 96°C/15s-62°C/20s-70°C/40s: 35 Zyklen
   - 70°C/60s: 1 Zyklus

### Verwendete Reaktionsbedingungen für PCR mit Taq-Polymerase:

50µl Reaktionsansätze mit 100-200ng Template, 200µM dNTP, 0,2-0,4 µM je Primer, 2.5U Ampli-Taq^{R}, 5µl des mitgelieferten 10x Reaktionspuffers

### Verwendete Programme für:

1. XL62-6 (mit Expand-Long Template PCR System^{R}, Boehringer Mannheim, Deutschland)
   - 94°C/60s: 1 Zyklus
   - 94°C/15s-62°C/30s-68°C/6min: 10 Zyklen
   - 94°C/15s-62°C/30s-68°C/(6min+15s/Zyklus): 25 Zyklen
   - 68°C/7min: 1 Zyklus
2. XL62-12 (mit Expand-Long Template PCR System^{R}, Boehringer Mannheim, Deutschland)
   - 94°C/60s: 1 Zyklus
   - 94°C/15s-62°C/30s-68°C/12min: 10 Zyklen
   - 94°C/15s-62°C/30s-68°C/(12min+15s/Zyklus): 25 Zyklen
   - 68°C/7min: 1 Zyklus

### Reaktionsbedingungen für PCR mit Expand-Long Template PCR System

50µl Reaktionsansätze mit 100-200ng Template, 500µM dNTP, 0,2-0,4 µM je Primer, 0,75µl Enzym-Mix, 5µl des mitgelieferten 10x Reaktionspuffers Nr. 2.

### Beispiel 1:

Ausgehend von Sequenzen des AHV-Sema (Ensser u. Fleckenstein (1995), J. General Virol. 76: 1063-1067) wurden PCRs und RACE-PCRs durchgeführt. Als Ausgangsmaterial hierfür diente humane cDNA aus Plazenta-Gewebe, an welche Adapter zur RACE-Amplifikation ligiert wurden (Marathon™-cDNA Amplification Kit, Clontech Laboratories GmbH, Tullastraße 4, 69126 Heidelberg, Deutschland). Zunächst wurde mittels spezifischer Primer (Nr. 121234 + Nr. 121236, Tabelle 6) ein PCR-Fragment mit einer Länge von etwa 800bp (Basenpaaren) amplifiziert (PCR-Programm: (Taq60-60)). Dieses wurde kloniert und sequenziert (Taq-Dye-Deoxy-Terminator Sequenzierungs-Kit, Applied Biosystems, Foster: City, CA USA) Brunnenweg 13, Weilderstadt). Die Sequenzierung des PCR-Produkts ergab eine Sequenz, die eine hohe Homologie zu der DNA-Sequenz von AHV-Sema aufweist, identisch zu der Sequenz der beiden ESTs.

Ein PCR-Fragment von 600bp wurde mit dem Primerpaar (Nr. 121237 + Nr. 121239, Tabelle 6) identifiziert. Es zeigte sich, das es sich um Klone mit DNA-Sequenzen des selben Gens handelte.

### Beispiel 2:

Das 800bp PCR-Fragment aus Beispiel 1 wurde radioaktiv markiert (Random-Priming nach der Methode von {Feinberg (1983) Anal. Biochem. 132:6-13}, mit ³²P-α-dCTP) und als Sonde für einen Multi-Tissue Northern-Blot (Human Multiple Tissue Northern Bot II, Clontech, Heidelberg, Germany), der mRNA-Proben aus den Geweben Milz, Thymus, Prostata, Testis, Ovarien, Dünndarm, Dickdarm und Leukozyten (PBL) enthält, verwendet. Dabei Zeigte sich deutlich die Expression einer mRNA mit einer Länge von etwa 3.3kb in Milz und Keimdrüsen (Hoden, Eierstöcken), sowie schwächer in Thymus und Darm. Eine Hybridisierung eines Master-Blots (Dotblot mit RNA aus zahlreichen Geweben (Human RNA Master Blot™, Clontech) bestätigte dieses Ergebnis und zeigte auch eine starke Expression in Plazenta-Gewebe.

Die Hybridisierung wurde für 16 Stunden unter stringenten Bedingungen (5xSSC, 50 mM Na-Phosphat pH 6.8, 50 % Formamid, 100 µg/ml Hefe-RNA) bei 42°C durchgeführt. Die Blots wurden stringent gewaschen (65°C, 0,2XSSC, 0.1 % SDS) und einem Fuji BAS2000 Phosphoimager ™ exponiert

### Beispiel 3:

Eine cDNA-Bibliothek aus humaner Milz, kloniert in dem Bakteriophagen Lambda gt10 (Human Spleen 5' STRETCH PLUS cDNA, Clontech) wurde mit dieser Sonde durchsucht und ein Lambda-Klon identifiziert. Die in diesem Klon inserierte cDNA mit einer Länge von 1.6kb wurde mittels PCR (Expand™ Long Template PCR System, Boehringer Mannheim GmbH, Sandhofer Straße 116, 68305 Mannheim) amplifiziert wobei die vektorspezifischen Primer Nr. 207608 + Nr. 207609 (Tabelle 6) verwendet wurden (flankierend der EcoRI-Klonierungsstelle) und das erhaltene PCR-Fragment sequenziert. Dieser Klon enthielt das 5' Ende der cDNA und erweiterte die bekannte cDNA Sequenz auch nach 3'. Ausgehend von den neuen Teilsequenzen der cDNA wurden neue Primer für die RACE-PCR entwickelt (Nr. 232643, Nr. 232644, Nr. 233084, Tabelle 6). Zusammen mit einer verbesserten Thermocyclertechnik (PTC-200 von MJ-Research, Biozym Diagnostik GmbH, 31833 Hess. Oldendorf) mit deutlich besseren Leistungsdaten (Heiz- und Kühlrate) wurde ein 3'-RACE PCR-Produkt amplifiziert wobei die Primer Nr. 232644 bzw. Nr. 232643 und AP1 verwendet wurden und in den Vektor pCR2.1 (Invitrogen, De Schelp 12, 9351 NV Leek, Niederlande) kloniert. Das 3'-RACE PCR-Produkt wurde Sequenziert und auf diese Weise das 3'Ende der cDNA identifiziert. Eine RACE-Amplifikation nach 5' (Primer Nr. 131990 bzw. Nr. 233084 und AP1) erweiterte das 5' Ende der cDNA um wenige Nukleotide und bestätigte den im identifizierten Lambda-Klon gefundenen Aminoterminus des H-SemaL.

### Beispiel 4:

Ausgehend von einem kurzen murinen EST (Zuordnungs-Nr. AA260340) und einem daraus abgeleiteten Primer Nr. 260813 (Tabelle 6) und dem H-SemaL spezifischen Primern Nr. 121234 (Tabelle 6) wurde mittels PCR (Bedingungen: Taq52-60) ein DNA-Fragment mit einer Länge von ca. 840 bp muriner cDNA amplifiziert und in den Vektor pCR2.1 kloniert. Das dieses DNA-Fragment enthaltende Gen wurde M-SemaL genannt. Mit dem erhaltenen M-SemaL DNA-Fragment eine cDNA-Bank aus Mäuse-Milz (Mouse Spleen 5' STRETCH cDNA, Clontech) untersucht, wobei bereits mehrere Klone identifiziert werden konnten.

PCR (Taq60-30) mit den Primern Nr. 260812, Nr.260813 aus muriner, endothelialer cDNA lieferte ein PCR-Fragment mit einer Länge von 244 Basenpaaren. Die PCR-Ergebnisse zeigten, daß eine deutliche basale Expression in murinen Endothelzellen vorhanden ist, welche nach Stimulation mit dem Zytokin Interferon-γ und Lipopolysacchariden zurückgeht.

### Beispiel 5:

Untersuchungen zur Chromosomalen Lokalisation wurden mittels Fluoreszenz in-situ Hybridisierung (FISH) durchgeführt. Dazu wurden Metaphase Chromosomen von Mensch und Maus, ausgehend von einer humanen Blutprobe bzw. der Mauszellinie BINE 4.8 (Keyna et al. (1995) J. Immunol. 155, 5536-5542), hergestellt (Kraus et al. (1994) Genomics 23, 272-274). Die Objektträger wurden mit RNase und Pepsin behandelt (Liehr et al. (1995) Appl. Cytogenetics 21, 185-188). Für die Hybridisierung wurden 120 mg humane Nick-translatierte Semaphorin-Probe bzw. 200 mg einer entsprechenden Mausprobe verwendet. Die Hybridisierung wurde jeweils in Gegenwart von 4.0 µg COT1-DNA und 20 µg STD bei 37°C (3 Tage) in einer befeuchteten Kammer durchgeführt.

Die Objektträger wurden mit 50% Formamid/2x SSC (3 mal je 5 min bei 45°C) und dann mit 2 x SSC (3 mal je 5 min bei 37°C) gewaschen und die biotinylierte Probe mit dem FITC-Avidin-System (Liehr et al. (1995)), detektiert. Die Objektträger wurden mit Hilfe eines Fluoreszenz-Mikroskops ausgewertet. Es wurden 25 Metaphasen/Probe ausgewertet, wobei jedes Experiment doppelt durchgeführt wurde. Es zeigt sich, daß H-SemaL auf Chromosom 15q23 lokalisiert ist Chromosomal benachbart liegen der Locus für das Bardet-Biedl-Sydrom und Tay-Sachs Erkrankung (Hexosaminidase A).

### Beispiel 6:

Die genomische Intron-Exon Struktur des H-SemaL Gens ist zum größten Teil aufgeklärt.

Genomische DNA Fragmente wurden ausgehend von 250 mg humaner genomischer DNA, die aus PHA stimmulierten pheripheren Lymphozyten (Blut) isoliert worden waren, amplifiziert. Kürzere Fragmente wurden mit Ampli Taq^{R} (Perkin Elmer), längere Fragmente mit dem Expanded Long Templat PCR System^{R} (Boehringer Mannheim) amplifiziert.

Durch PCR-Amplifikation konnte bisher fast der vollständige genomische Locus des H-SemaL kloniert und charakterisiert werden. Insgesamt konnten bereits mehr als 8888 bp der genomischen Sequenz bestimmt werden und so die Intron-Exon-Struktur des Gens weitgehend aufgeklärt werden.

### Beispiel 7:

Expressionsklonierungen:
Da kein kompletter Klon des Semaphoringens aus der Lambda-gt10 cDNA-Bank isoliert werden konnte, und auch mittels PCR ein vollständiger Klon nicht zu erhalten war, wurde der kodierende Bereich der cDNA in 2 überlappenen Subfragmenten mittels PCR (XL62-6) mit Hilfe der Primer Nr. 240655 und Nr. 121339 für das N-terminale DNA-Fragment, sowie den Primern Nr. 240656 (enthält HindIII und Pmel Schnittstellen) und Nr. 121234 für das C-terminale DNA-Fragment amplifiziert. Die erhaltenen DNA-Fragmente (Subfragmente) wurden in den Vektor pCR21 kloniert. Die beiden Subfragmente wurden komplett sequenziert und schließlich die vollständige H-SemaL cDNA durch Insertion eines 0.6kb C-terminalen Sstl-HindIII Restriktions-Fragments in das mit den Restriktionsenzymen Sstl und HindIII geschnittene, das N-terminale DNA-Fragment enthaltende Plasmid, hergestellt. Aus diesem Plasmid pCR2.1-H-SemaL (gemäß Sequenz in Tabelle 7, SEQ ID NO. 34) wurde das komplette Gen mittels der EcoRI-Schnittstelle (in pCR2.1) und HindIII-Schnittstelle (in Primer Nr. 240656, Tabelle 6) herausgeschnitten und in einen entsprechend geschnittenen, konstitutiven Expressionsvektor pCDNA3.1(-)MycHisA (Invitrogen) ligiert. Aus dem resultierenden rekombinanten Plasmid pCDNA3.1(-)H-SemaL-MycHisA (gemäß Sequenz in Tabelle 8) wurde das EcoRI-Apal Fragment (ohne Myc-His-Tag) herausgeschnitten und in den induzierbaren Vektor plND ligiert (Ecdysone-Inducible Mammalian Expression System, Invitrogen), der zuvor ebenfalls mit EcoRI-Apal geschnitten worden war. Das rekombinante Plasmid wurde mit plND-H-SemaL-EA (Sequenz gemäß Tabelle 11) bezeichnet Ein EcoRI-Pmel-Fragment (mit Myc-His-Tag) aus pCDNA3.1(-)H-SemaL-Myc-HisA (Sequenz gemäß Tabelle 9) wurde in einen mit EcoRI-EcoRV geschnittenen Vektor plND eingesetzt. Das rekombinante Plasmid wurde mit plND-H-SemaL-EE (Sequenz gemäß Tabelle 10) bezeichnet.
Ein Fusionsgen von H-SemaL mit Enhanced Green Fluorescent Protein (EGFP) wurde hergestellt durch Ligation des mit PCR amplifizierten EGFP-Leserahmens (aus dem Vektor pEGFP-C1 (Clontech), mit Hilfe der Primer Nr. 243068 + Nr. 243069, Taq52-60) in die Pmel-Schnittstelle des Plasmids pCDNA3.1(-)H-SemaL-MycHisA wodurch das Plasmid pCDNA3.1(-)H-SemaL-EGFP-MycHisA (Sequenz gemäß Tabelle 9) erhalten wurde.

In den Tabellen 7 bis 13 bedeuten kleine Buchstaben die Sequenz von H-SemaL, Teilen oder Derivaten derselben und große Buchstaben die Sequenz des Plasmids.

### Beispiel 8:

Zur Herstellung von H-SemaL spezifischen Antikörpern wurden cDNA-Fragmente von H-SemaL in prokaryotische Expressionsvektoren integriert, in E. coli exrimiert und die Semaphorin-Derivate aufgereinigt. Die Semaphorin-Derivate wurden als Fusionsproteine mit einem His-Tag exprimiert Dementsprechend wurden Vektoren verwendet, die die Sequenz für ein His-Tag enthalten und eine Integration des Semaphorin cDNA-Fragments im Leserahmen ermöglichten. Ein N-terminales 6xHistidin-Tag ermöglicht z.B. eine Aufreinigung mittels Nickel-Chelat-Affinitätschromalographie (Qiagen GmbH, Max-Volmer Straße 4, 40724 Hilden):
1. Der für die Aminosäuren 179-378 kodierende Teil der H-SemaL cDNA wurde mittels PCR mit den Primern Nr. 150788 und Nr. 150789 amplifiziert und dieses DNA-Fragment in den Vektor pQE30 (Qiagen), der zuvor mit den Restriktionsenzymen BamHI und HindIII geschnitten worden war, ligiert (Konstrukt pQE39-H-SemaL-BH (Sequenz gemäß Tabelle 12)).
2. Der für die C-terminalen Aminosäuren 480-666 kodierende Abschnitt der H-SemaL cDNA wurde mit den Restriktionsenzymen Sstl und HindIII aus dem Plasmid pCR 2.1 geschnitten und in den Vektor pQE31 (Qiagen), der zuvor mit Sstl und HindIII geschnitten worden war ligiert (Konstrukt pQE31-H-SemaL-SH (Sequenz gemäß Tabelle 13)).

Die korrekte Integration der Sequenzen im richtigen Leserahmen wurde durch DNA-Sequenzierung überprüft. Die Fusionsproteine, bestehend aus einem N-terminalen 6xHistidin-Taq und einem Teil des Semaphorins H-SemaL wurden mittels Ni²⁺-Affinitätschromatographie aufgereinigt. Die aufgereinigten Fusionsproteine wurden zur Immunisierung von verschiedenen Tieren (Hase, Huhn, Maus) benutzt

### Beispiel 9:

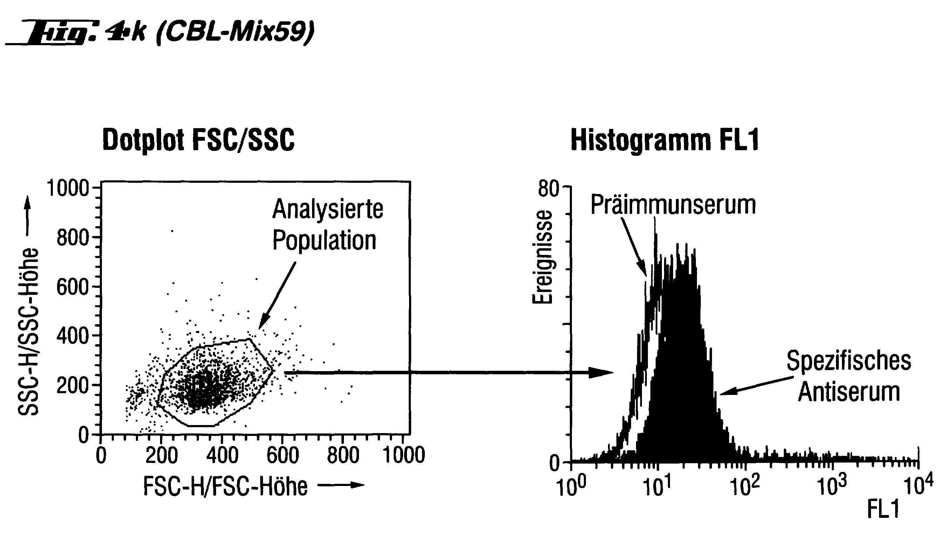

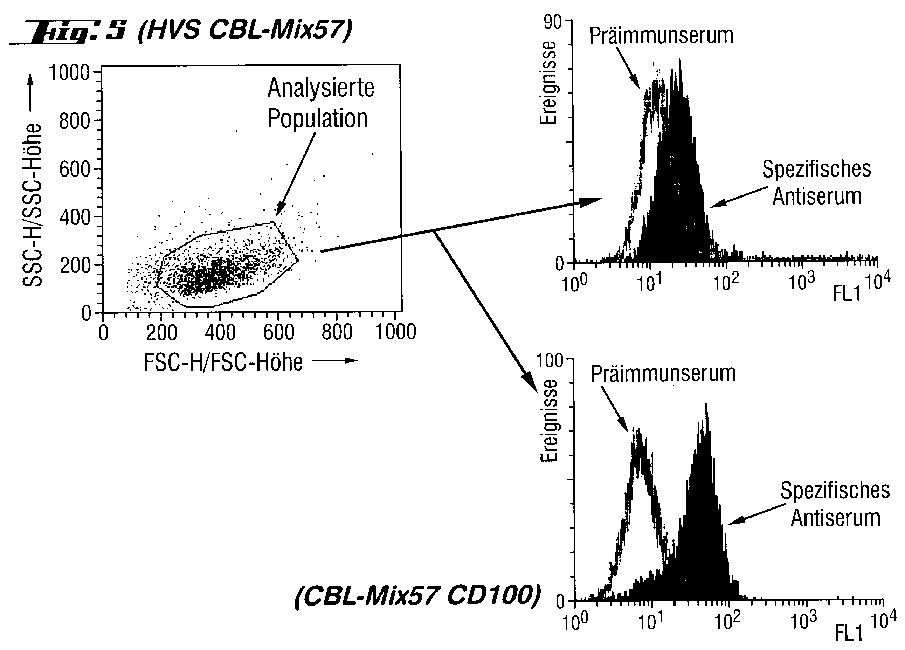

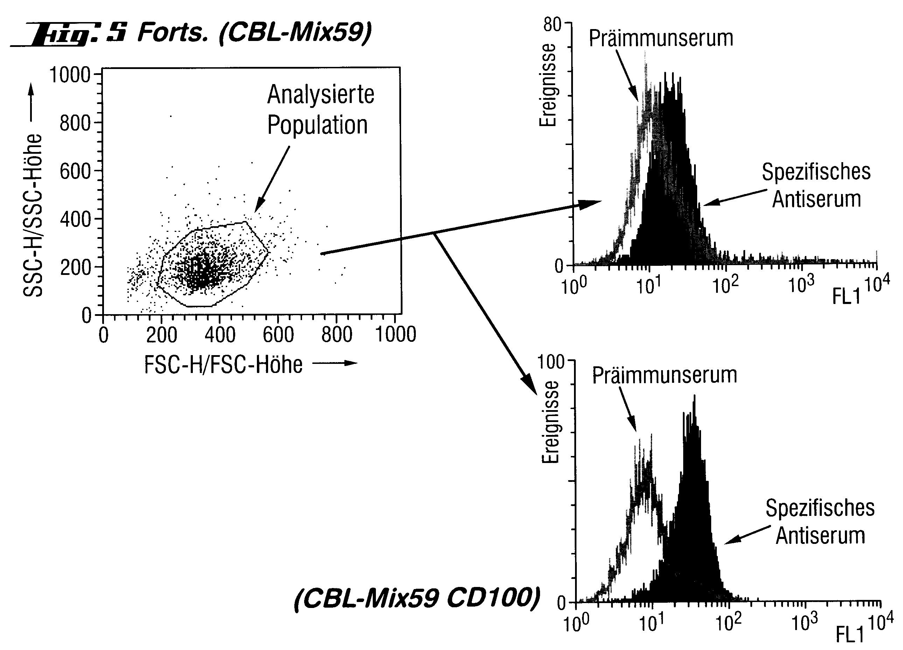

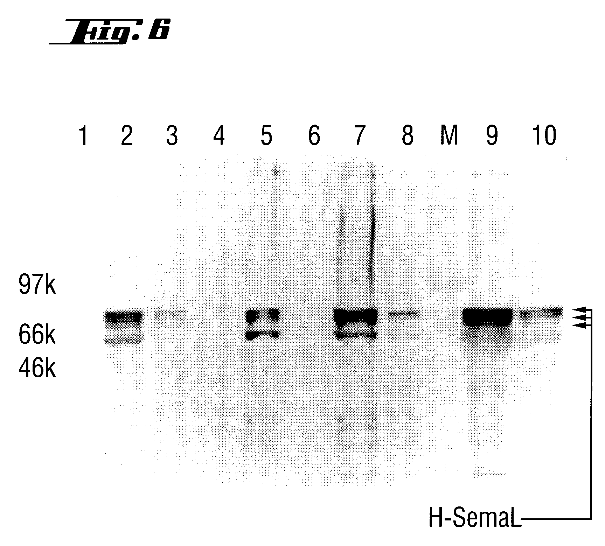

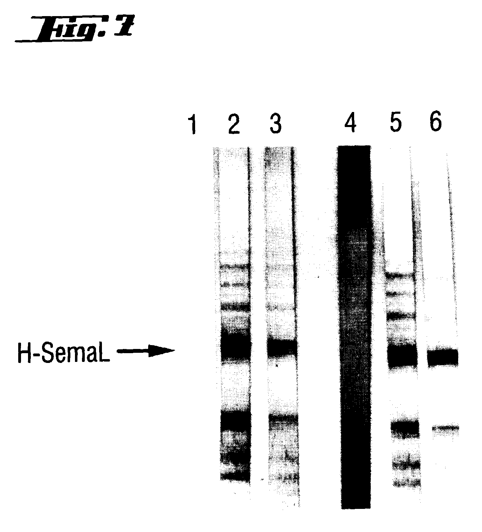

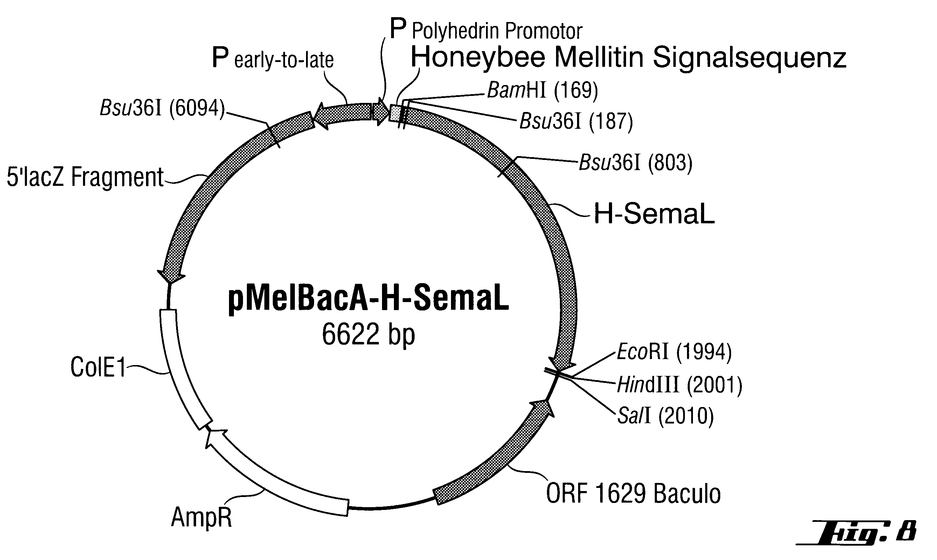

FACS-Analyse verschiedener Zelltypen (Figuren 4 und 5)
Die Zellen (ca. 0.2-0.5 x 10⁶) wurden mit FACS-Puffer gewaschen (Phosphate-buffered Saline (PBS) mit 5% fötalem Kälberserum (FCS) und 0.1% Na-Azid) und dann jeweils (auf Eis) für 1 Stunde mit den Antiseren inkubiert.
Als primäre Antikörper dienten für die Kontrolle (Overlay Hühner-Präimmunserum (1 : 50) und für den spezifischen Nachweis (Spezifische Färbung) ein H-SemaL spezifisches Hühner-Antiserum (1 : 50).
Das spezifische Antiserum mit Antikörpern gegen Aminosäuren (Aa) 179-378 (mit N-terminalem His-Tag) von H-SemaL wurde durch Immunisierung von Hühnern mit dem durch Ni-Chelat-Affinitãtschromatographie gereinigten Protein erzeugt (wie in Beispiel 8 beschrieben).
Als zweiter Antikörper wurde ein FITC-markierter anti-Huhn F(ab') Antikörper aus Kaninchen verwendet (Dianova Jackson Laboratories, Best.-Nr. 303-095-006, Hamburg, Deutschland) (1mg/ml).
Für die CD100-Färbung: wurde ein Rabbit-anti-Maus IgG, FITC-markiert verwendet. Der zweite Antikörper wurde jeweils in 1 : 50 Verdünnung in FACS-Puffer eingesetzt.
Dann wurden die Zellen gewaschen, in PBS resuspendiert und im FACS analysiert. Die FACS-Analyse wurde mit einem FACS-Trak Gerät (Becton―Dickinson) durchgeführt Prinzip: Eine Einzelzellsuspension wird in einen Messkanal vorbeigeleitet, dort werden die Zellen mit Laserlicht von 488nm bestrahlt und so Fluoreszenzfarbstoffe (FITC) angeregt. Gemessen werden Streulicht nach vorne (forward scatter, FSC: korreliert mit der Zellgröße), zur Seite (sideward scatter, SSC: korreliert mit dem Granulargehalt: bei unterschiedlichen Zelltypen unterschiedlich) und Fluoreszenz im Kanal 1 (FL 1) (für Wellenlängen im FITC Emissionsbereich, max. bei 530nm). Auf diese Weise wurden je 10000 Ereignisse (Zellen) gemessen.
Der Dotplot (Figuren 4a - k) (jeweils linke Abbildung): FSC gegen SSC (Größe gegen Granulargehalt/Streuung), darin eingegrenzt ist die im rechten Fenster (jeweils zugehörige rechte Abbildung) analysierte (einheitliche) Zellpopulation von ähnlicher Größe und Granulargehalt. Das rechte Fenster zeigt die Intensität von FL1 (X-Achse) gegen die Zahl der Ereignisse (Y-Achse), also eine Häufigkeitsverteilung.
Hierbei istjeweils das Ergebnis mit dem Kontrollserum (nichtgefüllte Kurve) dem Ergebnis der spezifischen Färbung (ausgefüllte Kurve) überlagert. Eine Verschiebung der Kurve für die spezifische Färbung gegenüber der Kontrolle nach rechts entspricht einer Expression von H-SemaL in den entsprechenden Zellen. Je weiter die Verschiebung, desto stärker ist die Expression.

Für FACS Analyse verwendete Zellinien:
a) Zellinie U937
   American Type Culture Collection ATCC; ATCC Nummer: CRL-1593 Name: U-937
   Gewebe: lymphoma; histiocytic; Monozytenähnlich
   Species: human;
   Hinterleger: H. Koren
b) Zellinie THP-1
   ATCC Nummer: TIB-202
   Gewebe: monocyte; acute monocytic leukemia
   Species: human
   Hinterleger: S. Tsuchiya
c) Zellinie K-562
   ATCC Nummer: CCL-243
   Gewebe: chronic myelogenous leukemia
   Species: human;
   Hinterleger H.T. Holden
d) Zellinie L-428
   DSMZ-Deutsche Sammlung von Mikroorganismen und Zellkulturen GmbH, DSMZ Nr: ACC 197
   Zelltyp: human Hodgkin's lymphoma
e) Zellinie Jurkat
   DSMZ-Deutsche Sammlung von Mikroorganismen und zellkulturen GmH, DSMZ Nr: ACC 282
   Zelltyp: human T cell leukemia
f) Zellinie Daudi
   ATCC Number: CCL-213
   Gewebe: Burkitt's lymphoma; B lymphoblast; B Zellen
   Species : human
   Hinterleger: G. Klein
g) Zellinie LCL
   EBV-transformierte lymphoblastoide B-Zellinie.
h) Zellinie Jiyoye (P-2003)
   ATCC Number: CCL-87
   Gewebe: Burkitt's lymphoma; B Zellen; B lymphocyte
   Species: human
   Hinterleger: W. Henle
i) CBL-Mix57
   humane T-Zellinie (isoliert aus Blut) transformiert mit rekombinantem H. Saimiri (Wild-typ ohne Deletion)
j) CBL-Mix59
   humane T-Zellinie (isoliert aus Blut) transformiert mit H. Samiri (Deletion von ORF71).

### Beispiel 10: Proteingel und Western-Blot

Sekretierbare humane SemaL (Aminosäuren 42-549 in Tabelle 4 (ohne Signalpeptid und ohne Transmembrandomäne) wurde in das Plasmid pMelBac-A (Invitrogen, De Sehelp, Leck, Niederlande, Cv 1950-20) kloniert und auf diese Weise das Plasmid pMelBacA-H-SemaL (Länge 6622bp) erzeugt (Tabelle 15,Figur 8). Das H-SemaL Derivat wurde im Baculovirus-System (Bac-N-Blue, Invitrogen) exprimiert. Die Expression wurde in den Insekten-Eizellen abgeleiteten Zellinien Sf9 (von Spodoptera flugiperda) und High Five™ (von Trichoplusia ni, U.S. Pat. No. 5,300,435, gekauft von Invitrogen) durch Infektion mit den rekombinanten, plaquegereinigten Baculoviren ausgeführt.

Die Expression wurde nach den Angaben des Herstellers durchgeführt.
Anschließend wurden die Proteine in einem Gel aufgetrennt und das H-SemaL Derivat im Western-Blot nachgewiesen. Die Detektion wurde mit dem H-SemaL spezifischen Hühnerantiserum (vergl. Beispiel 8 und Figur 7) (Verdünnung: 1:100) durchgeführt. Der spezifische Hühnerantikörper wurde mit anti-IgY-HRP Konjugat (Verdünnung: 1:3000, vom Kaninchen; Dianova Jackson Laberatories) nach Angaben des Herstellers nachgewiesen.

### Beispiel 11: Herstellung von pMelBacA-HSEMAL

Der rekombinante Vektor (pMelBacA-HSEMAL, 6622bp) wurde hergestellt, indem ein entsprechendes DNA-Fragment, das für die Aminosäuren 42-649 von H-SemaL kodiert, in den Vektor pMelBacA (4,8 kb, Invitrogen) kloniert wurde (vergl. Annotation zu pMelBacA-H-SemaL). Die Klonierung erfolgte über BamHI und EcoRI in frame hinter die in dem Vektor vorliegende Signalsequenz ( 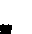 honeybee melittin signal sequence"). Ein entsprechendes H-SemaL DNA-Fragment wurde mit dem Primerpaar H-Sema 1 baculo 5' und H-Sema 1 baculo 3' amplifiziert.

Primer zur Amplifikation (TaKaRa Ex Taq-Polymerase) und Klonierung:
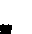 H-Sema 1 baculo 5' 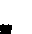 zur Amplifikation ohne Signalsequenz und zur Einführung einer BamHI-Schnittstelle
5'-CCGGATCCGCCCAGGGCCACCTAAGGAGCGG-3' (SEQ ID NO: 43)
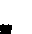 H-Sema 1 baculo 3' 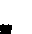 zur Amplifikation ohne Transmembrandomäne und zur Einführung einer EcoRI-Schnittstelle
5'-CTGAATTCAGGAGCCAGGGCACAGGCATG-3' (SEQ ID NO: 44).

### Abbildungen:

### Figur 1:

Gewebespezifische Expression von H-SemaL
A) Mehrfach-Gewebe Northern Blot (Clontech, Heidelberg, Deutschland). Von links nach rechts sind aufgetragen: Je 2 µg Poly-A-RNA pro Spur aus Milz, Thymus, Prostata, Hoden, Eierstöcken, Dünndarm, Dickdarm Mukosa, pheripheren (Blut-) Leukozyten. Größenstandards sind markiert.

Die Blots wurden unter stringenten Bedingungen mit einer 800 Basenpaaren langen H-SemaL Probe hybridisiert.

### Figur 2:

Schematische Darstellung der Klonierung der H-SemaL cDNA und der genomischen Organisation der H-SemaL kodierenden Sequenzen (H-SemaL Gen)
Oben: Lokalisation der EST-Sequenzen (Zugangsnummern; Lage der EST-Sequenzen ist relativ zur AHV-Sema Sequenz dargestellt).
Darunter: Amplifizierte PCR- und RACE-Produkte sowie die Position der cDNA Klone im Bezug auf die Lokalisation in der vollständigen H-SemaL cDNA und dem offenen Leserahmen (ORF) für das kodierte Protein.
Unten: Relative Position der Exons im H-SemaL Gen im Bezug auf die genomische Sequenz.
Die Position der verwendeten Oligonukleotid Primer ist durch Pfeile angezeigt

### Figur 3:

Phylogenetischer Baum: Erhalten durch mehrfaches Alignment der aufgeführten Semaphorin Sequenzen. Aufgrund der Gruppierung der Semaphorine in dem phylogenetischen Baum kann auf deren phylogenetische Verwandtschaft geschlossen werden.

### Figur 4:

FACS Analyse der H-SemaL Expression in verschiedenen Zellinien bzw. verschiedenen Zelltypen (vergl. Beispiel 8).

### Figur 5:

Vergleichende Analyse der CD 100 und H-SemaL Expression (vergl. Beispiel 9).

### Figur 6: Expression von sekretierbarem humanen SEMAL (H-SemaL) in HiFive und Sf3-Zellen (vergl. Beispiel 10).

(Aa 42-649 in pMelBac-A (Invitrogen) im Baculovirus-System (Bac-N-Blue, Invitrogen) Detektion mit spezifischem Hühner-Antiserum (1:100) und anti-IgY-HRP Konjugat (1:3000, vom Kaninchen, Jackson Lab.)
1,4,6 uninfizierte HiFive Zellen (serumfrei)
2,3,5,7,8 mit rekombinantem Baculovirus infizierte HiFive Zellen (serumfrei)
M Rainbow molecular weight marker (Amersham RPN756)
9,10 infizierte Sf9 Zellen (serumhaltiges Medium).

### Figur 7: Spezifität des Antiserums

Spuren 1-3: Huhn 1; Spuren 4-6: Huhn 2
Spuren 1 und 4: Präimmunserum
Spuren 2 und 5: 60. Immunisierungstag
Spuren 4 und 6: 105. Immunisierungstag

Immunisiert wurde mit den Aminosäuren 179-378 von H-SemaL (mit aminoterminalem His-Tag) (vergl. Beispiel 8, Punkt 1.)

### Figur 8: Abbildung der Plasmidkarte von pMelBacA-HSEMAL.

Das rekombinante Plasmid wurde wie in Beispiel 11 beschrieben, hergestellt.

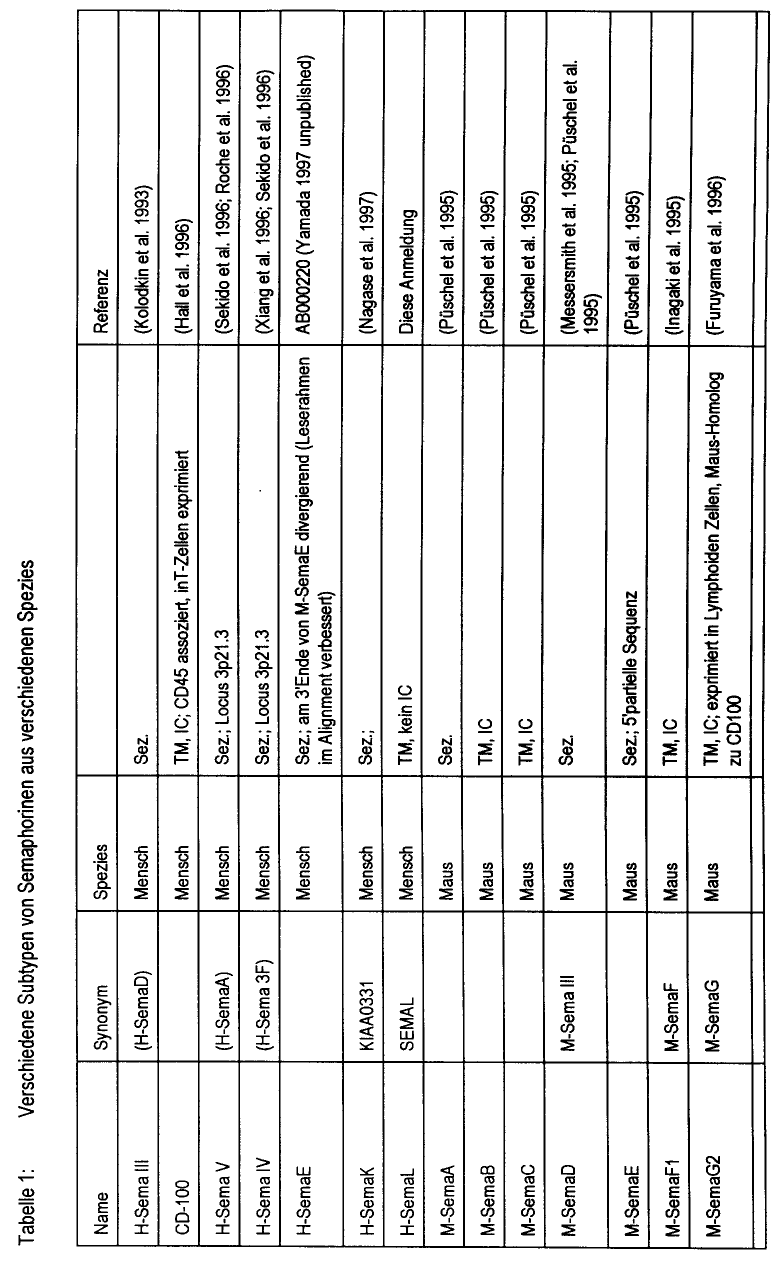

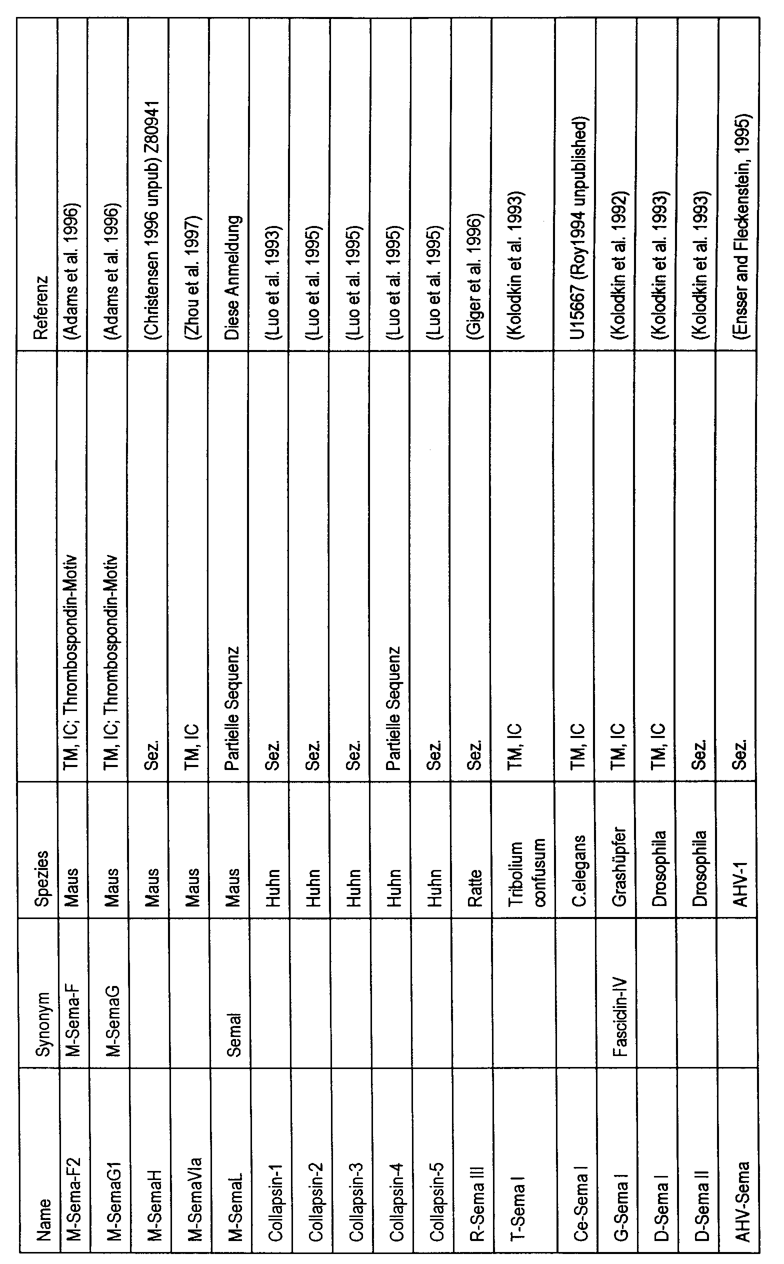

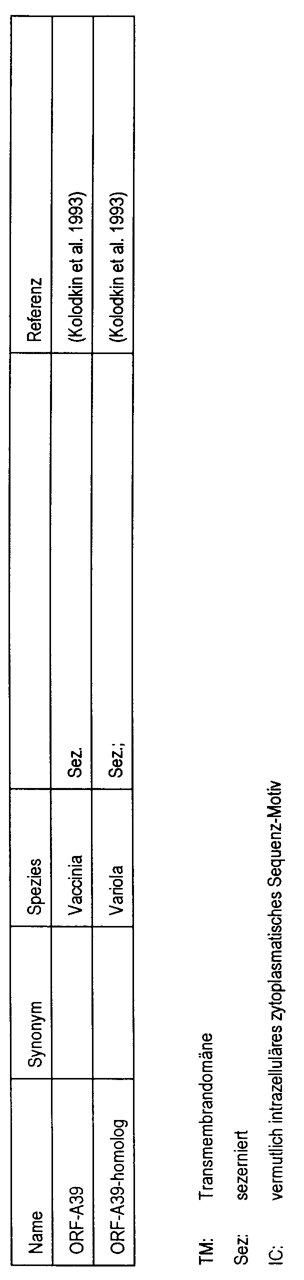

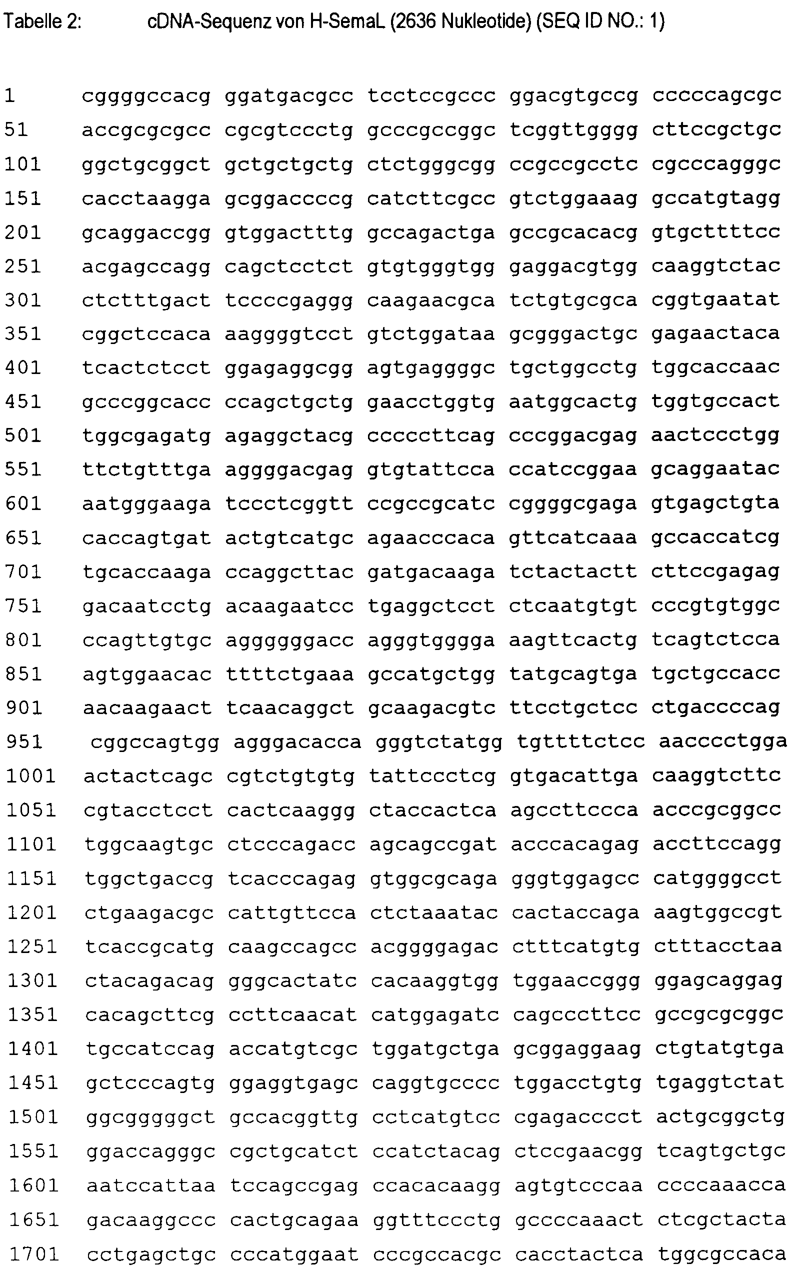

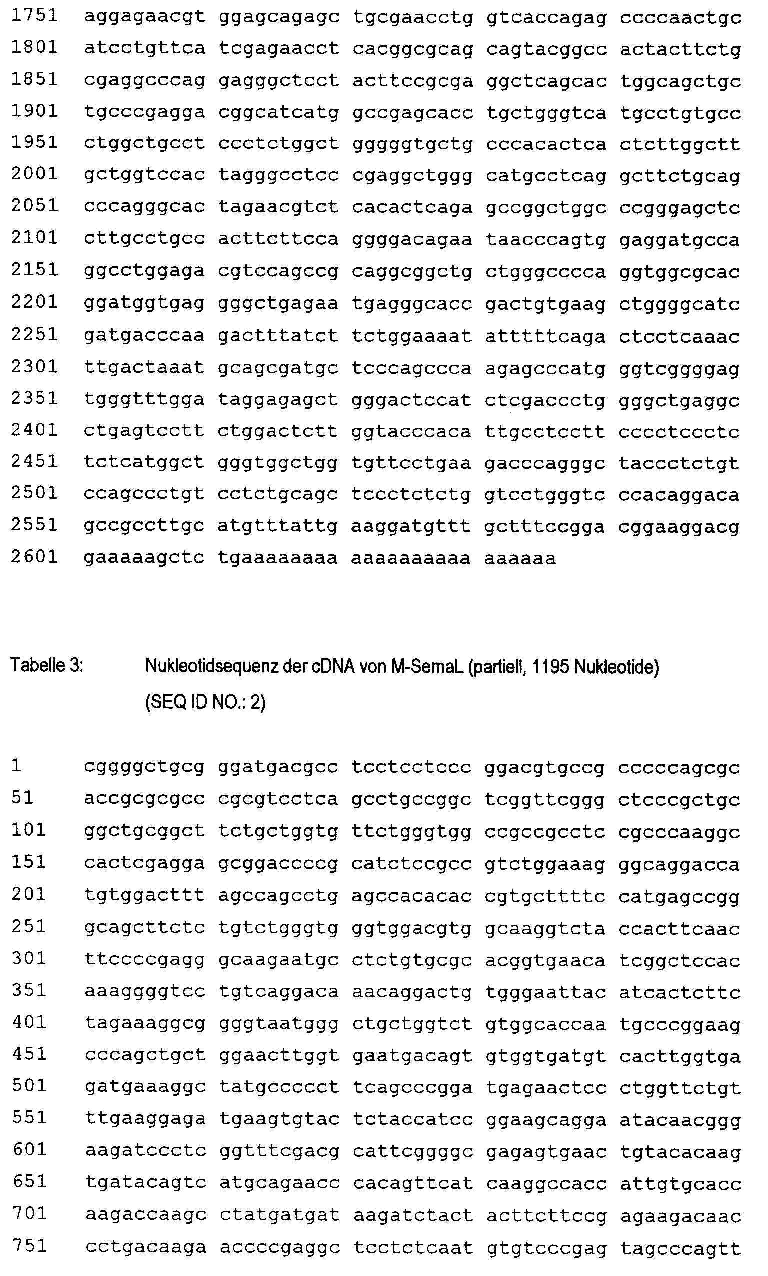

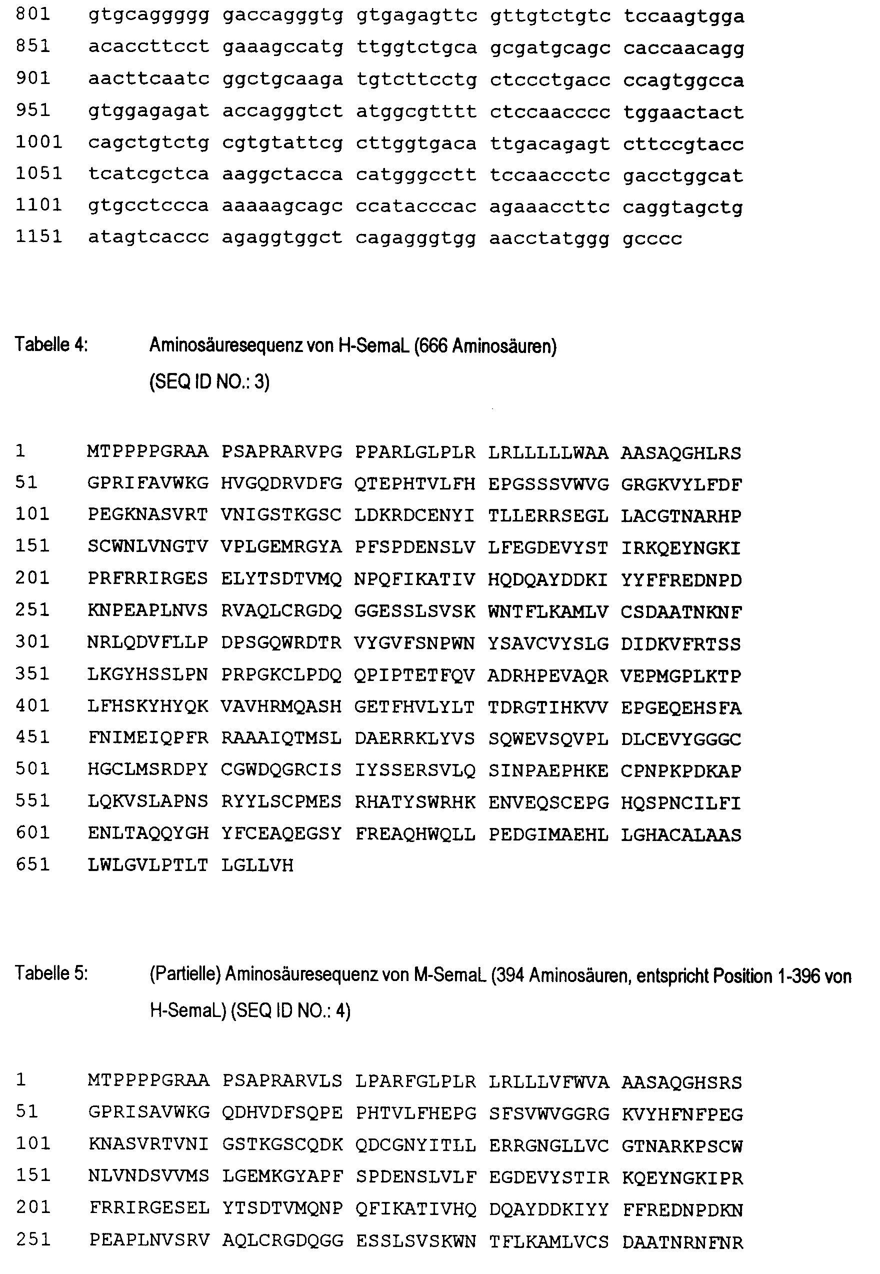

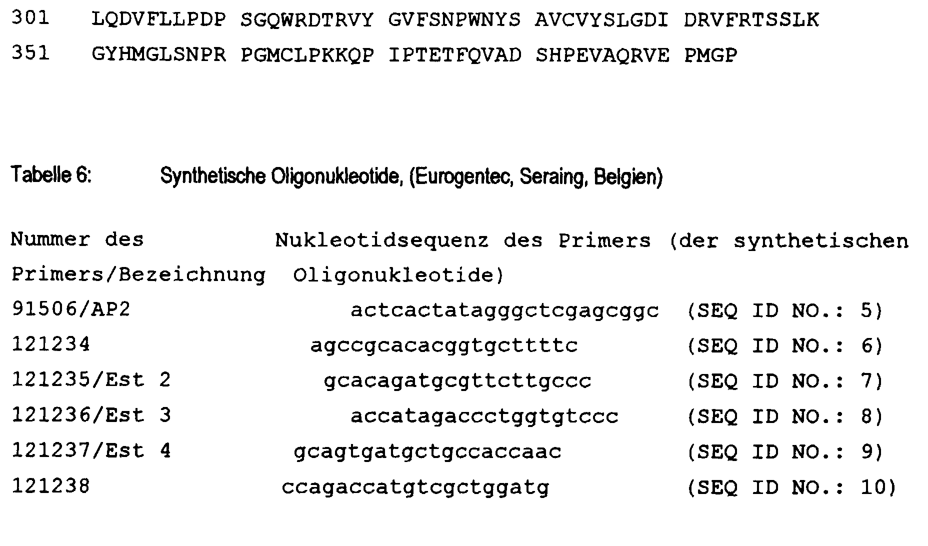

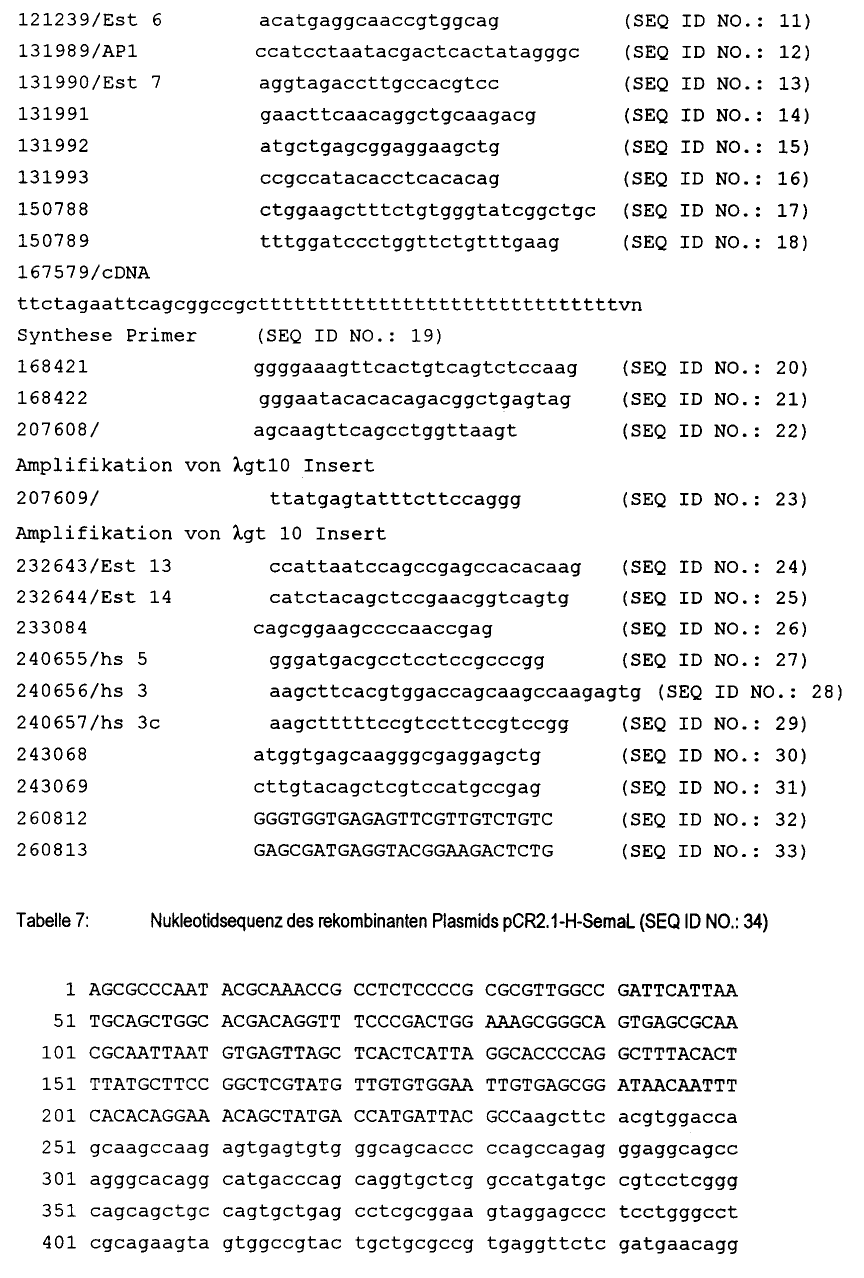

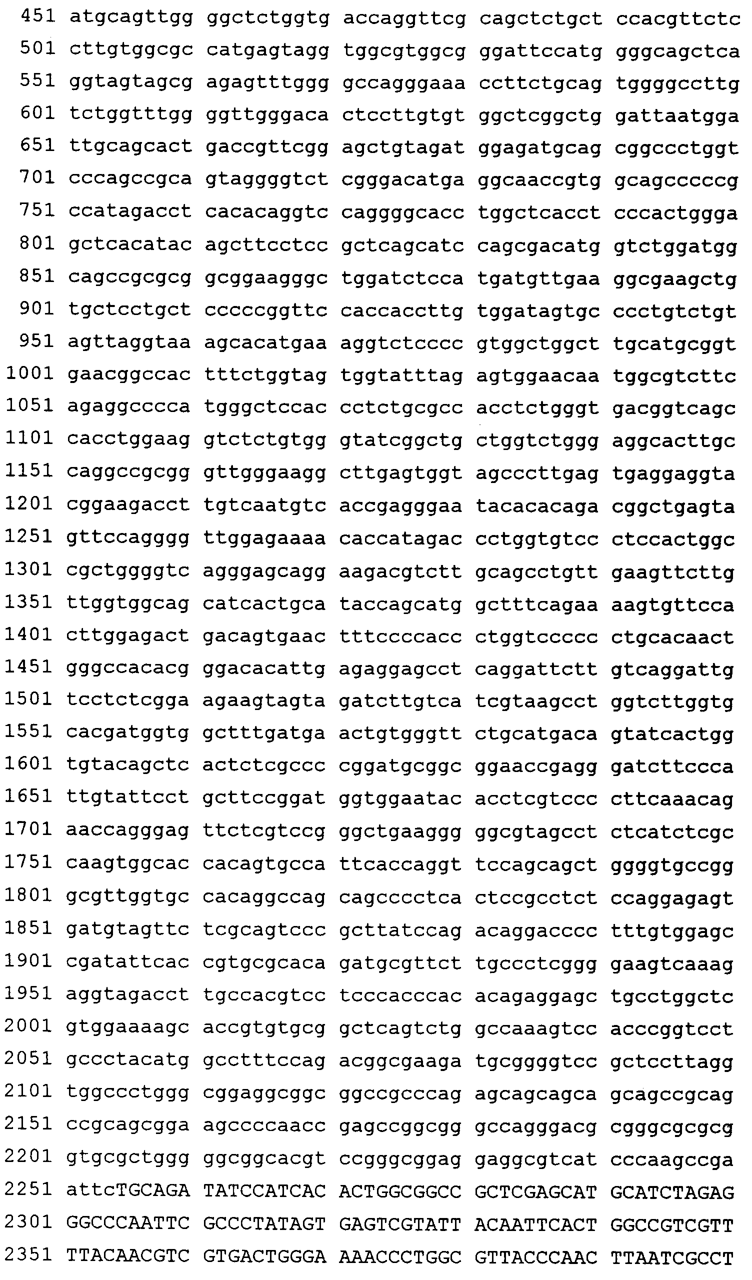

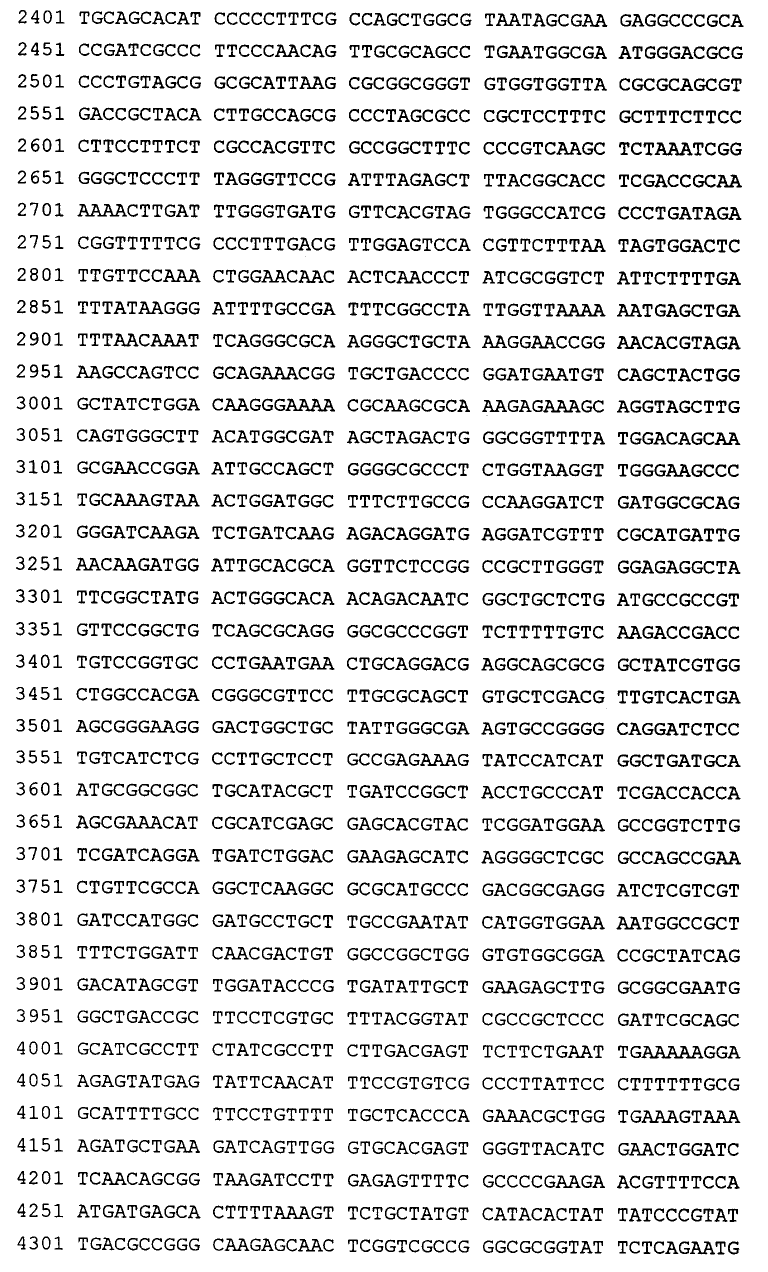

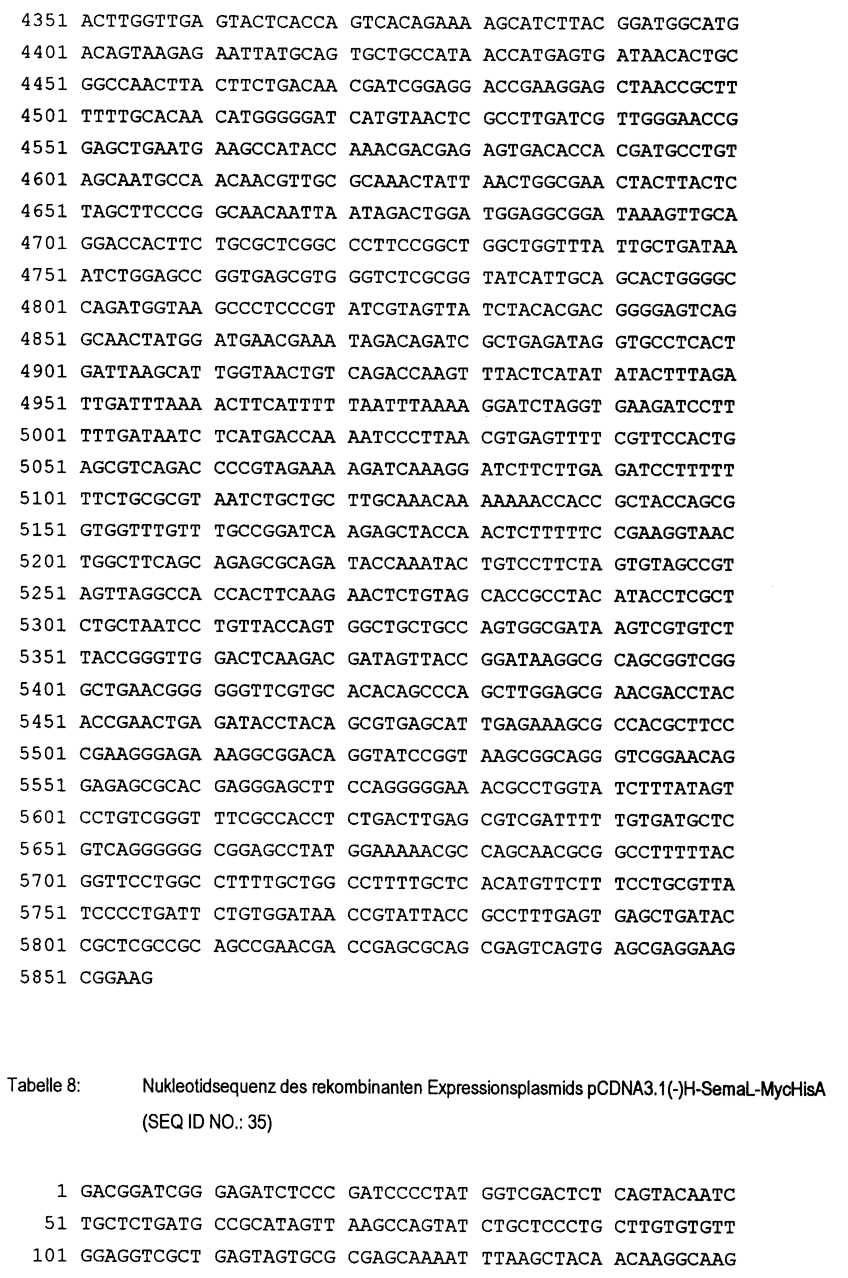

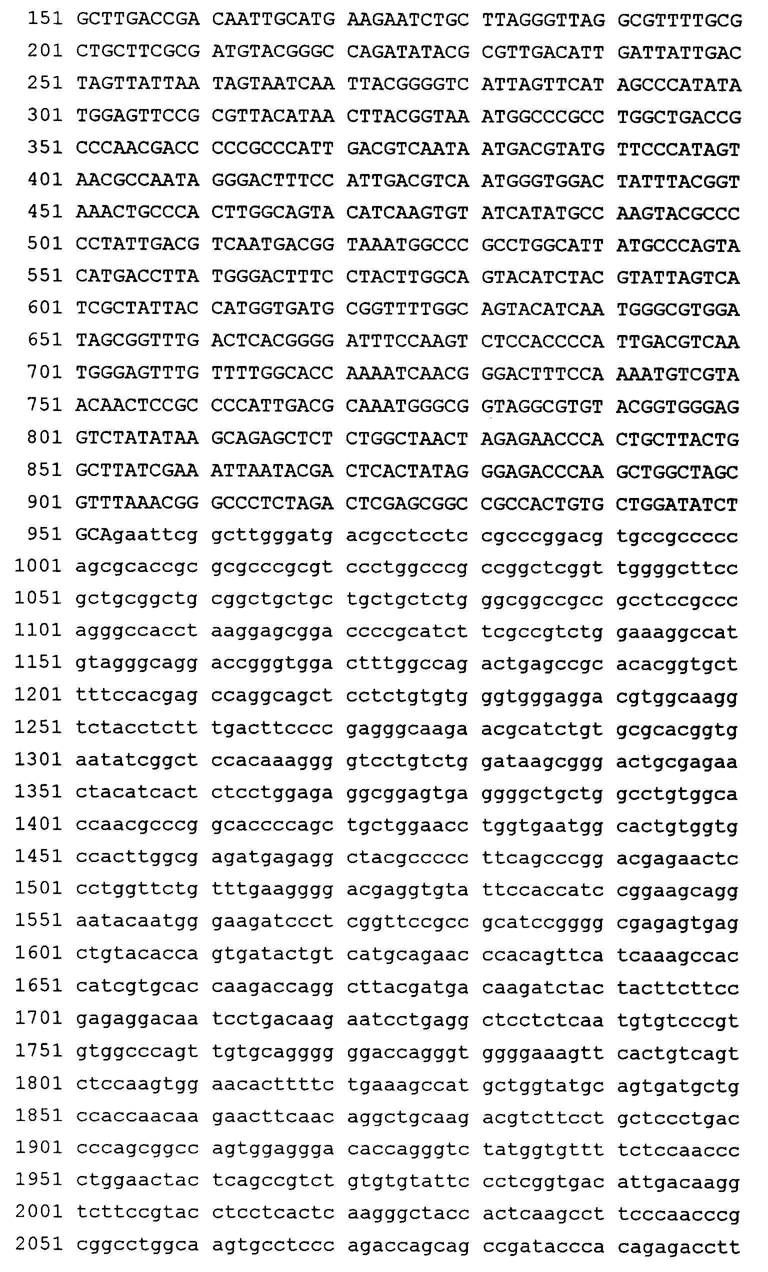

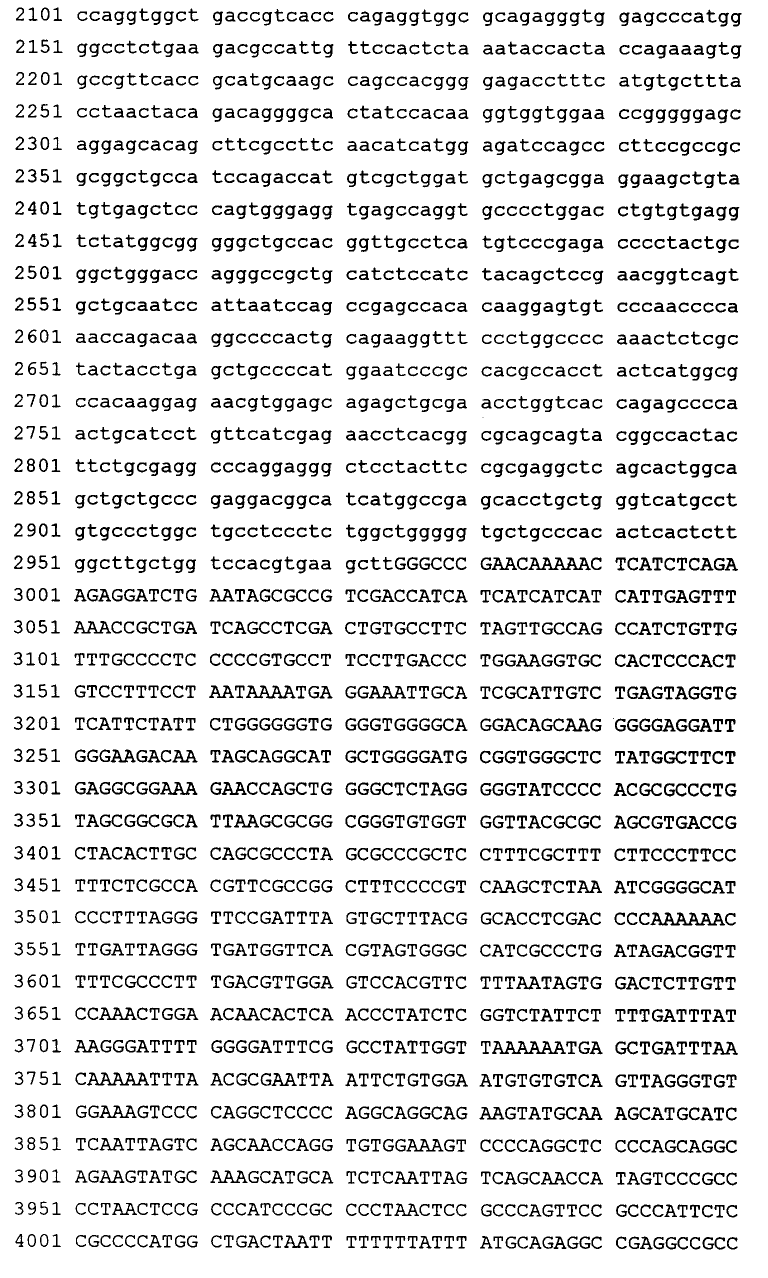

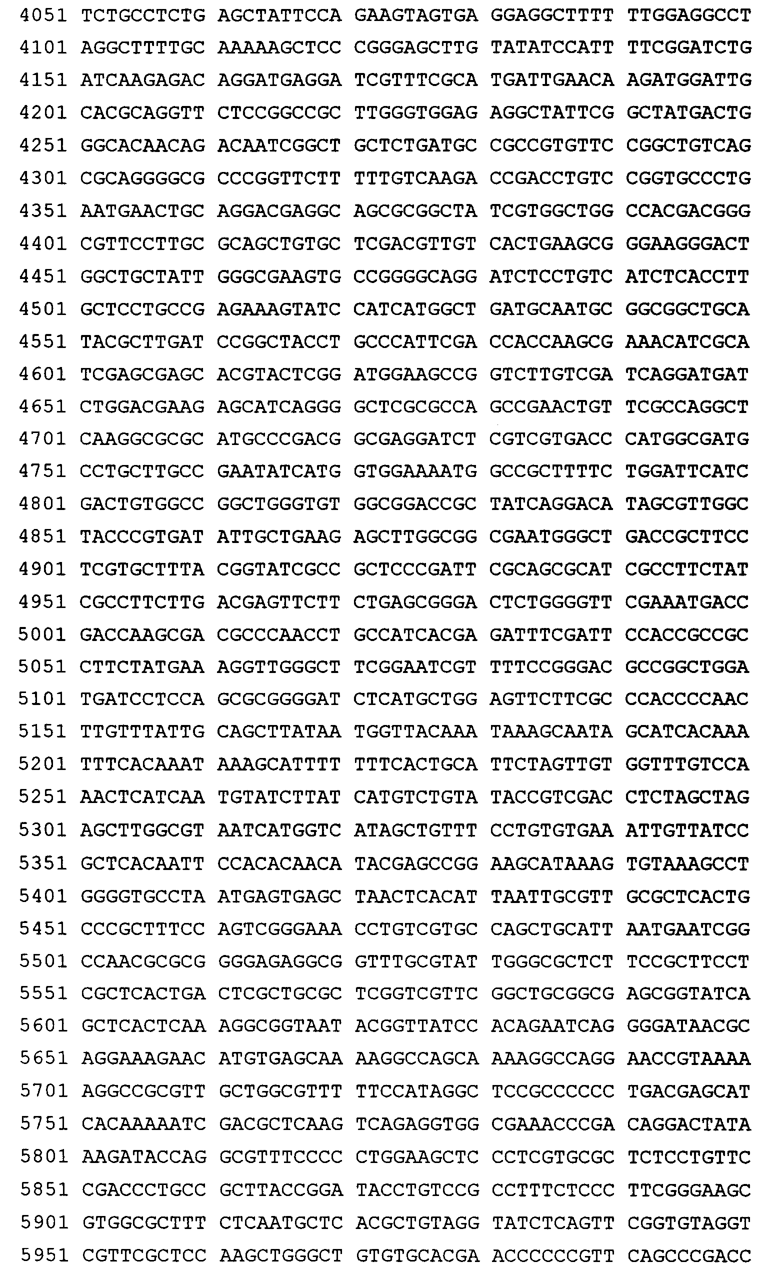

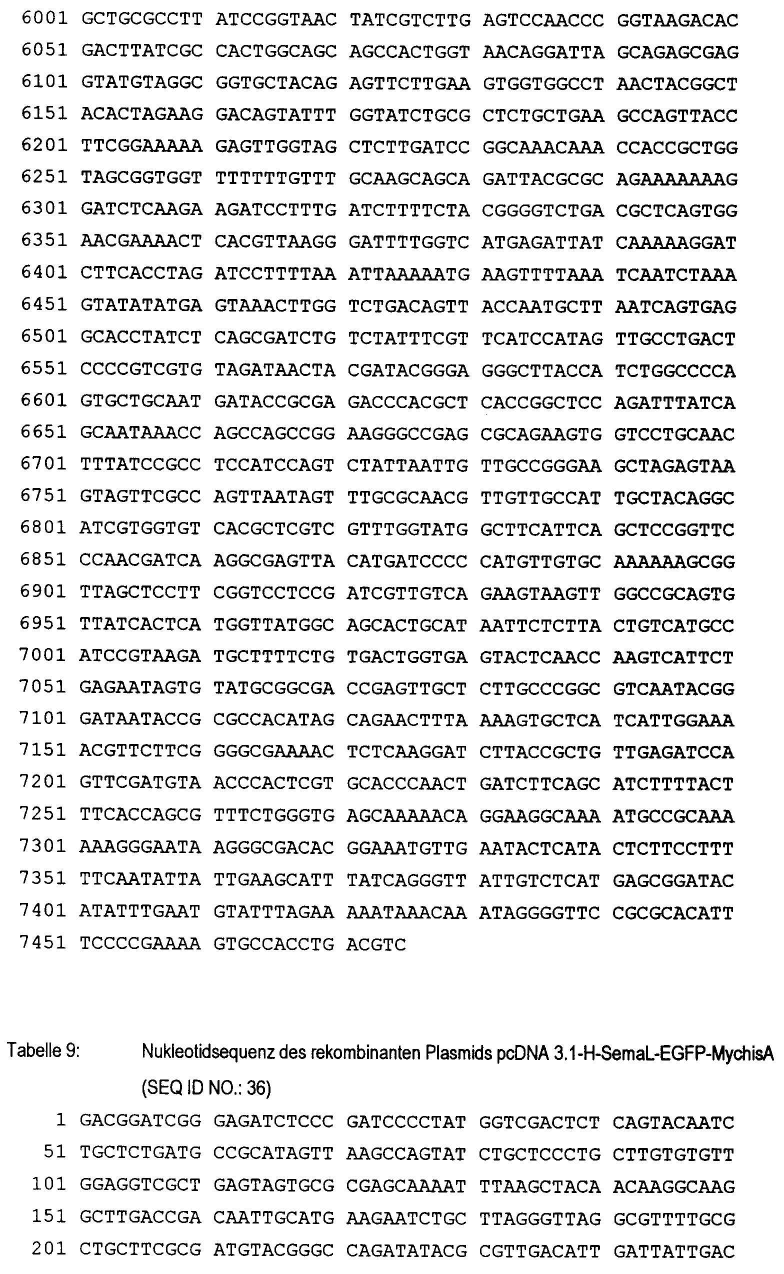

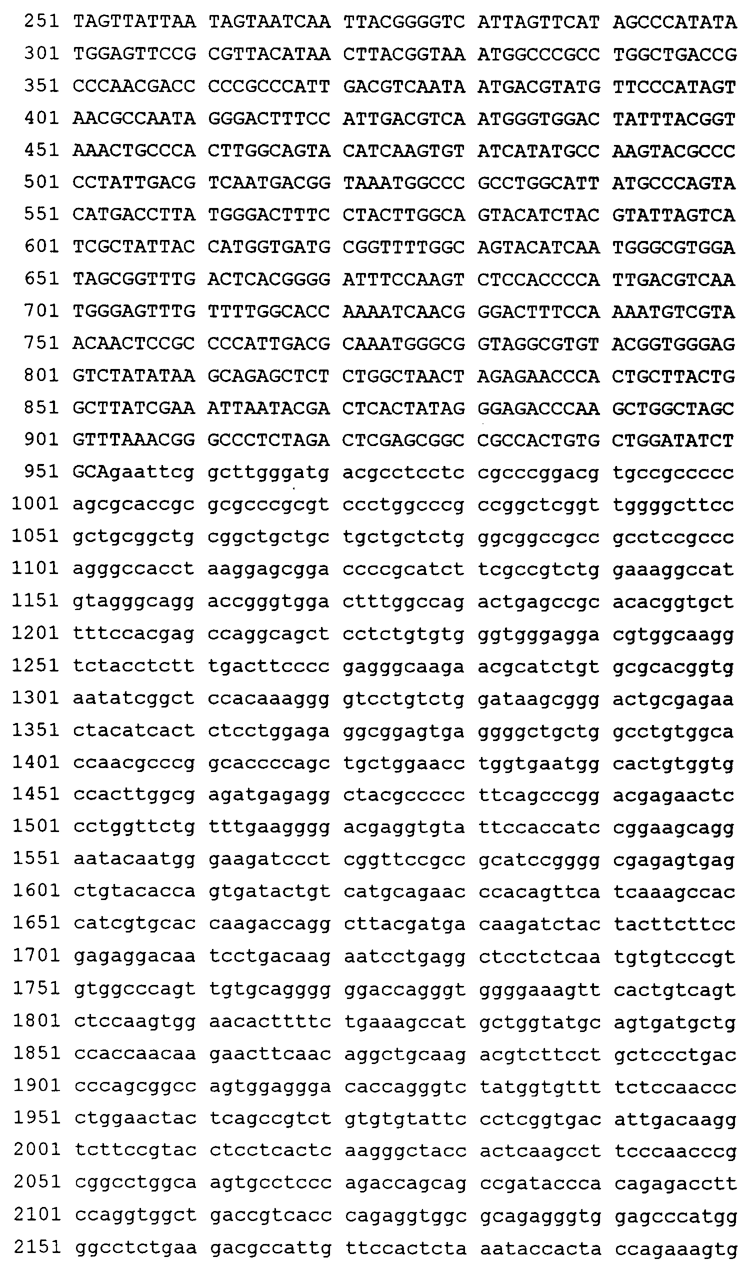

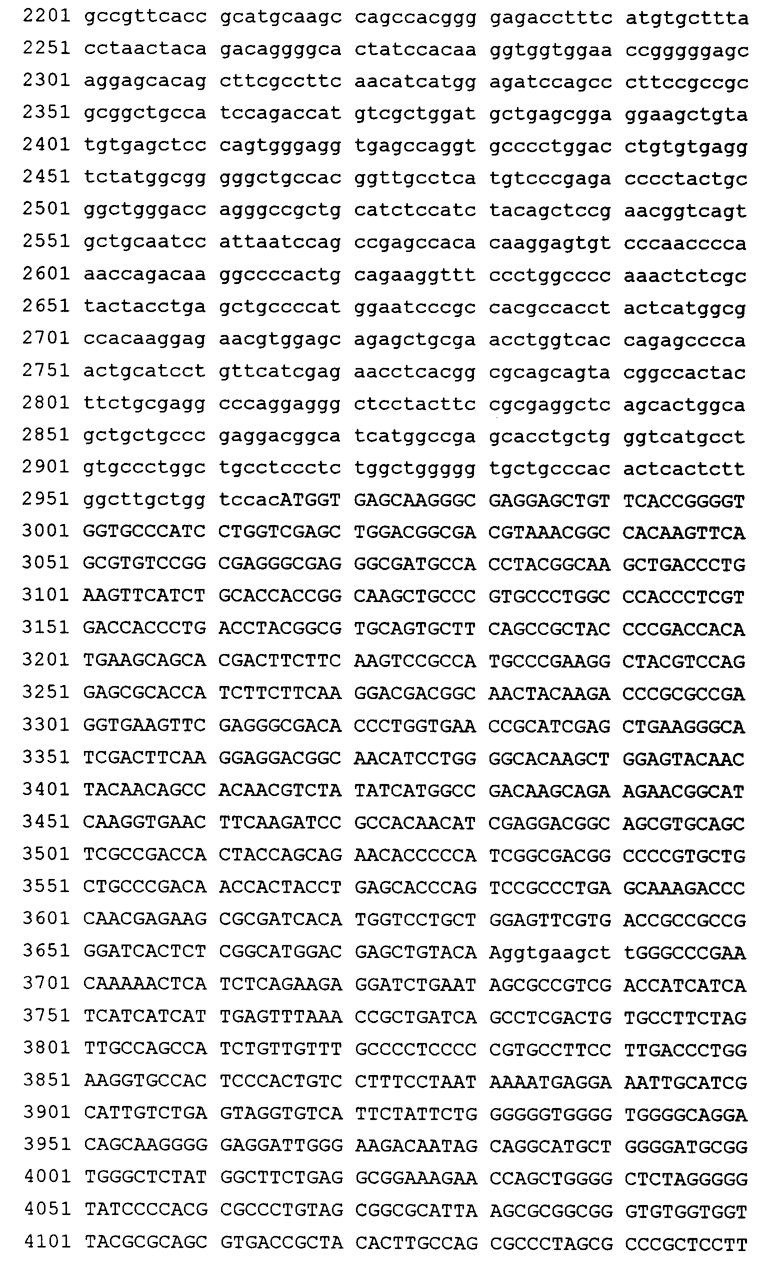

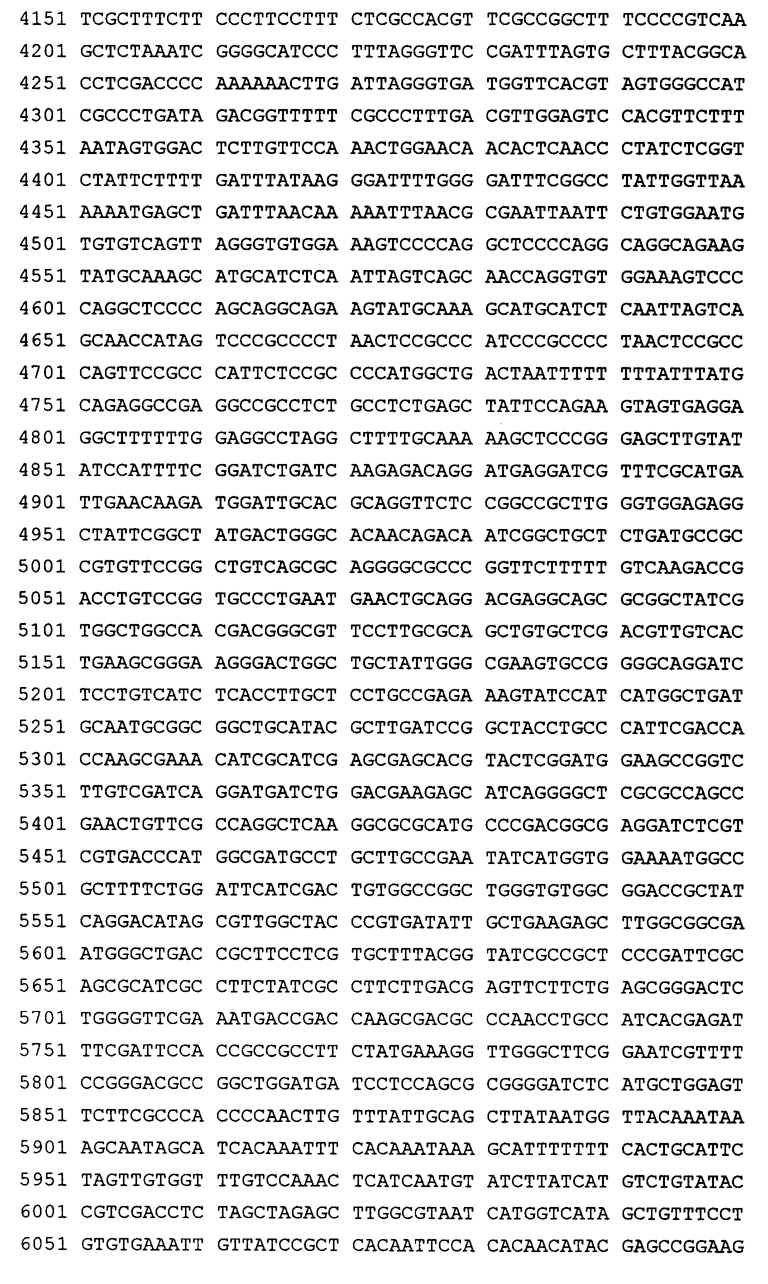

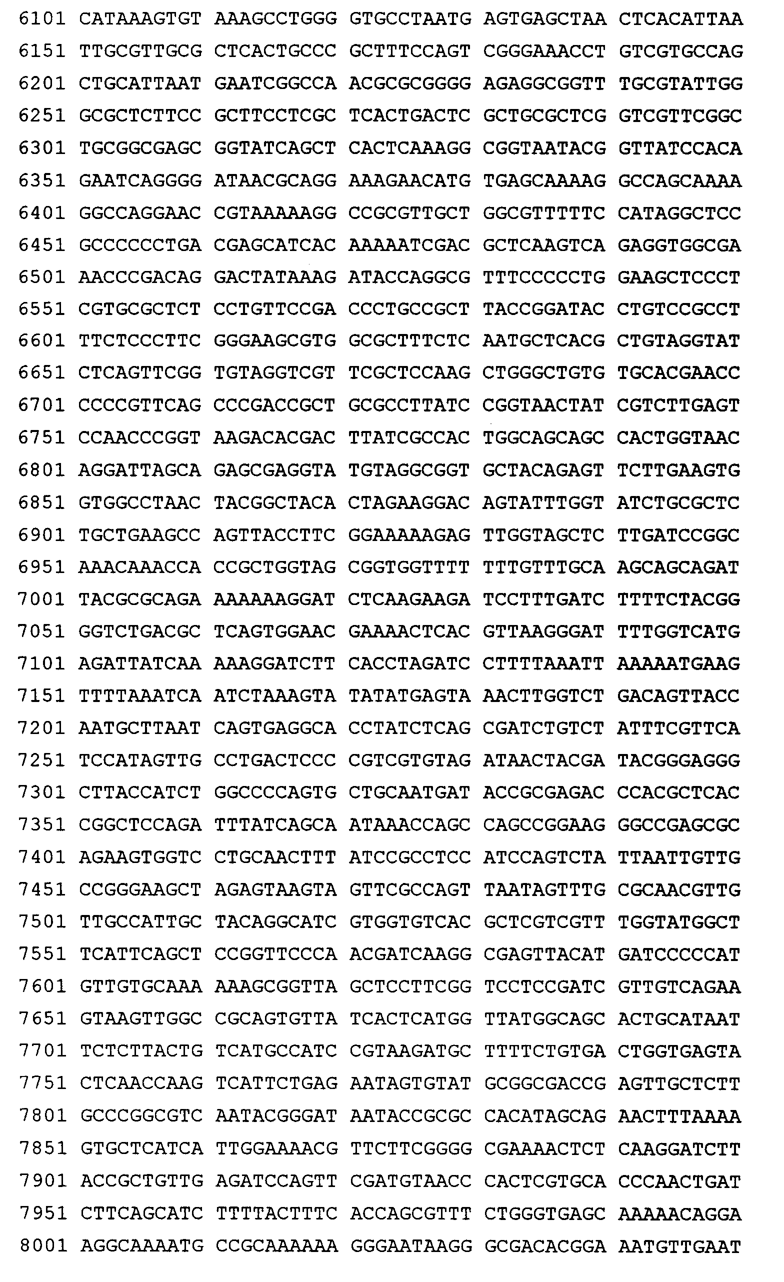

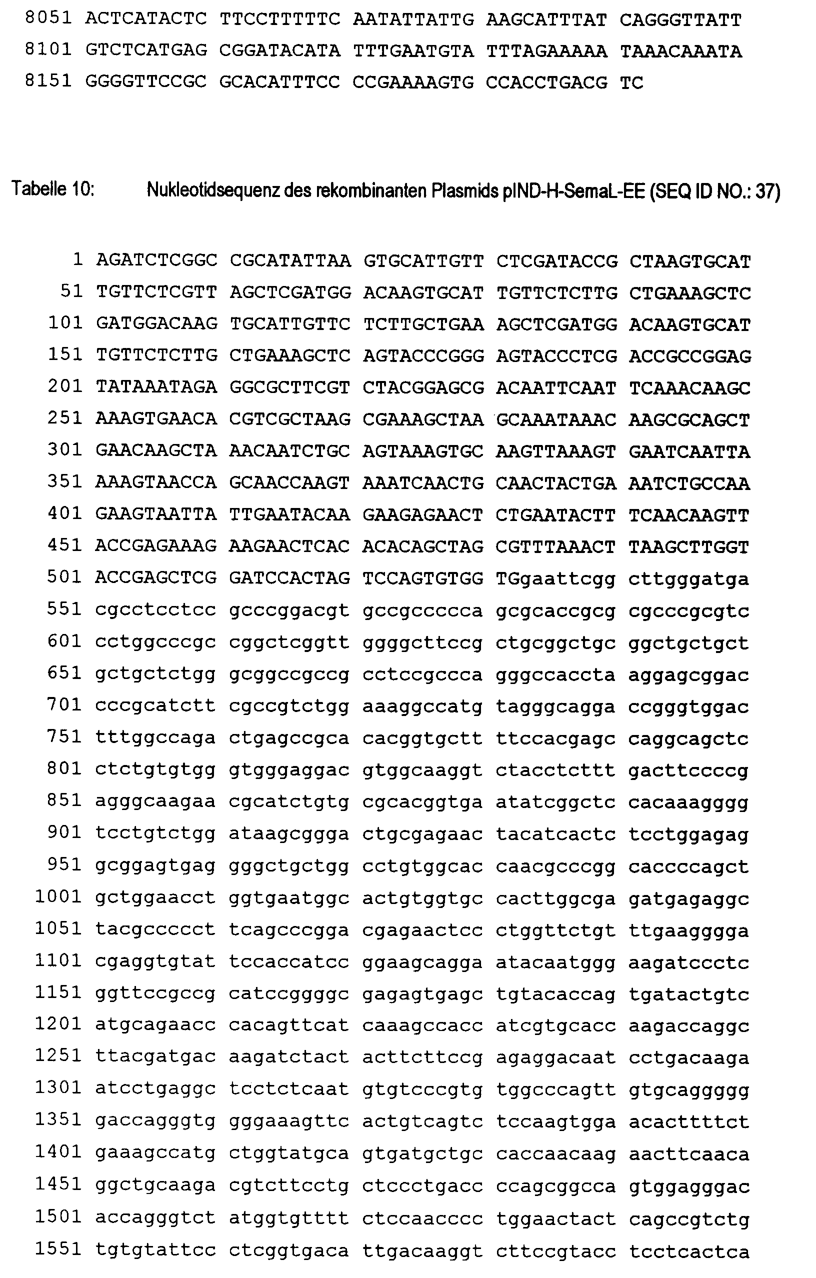

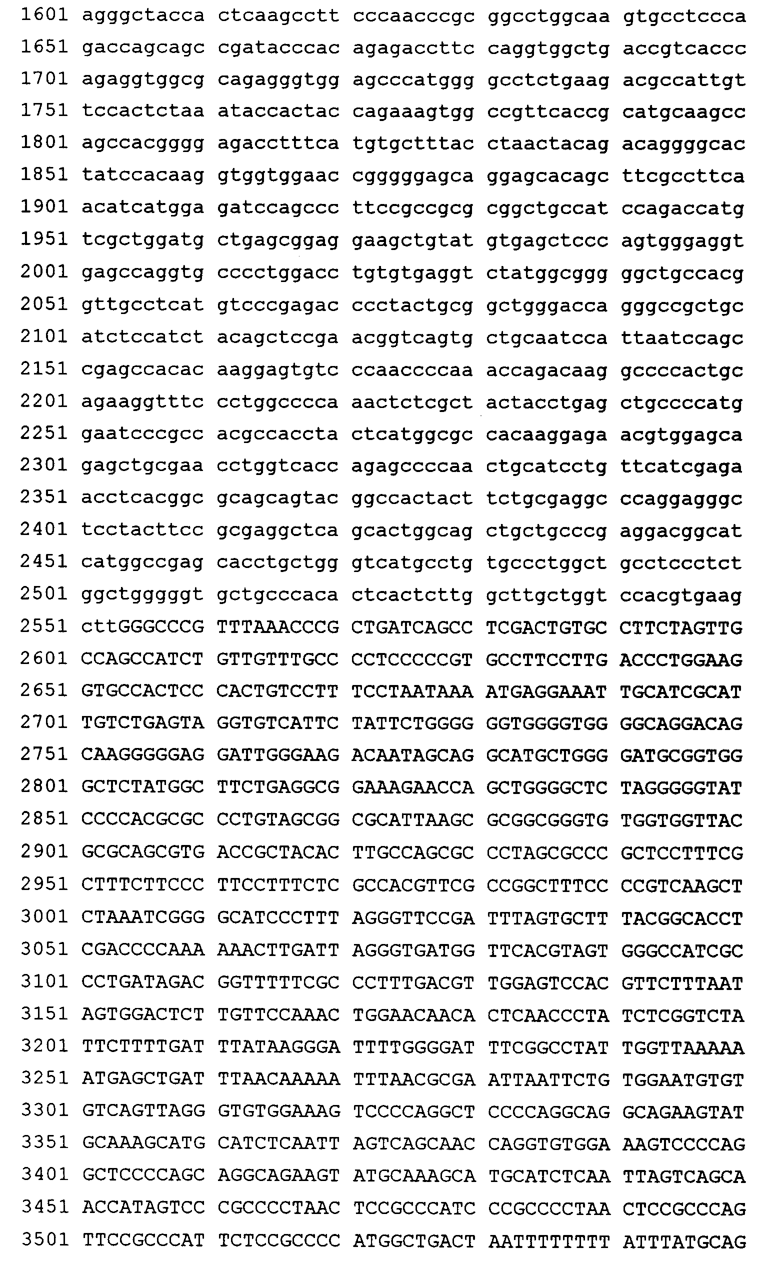

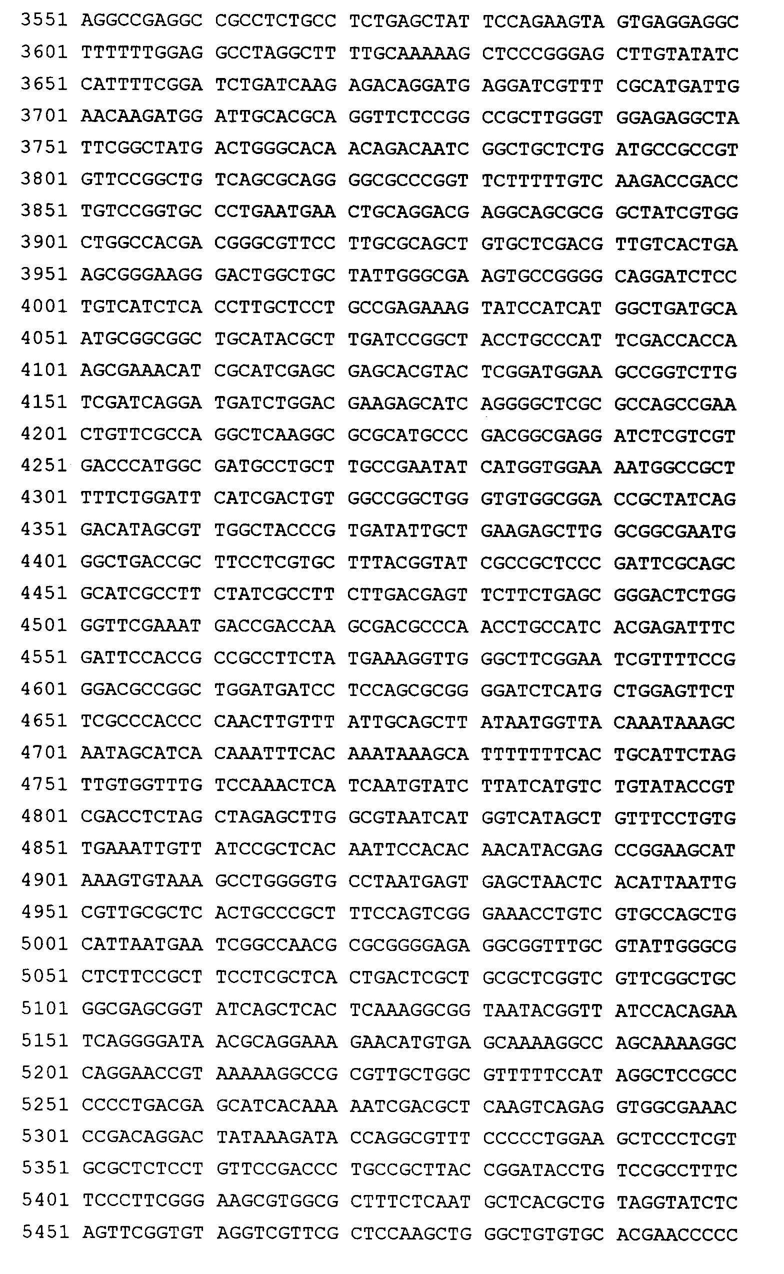

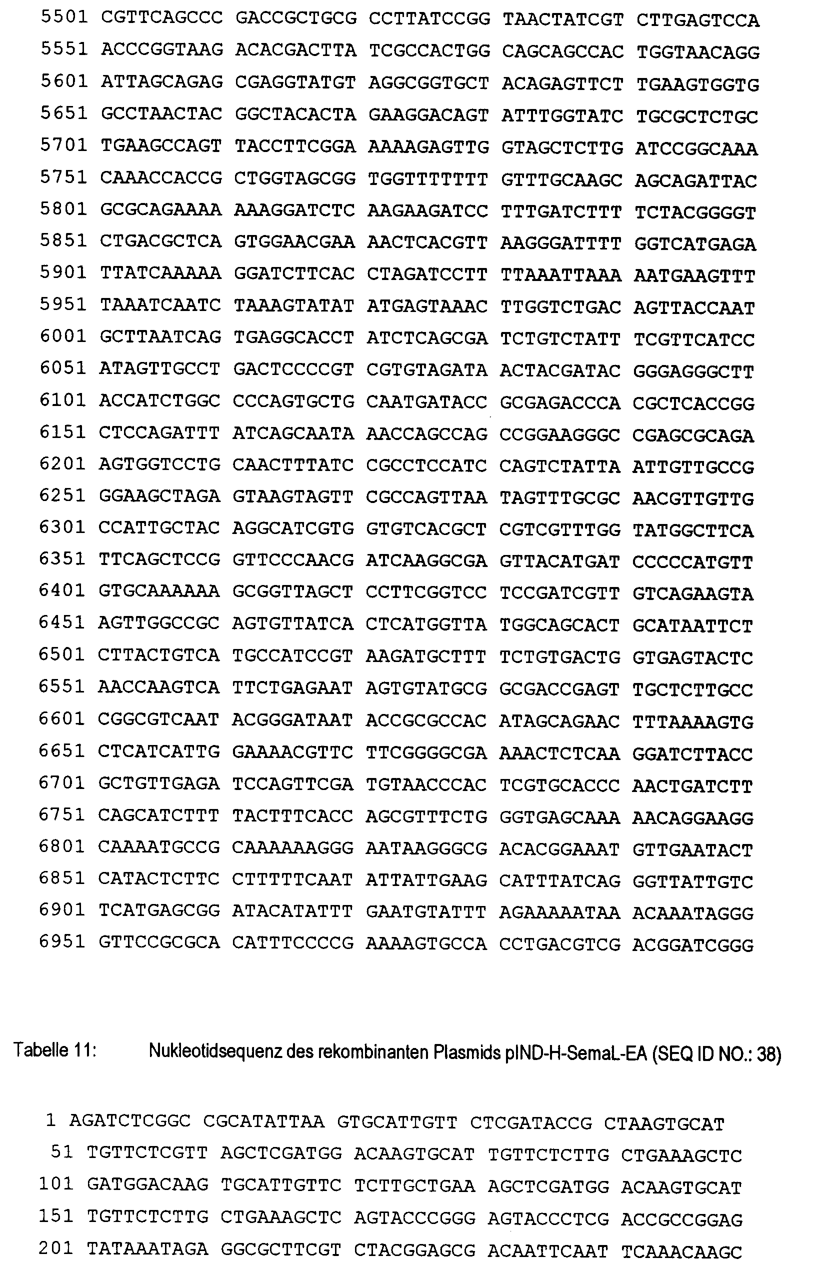

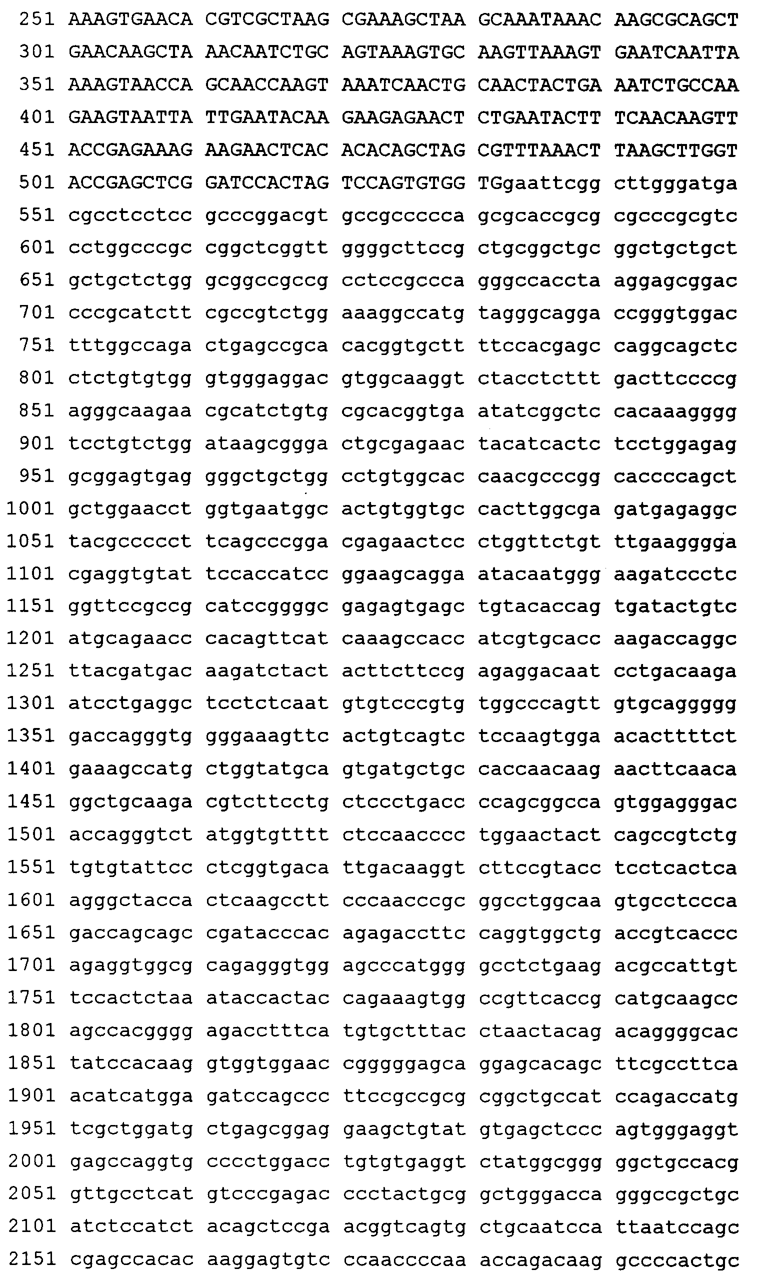

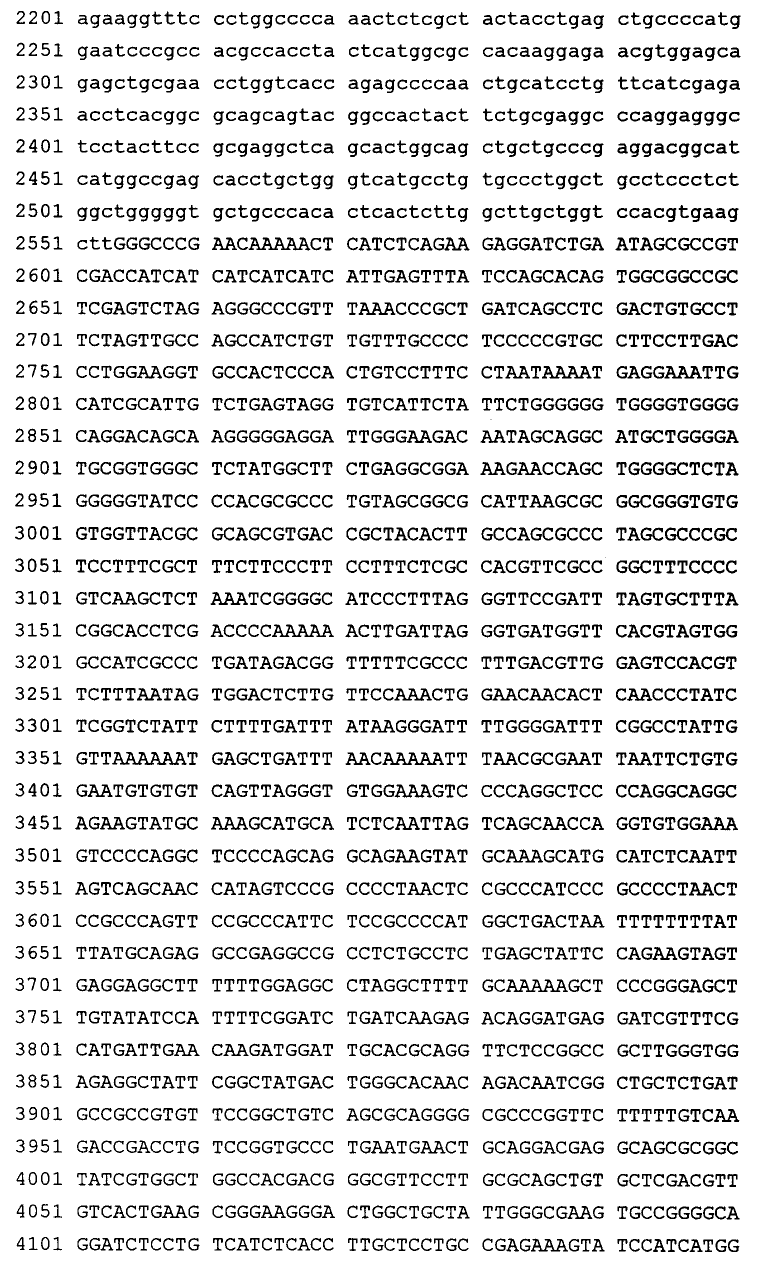

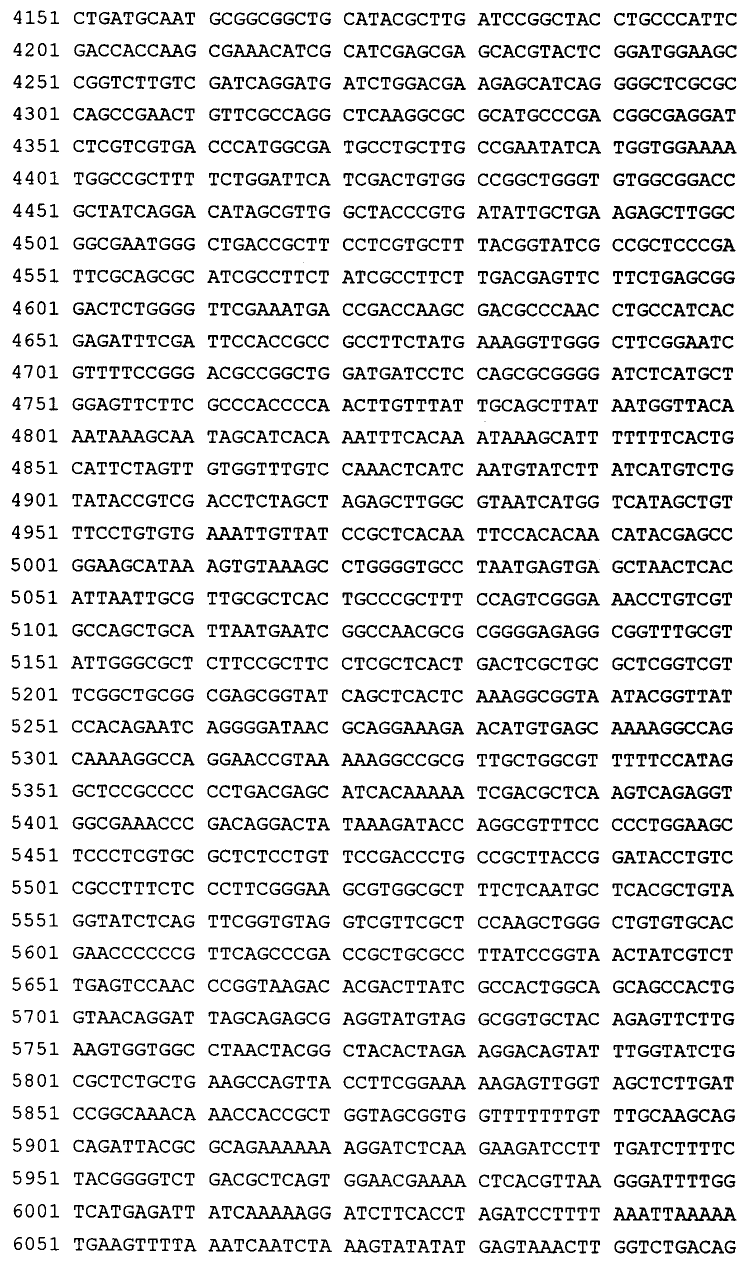

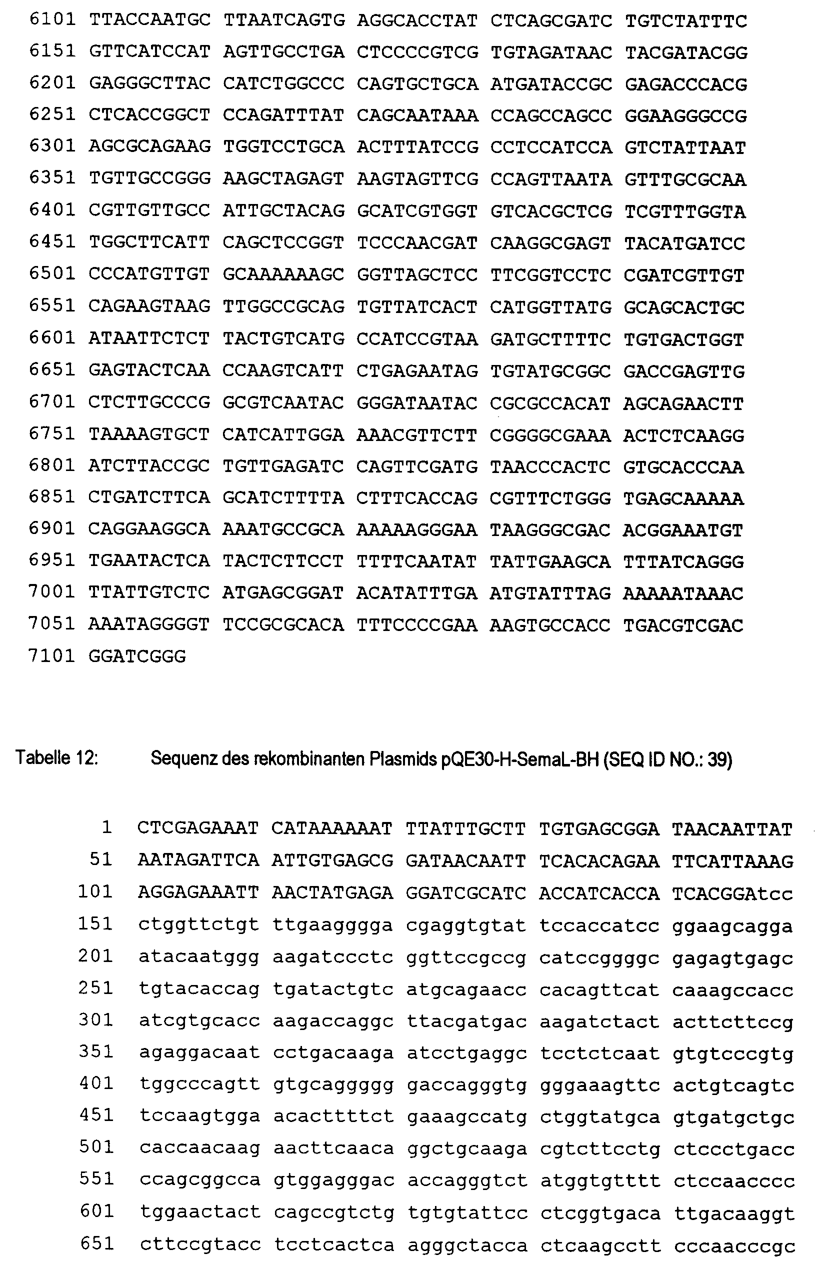

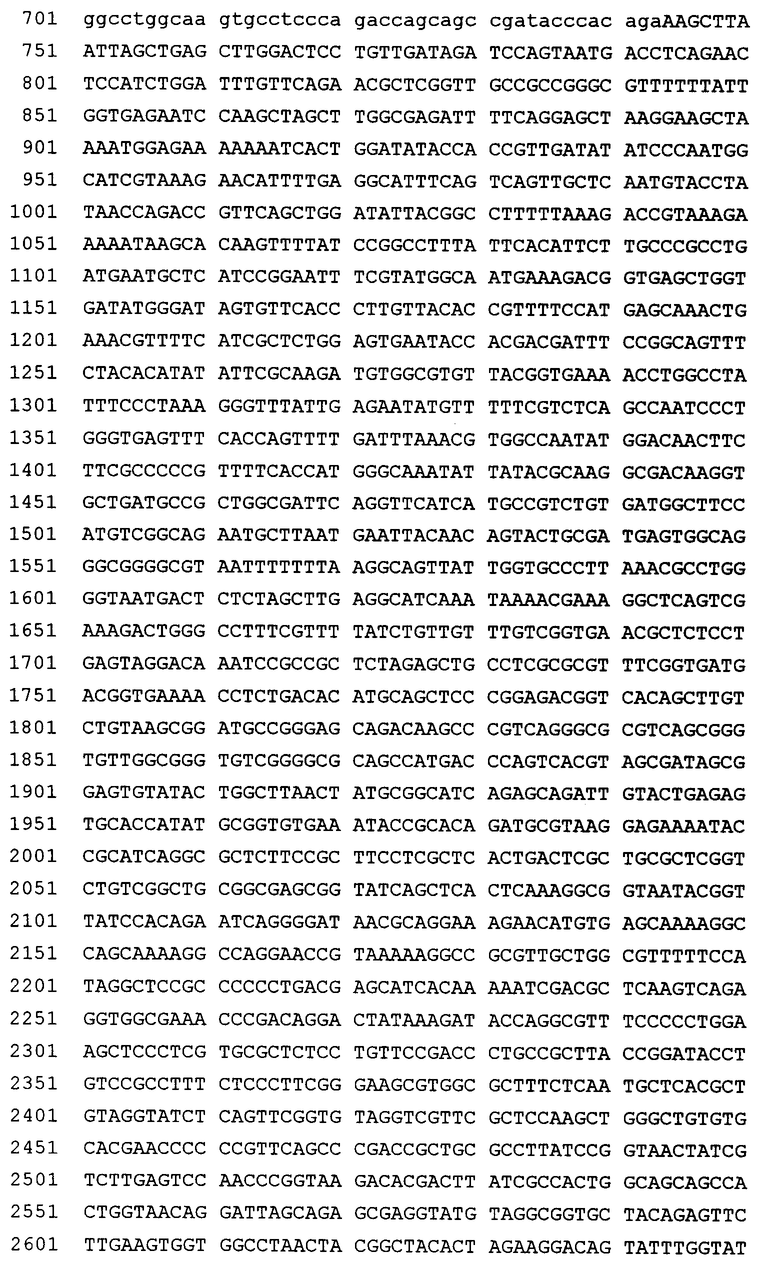

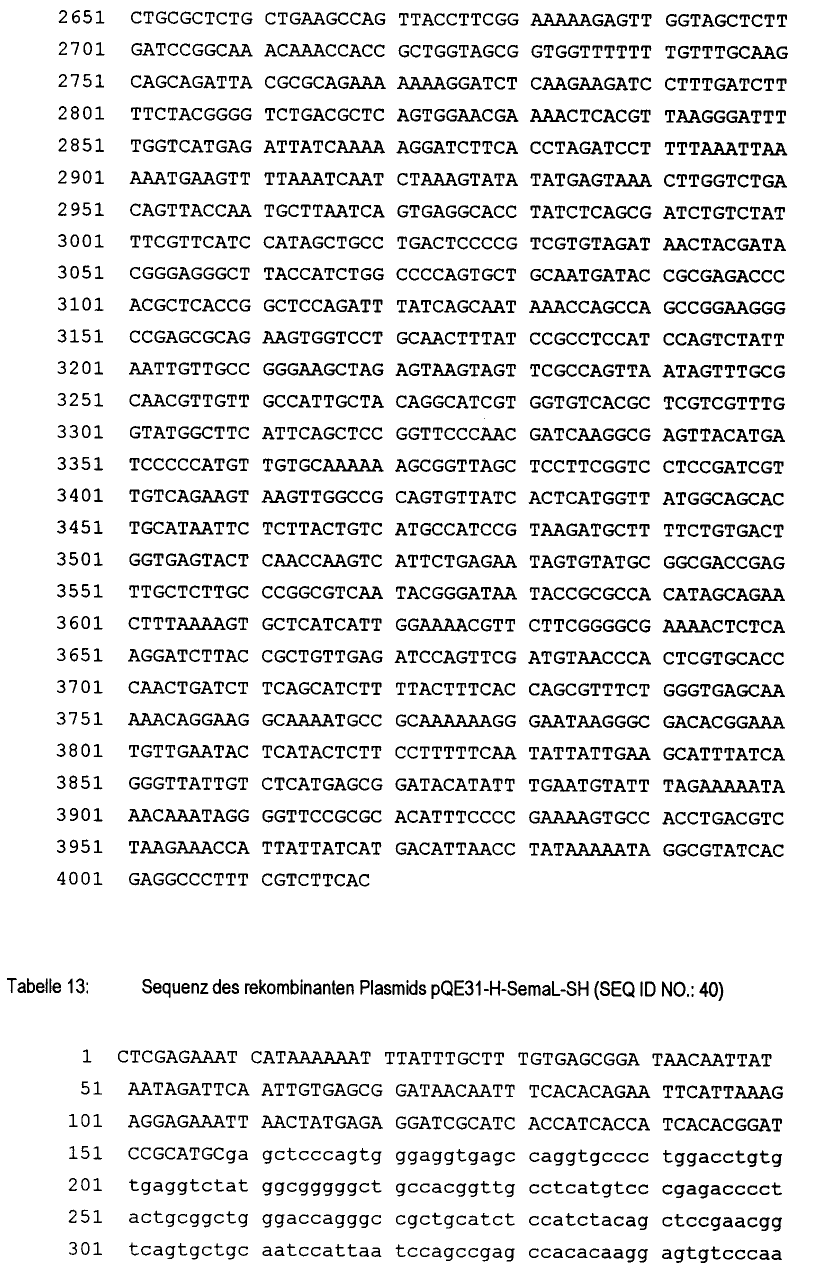

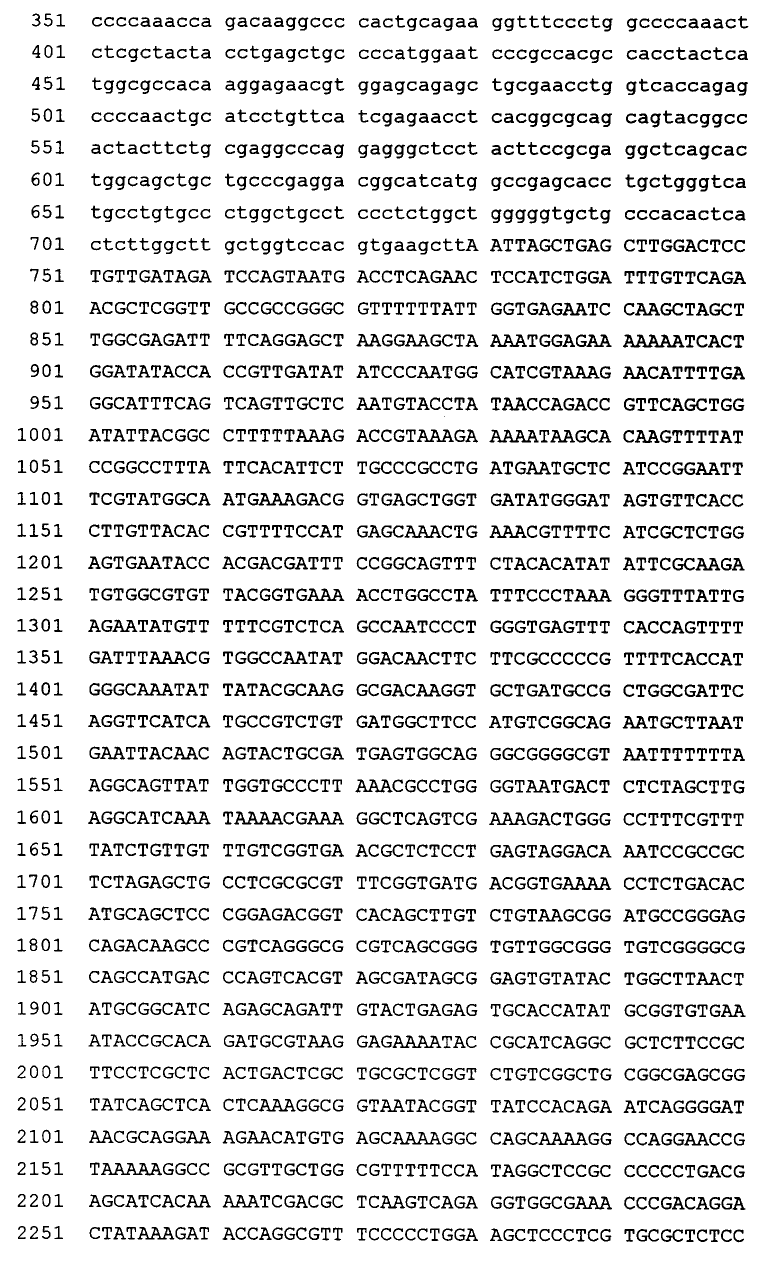

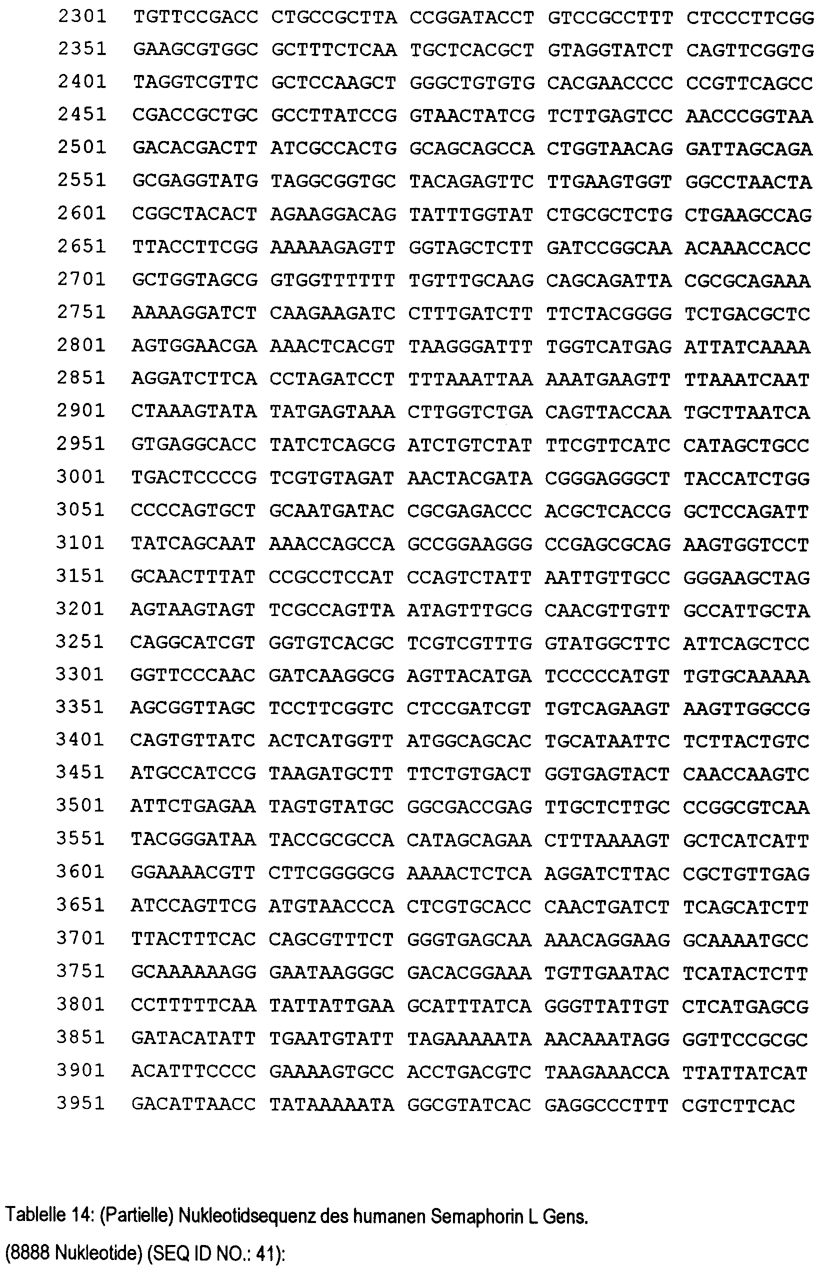

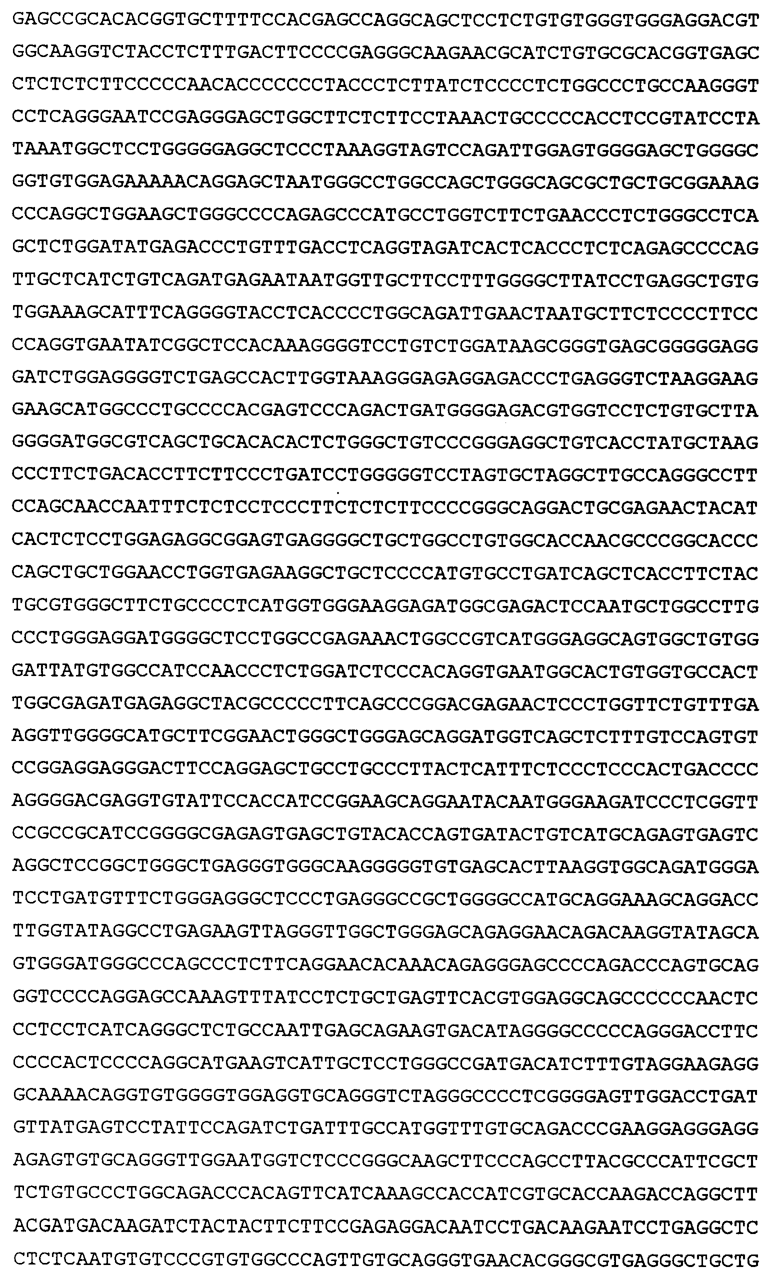

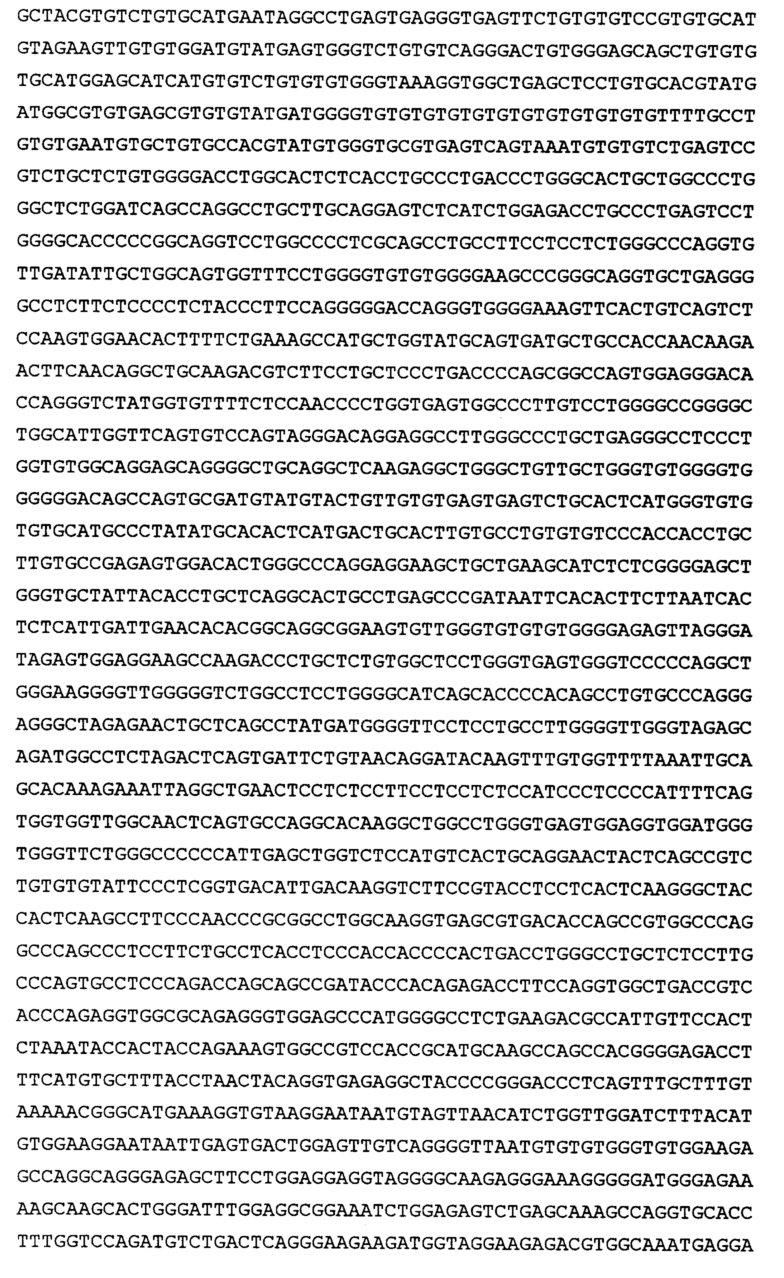

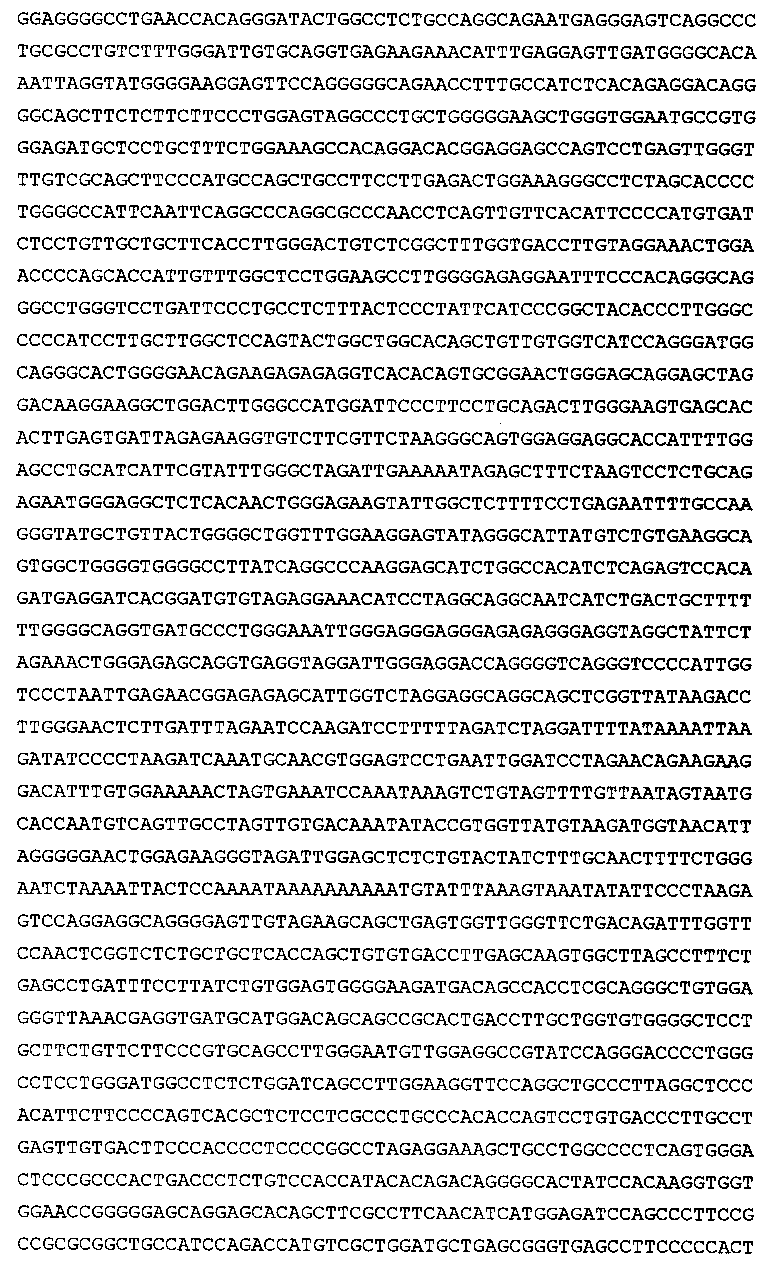

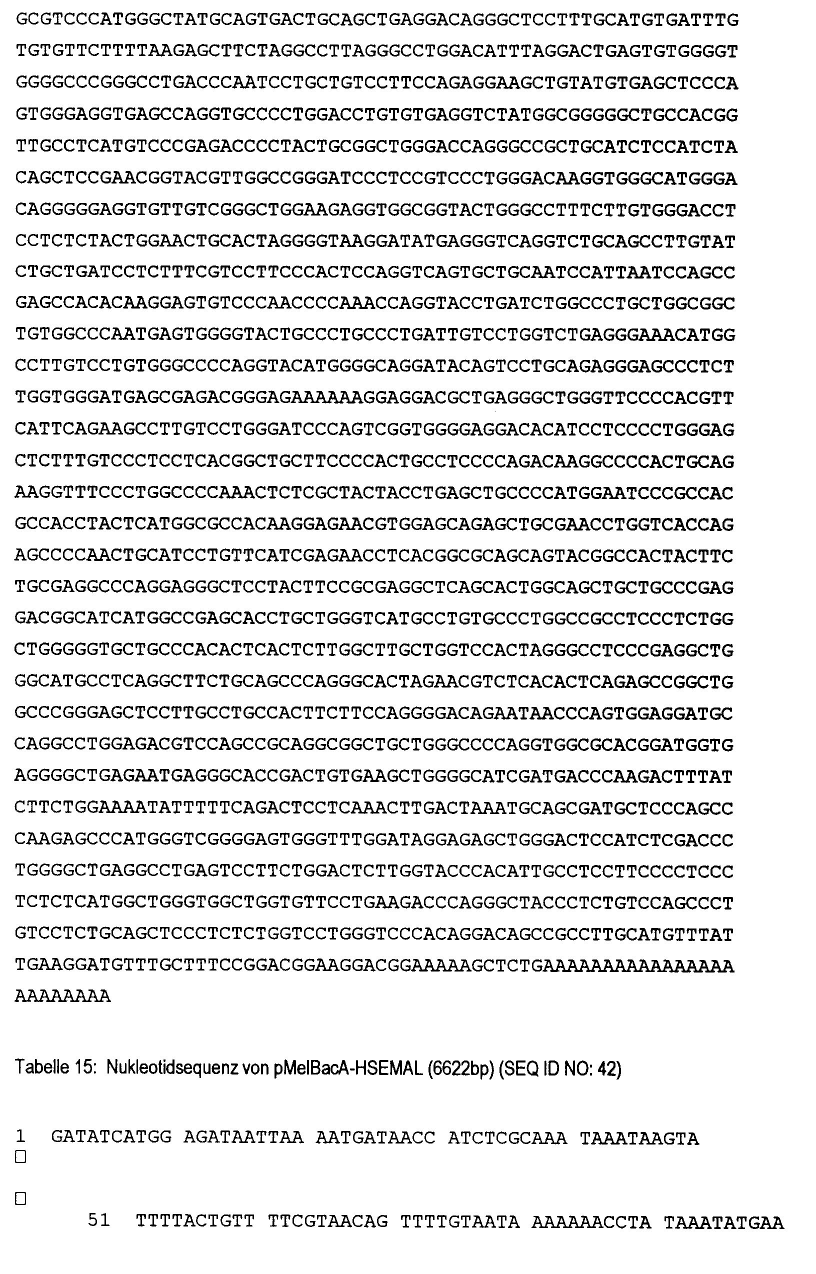

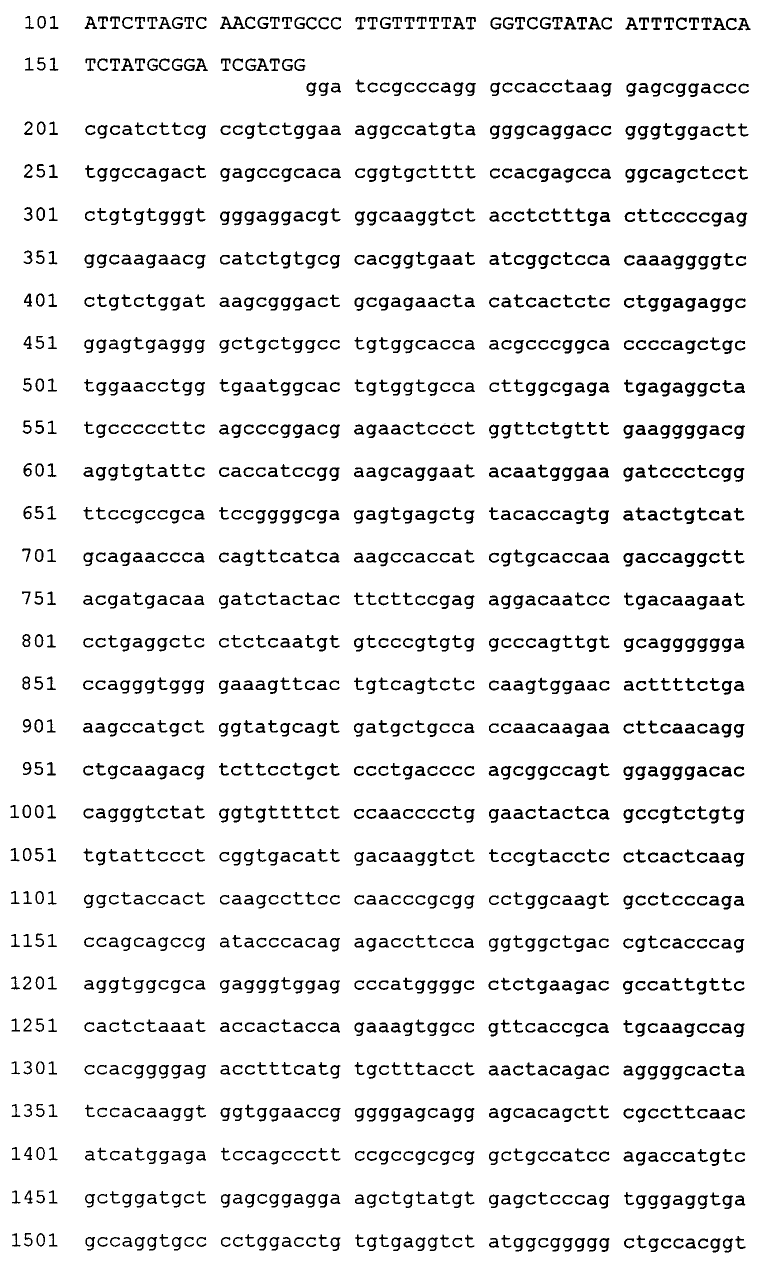

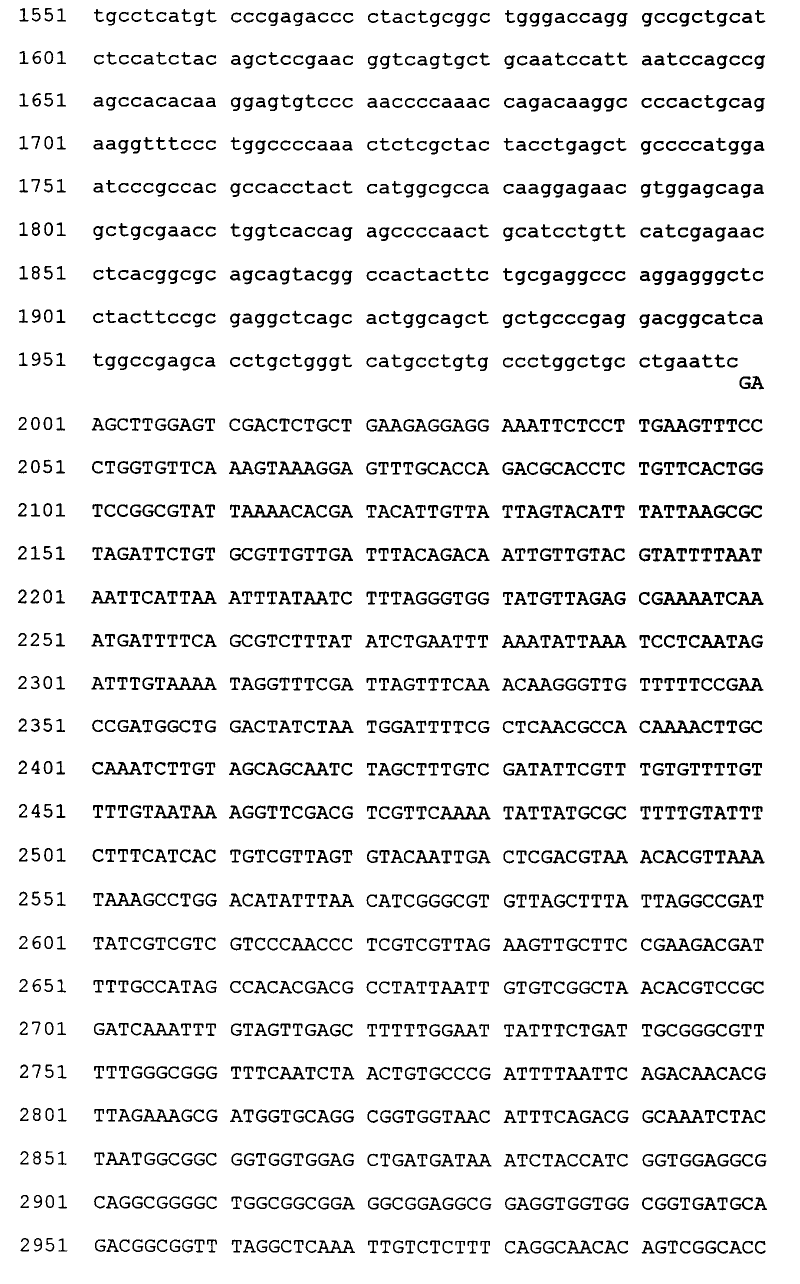

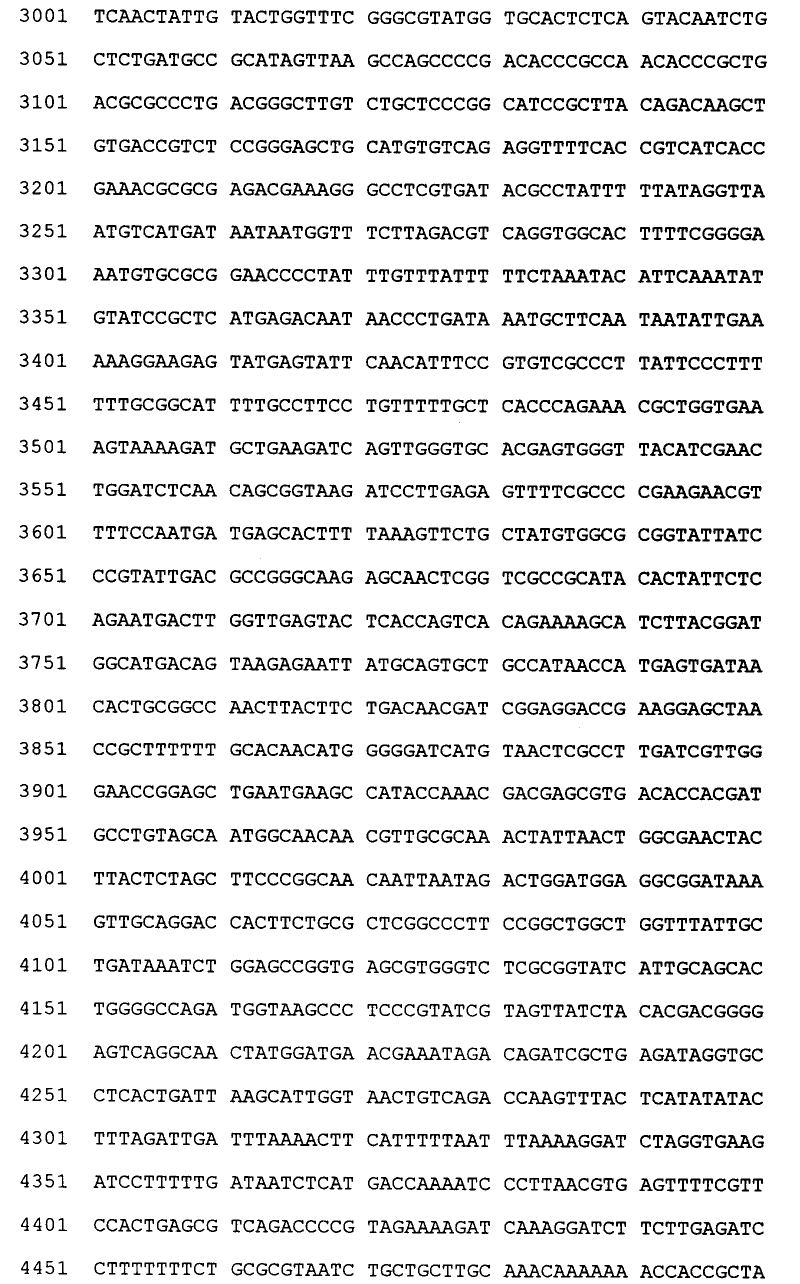

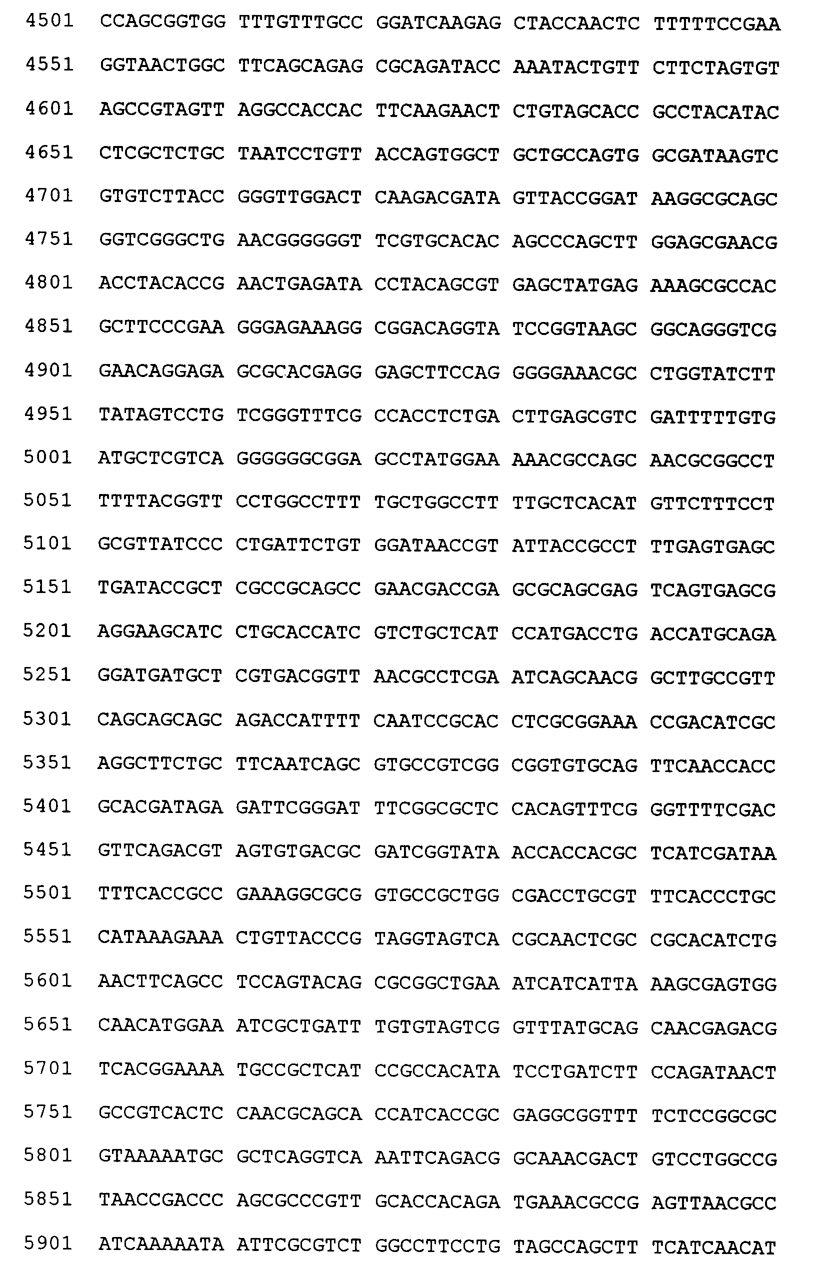

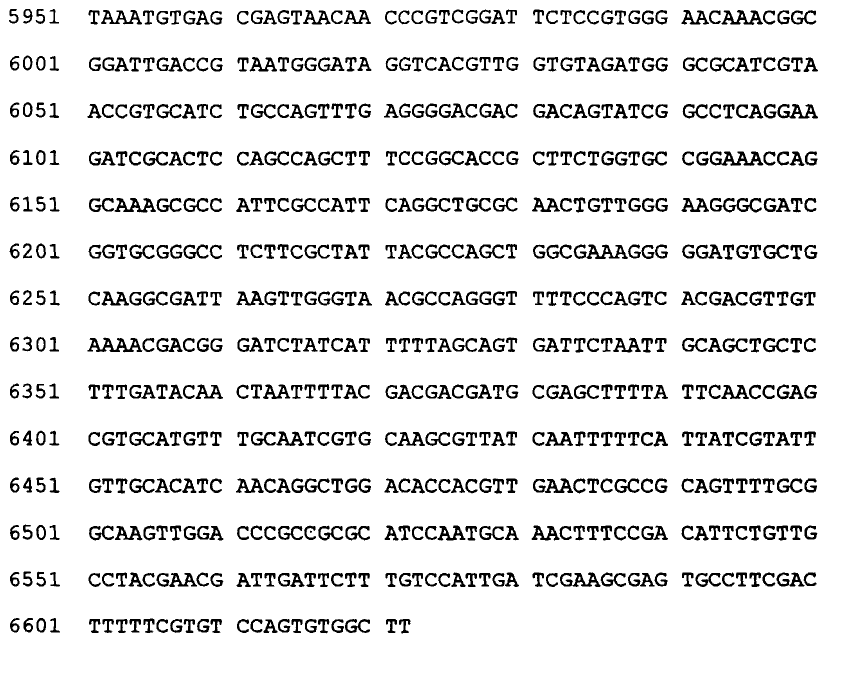

## Patentansprüche

1. Semaphorin enthaltend eine charakteristische Sema-Domäne, dadurch gekennzeichnet, daß das Protein ein N-terminales Signalpeptid und im C-terminalen Bereich eine Immunglobulin-ähnliche Domäne und eine Transmembrandomäne aufweist, wobei das Semaphorin als Semaphorin vom Typ L (SemaL) bezeichnet wird und Derivate des Semaphorins vom Typ L.

2. Semaphorin nach Anspruch 1, wobei das Protein (humanes Semaphorin vom Typ L (H-SemaL)) die Aminosäuresequenz SEQ ID NO. 3 hat.

3. Semaphorin nach einem oder mehreren der Ansprüche 1 und 2, wobei das Protein im Bereich der Sema-Domäne eine Aminosäureidentität von mindestens 40 % im Bezug auf die Sema-Domäne von H-SemaL aufweist.

4. Semaphorin nach einem oder mehreren der Ansprüche 1 bis 2, wobei das Protein die partielle Aminosäuresequenz SEQ ID NO. 4 enthält (murines Semaphorin (M-SemaL)).

5. Nukleinsäure enthaltend eine Nukleinsäure-Sequenz, die für ein Semaphorin vom Typ L gemäß einem oder mehreren der Ansprüche 1 bis 4 kodiert sowie Derivate derselben.

6. Nukleinsäure nach Anspruch 5, wobei besagte Nukleinsäure-Sequenz ein Semaphorin L Gen ist.

7. Nukleinsäure nach einem oder mehreren der Ansprüche 5 und 6, wobei besagte Nukleinsäure-Sequenz das Gen von H-SemaL enthält.

8. Nukleinsäure nach Anspruch 5, wobei besagte Nukleinsäure-Sequenz die cDNA eines Semaphorins vom Typ L enthält.

9. Nukleinsäure nach Anspruch 8, wobei die cDNA die cDNA von H-SemaL ist.

10. Nukleinsäure nach Anspruch 8, wobei die cDNA die cDNA von M-SemaL ist.

11. Verfahren zur Herstellung eines Semaphorins vom Typ L gemäß einem oder mehreren der Ansprüche 1 bis 4, dadurch gekennzeichnet, daß eine Nukleinsäure-Sequenz, die für ein Semaphorin vom Typ L oder ein Derivat derselben kodiert, in einen Expressionsvektor kloniert und exprimiert wird.

12. Verfahren gemaß Anspruch 11, wobei für die Expression eine eukaryotische Zelle verwendet wird.

13. Verwendung eines Semaphorins vom Typ L oder eines Derivats desselben oder einer Nukleinsäure-Sequenz, die für ein Semaphorin vom Typ L kodiert oder eines Derivats derselben zur Herstellung eines Arzneimittels, das zur Behandlung oder Prävention von immunologischen Erkrankungen verwendet werden kann.

14. Verwendung einer Nukleinsäure-Sequenz oder eines Derivats derselben nach Anspruch 13 in der Gentherapie.

15. Verwendung eines Semaphorins vom Typ L oder einer Nukleinsäure-Sequenz, die für ein Semaphorin vom Typ L kodiert in einem Verfahren zur Identifizierung von immunmodulierenden Wirkstoffen.

16. Verfahren zur Identifizierung von immunmodulierenden Wirkstoffen, dadurch gekennzeichnet, daß ein Semaphorin vom Typ L unter definierten Bedingungen mit einem zu untersuchenden Wirkstoff inkubiert wird, parallel ein zweiter Ansatz ohne den zu untersuchenden Wirkstoff, aber unter ansonsten gleichen Bedingungen durchgeführt wird und dann die inhibierende bzw. aktivierende Wirkung des zu untersuchenden Wirkstoffs bestimmt wird.

17. Verfahren zur Identifizierung von immunmodulierenden Wirkstoffen, dadurch gekennzeichnet, daß eine Nukleinsäure-Sequenz, die für ein Semaphorin vom Typ L kodiert unter definierten Bedingungen und in Gegenwart eines zu untersuchenden Wirkstoffs exprimiert wird und das Ausmaß der Expression bestimmt wird.

18. Verfahren zur Herstellung einer Nukleinsäure, die für ein Semaphorin vom Typ L kodiert, wobei diese Nukleinsäure mit Hilfe der Polymerase Ketten Reaktion unter Verwendung von spezifischen Primern amplifiziert wird.

19. Semaphorin Antikörper, dadurch gekennzeichnet, daß er entweder das Epitop von H-SemaL, das den Aminosäuren 179-378 in SED ID NO: 4 entspricht, erkennt oder daß er das Epitop von H-SemaL, das den Aminosäuren 480-666 in SED ID NO: 4 entspricht, erkennt.

20. Verfahren zur Herstellung eines Semaphorin Antikörpers nach Anspruch 19, wobei die Epitope als Fusionsproteine mit einem Epitop-Tag exprimiert und über dieses Epitop-Tag aufgereinigt werden und die aufgereinigten Fusionsproteine zur Immunisierung verwendet werden.


SEQUENZPROTOKOLL

(1) ALLGEMEINE INFORMATION:

(i) ANMELDER:
(A) NAME: Hoechst Marion Roussel Deutschland GmbH
(B) STRASSE: -
(C) ORT: Frankfurt
(D) BUNDESLAND: -
(E) LAND: Deutschland
(F) POSTLEITZAHL: 65926
(G) TELEPHON: 069-305-7072
(H) TELEFAX: 069-35-7175
(I) TELEX: -

(ii) ANMELDETITEL: Humanes Semaphorin L (H-Sema-L) und
korrespondierende Semaphorine in anderen Spezies

(iii) ANZAHL DER SEQUENZEN: 44

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 2636 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..2636

(xi) SEQUENZBESCHREIBUNG: SEQ ID NO: 1:

CGGGGCCACG GGATGACGCC TCCTCCGCCC GGACGTGCCG CCCCCAGCGC ACCGCGCGCC 60

CGCGTCCCTG GCCCGCCGGC TCGGTTGGGG CTTCCGCTGC GGCTGCGGCT GCTGCTGCTG 120

CTCTGGGCGG CCGCCGCCTC CGCCCAGGGC CACCTAAGGA GCGGACCCCG CATCTTCGCC 180

GTCTGGAAAG GCCATGTAGG GCAGGACCGG GTGGACTTTG GCCAGACTGA GCCGCACACG 240

GTGCTTTTCC ACGAGCCAGG CAGCTCCTCT GTGTGGGTGG GAGGACGTGG CAAGGTCTAC 300

CTCTTTGACT TCCCCGAGGG CAAGAACGCA TCTGTGCGCA CGGTGAATAT CGGCTCCACA 360

AAGGGGTCCT GTCTGGATAA GCGGGACTGC GAGAACTACA TCACTCTCCT GGAGAGGCGG 420

AGTGAGGGGC TGCTGGCCTG TGGCACCAAC GCCCGGCACC CCAGCTGCTG GAACCTGGTG 480

AATGGCACTG TGGTGCCACT TGGCGAGATG AGAGGCTACG CCCCCTTCAG CCCGGACGAG 540

AACTCCCTGG TTCTGTTTGA AGGGGACGAG GTGTATTCCA CCATCCGGAA GCAGGAATAC 600

AATGGGAAGA TCCCTCGGTT CCGCCGCATC CGGGGCGAGA GTGAGCTGTA CACCAGTGAT 660

ACTGTCATGC AGAACCCACA GTTCATCAAA GCCACCATCG TGCACCAAGA CCAGGCTTAC 720

GATGACAAGA TCTACTACTT CTTCCGAGAG GACAATCCTG ACAAGAATCC TGAGGCTCCT 780

CTCAATGTGT CCCGTGTGGC CCAGTTGTGC AGGGGGGACC AGGGTGGGGA AAGTTCACTG 840

TCAGTCTCCA AGTGGAACAC TTTTCTGAAA GCCATGCTGG TATGCAGTGA TGCTGCCACC 900

AACAAGAACT TCAACAGGCT GCAAGACGTC TTCCTGCTCC CTGACCCCAG CGGCCAGTGG 960

AGGGACACCA GGGTCTATGG TGTTTTCTCC AACCCCTGGA ACTACTCAGC CGTCTGTGTG 1020

TATTCCCTCG GTGACATTGA CAAGGTCTTC CGTACCTCCT CACTCAAGGG CTACCACTCA 1080

AGCCTTCCCA ACCCGCGGCC TGGCAAGTGC CTCCCAGACC AGCAGCCGAT ACCCACAGAG 1140

ACCTTCCAGG TGGCTGACCG TCACCCAGAG GTGGCGCAGA GGGTGGAGCC CATGGGGCCT 1200

CTGAAGACGC CATTGTTCCA CTCTAAATAC CACTACCAGA AAGTGGCCGT TCACCGCATG 1260

CAAGCCAGCC ACGGGGAGAC CTTTCATGTG CTTTACCTAA CTACAGACAG GGGCACTATC 1320

CACAAGGTGG TGGAACCGGG GGAGCAGGAG CACAGCTTCG CCTTCAACAT CATGGAGATC 1380

CAGCCCTTCC GCCGCGCGGC TGCCATCCAG ACCATGTCGC TGGATGCTGA GCGGAGGAAG 1440

CTGTATGTGA GCTCCCAGTG GGAGGTGAGC CAGGTGCCCC TGGACCTGTG TGAGGTCTAT 1500

GGCGGGGGCT GCCACGGTTG CCTCATGTCC CGAGACCCCT ACTGCGGCTG GGACCAGGGC 1560

CGCTGCATCT CCATCTACAG CTCCGAACGG TCAGTGCTGC AATCCATTAA TCCAGCCGAG 1620

CCACACAAGG AGTGTCCCAA CCCCAAACCA GACAAGGCCC CACTGCAGAA GGTTTCCCTG 1680

GCCCCAAACT CTCGCTACTA CCTGAGCTGC CCCATGGAAT CCCGCCACGC CACCTACTCA 1740

TGGCGCCACA AGGAGAACGT GGAGCAGAGC TGCGAACCTG GTCACCAGAG CCCCAACTGC 1800

ATCCTGTTCA TCGAGAACCT CACGGCGCAG CAGTACGGCC ACTACTTCTG CGAGGCCCAG 1860

GAGGGCTCCT ACTTCCGCGA GGCTCAGCAC TGGCAGCTGC TGCCCGAGGA CGGCATCATG 1920

GCCGAGCACC TGCTGGGTCA TGCCTGTGCC CTGGCTGCCT CCCTCTGGCT GGGGGTGCTG 1980

CCCACACTCA CTCTTGGCTT GCTGGTCCAC TAGGGCCTCC CGAGGCTGGG CATGCCTCAG 2040

GCTTCTGCAG CCCAGGGCAC TAGAACGTCT CACACTCAGA GCCGGCTGGC CCGGGAGCTC 2100

CTTGCCTGCC ACTTCTTCCA GGGGACAGAA TAACCCAGTG GAGGATGCCA GGCCTGGAGA 2160

CGTCCAGCCG CAGGCGGCTG CTGGGCCCCA GGTGGCGCAC GGATGGTGAG GGGCTGAGAA 2220

TGAGGGCACC GACTGTGAAG CTGGGGCATC GATGACCCAA GACTTTATCT TCTGGAAAAT 2280

ATTTTTCAGA CTCCTCAAAC TTGACTAAAT GCAGCGATGC TCCCAGCCCA AGAGCCCATG 2340

GGTCGGGGAG TGGGTTTGGA TAGGAGAGCT GGGACTCCAT CTCGACCCTG GGGCTGAGGC 2400

CTGAGTCCTT CTGGACTCTT GGTACCCACA TTGCCTCCTT CCCCTCCCTC TCTCATGGCT 2460

GGGTGGCTGG TGTTCCTGAA GACCCAGGGC TACCCTCTGT CCAGCCCTGT CCTCTGCAGC 2520

TCCCTCTCTG GTCCTGGGTC CCACAGGACA GCCGCCTTGC ATGTTTATTG AAGGATGTTT 2580

GCTTTCCGGA CGGAAGGACG GAAAAAGCTC TGAAAAAAAA AAAAAAAAAA AAAAAA 2636

(2) INFORMATION ZU SEQ ID NO: 2:

(i) SEQUENZ CHARAKTERISTIKA:
(A) LÄNGE: 1195 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..1195

(xi) SEQUENZBESCHREIBUNG: SEQ ID NO: 2:

CGGGGCTGCG GGATGACGCC TCCTCCTCCC GGACGTGCCG CCCCCAGCGC ACCGCGCGCC 60

CGCGTCCTCA GCCTGCCGGC TCGGTTCGGG CTCCCGCTGC GGCTGCGGCT TCTGCTGGTG 120

TTCTGGGTGG CCGCCGCCTC CGCCCAAGGC CACTCGAGGA GCGGACCCCG CATCTCCGCC 180

GTCTGGAAAG GGCAGGACCA TGTGGACTTT AGCCAGCCTG AGCCACACAC CGTGCTTTTC 240

CATGAGCCGG GCAGCTTCTC TGTCTGGGTG GGTGGACGTG GCAAGGTCTA CCACTTCAAC 300

TTCCCCGAGG GCAAGAATGC CTCTGTGCGC ACGGTGAACA TCGGCTCCAC AAAGGGGTCC 360

TGTCAGGACA AACAGGACTG TGGGAATTAC ATCACTCTTC TAGAAAGGCG GGGTAATGGG 420

CTGCTGGTCT GTGGCACCAA TGCCCGGAAG CCCAGCTGCT GGAACTTGGT GAATGACAGT 480

GTGGTGATGT CACTTGGTGA GATGAAAGGC TATGCCCCCT TCAGCCCGGA TGAGAACTCC 540

CTGGTTCTGT TTGAAGGAGA TGAAGTGTAC TCTACCATCC GGAAGCAGGA ATACAACGGG 600

AAGATCCCTC GGTTTCGACG CATTCGGGGC GAGAGTGAAC TGTACACAAG TGATACAGTC 660

ATGCAGAACC CACAGTTCAT CAAGGCCACC ATTGTGCACC AAGACCAAGC CTATGATGAT 720

AAGATCTACT ACTTCTTCCG AGAAGACAAC CCTGACAAGA ACCCCGAGGC TCCTCTCAAT 780

GTGTCCCGAG TAGCCCAGTT GTGCAGGGGG GACCAGGGTG GTGAGAGTTC GTTGTCTGTC 840

TCCAAGTGGA ACACCTTCCT GAAAGCCATG TTGGTCTGCA GCGATGCAGC CACCAACAGG 900

AACTTCAATC GGCTGCAAGA TGTCTTCCTG CTCCCTGACC CCAGTGGCCA GTGGAGAGAT 960

ACCAGGGTCT ATGGCGTTTT CTCCAACCCC TGGAACTACT CAGCTGTCTG CGTGTATTCG 1020

CTTGGTGACA TTGACAGAGT CTTCCGTACC TCATCGCTCA AAGGCTACCA CATGGGCCTT 1080

TCCAACCCTC GACCTGGCAT GTGCCTCCCA AAAAAGCAGC CCATACCCAC AGAAACCTTC 1140

CAGGTAGCTG ATAGTCACCC AGAGGTGGCT CAGAGGGTGG AACCTATGGG GCCCC 1195

(2) INFORMATION ZU SEQ ID NO: 3:

(i) SEQUENZ CHARAKTERISTIKA:
(A) LÄNGE: 666 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..666

(xi) SEQUENZBESCHREIBUNG: SEQ ID NO: 3:

Met Thr Pro Pro Pro Pro Gly Arg Ala Ala Pro Ser Ala Pro Arg Ala
1               5                   10                  15

Arg Val Pro Gly Pro Pro Ala Arg Leu Gly Leu Pro Leu Arg Leu Arg
            20                  25                  30

Leu Leu Leu Leu Leu Trp Ala Ala Ala Ala Ser Ala Gln Gly His Leu
        35                  40                  45

Arg Ser Gly Pro Arg Ile Phe Ala Val Trp Lys Gly His Val Gly Gln
    50                  55                  60

Asp Arg Val Asp Phe Gly Gln Thr Glu Pro His Thr Val Leu Phe His
65                  70                  75                  80

Glu Pro Gly Ser Ser Ser Val Trp Val Gly Gly Arg Gly Lys Val Tyr
                85                  90                  95

Leu Phe Asp Phe Pro Glu Gly Lys Asn Ala Ser Val Arg Thr Val Asn
            100                 105                 110

Ile Gly Ser Thr Lys Gly Ser Cys Leu Asp Lys Arg Asp Cys Glu Asn
        115                 120                 125

Tyr Ile Thr Leu Leu Glu Arg Arg Ser Glu Gly Leu Leu Ala Cys Gly
    130                 135                 140

Thr Asn Ala Arg His Pro Ser Cys Trp Asn Leu Val Asn Gly Thr Val
145                 150                 155                 160

Val Pro Leu Gly Glu Met Arg Gly Tyr Ala Pro Phe Ser Pro Asp Glu
                165                 170                 175

Asn Ser Leu Val Leu Phe Glu Gly Asp Glu Val Tyr Ser Thr Ile Arg
            180                 185                 190

Lys Gln Glu Tyr Asn Gly Lys Ile Pro Arg Phe Arg Arg Ile Arg Gly
        195                 200                 205

Glu Ser Glu Leu Tyr Thr Ser Asp Thr Val Met Gln Asn Pro Gln Phe
    210                 215                 220

Ile Lys Ala Thr Ile Val His Gln Asp Gln Ala Tyr Asp Asp Lys Ile
225                 230                 235                 240

Tyr Tyr Phe Phe Arg Glu Asp Asn Pro Asp Lys Asn Pro Glu Ala Pro
                245                 250                 255

Leu Asn Val Ser Arg Val Ala Gln Leu Cys Arg Gly Asp Gln Gly Gly
            260                 265                 270

Glu Ser Ser Leu Ser Val Ser Lys Trp Asn Thr Phe Leu Lys Ala Met
        275                 280                 285

Leu Val Cys Ser Asp Ala Ala Thr Asn Lys Asn Phe Asn Arg Leu Gln
    290                 295                 300

Asp Val Phe Leu Leu Pro Asp Pro Ser Gly Gln Trp Arg Asp Thr Arg
305                 310                 315                 320

Val Tyr Gly Val Phe Ser Asn Pro Trp Asn Tyr Ser Ala Val Cys Val
                325                 330                 335

Tyr Ser Leu Gly Asp Ile Asp Lys Val Phe Arg Thr Ser Ser Leu Lys
            340                 345                 350

Gly Tyr His Ser Ser Leu Pro Asn Pro Arg Pro Gly Lys Cys Leu Pro
        355                 360                 365

Asp Gln Gln Pro Ile Pro Thr Glu Thr Phe Gln Val Ala Asp Arg His
    370                 375                 380

Pro Glu Val Ala Gln Arg Val Glu Pro Met Gly Pro Leu Lys Thr Pro
385                 390                 395                 400

Leu Phe His Ser Lys Tyr His Tyr Gln Lys Val Ala Val His Arg Met
                405                 410                 415

Gln Ala Ser His Gly Glu Thr Phe His Val Leu Tyr Leu Thr Thr Asp
            420                 425                 430

Arg Gly Thr Ile His Lys Val Val Glu Pro Gly Glu Gln Glu His Ser
        435                 440                 445

Phe Ala Phe Asn Ile Met Glu Ile Gln Pro Phe Arg Arg Ala Ala Ala
    450                 455                 460

Ile Gln Thr Met Ser Leu Asp Ala Glu Arg Arg Lys Leu Tyr Val Ser
465                 470                 475                 480

Ser Gln Trp Glu Val Ser Gln Val Pro Leu Asp Leu Cys Glu Val Tyr
                485                 490                 495

Gly Gly Gly Cys His Gly Cys Leu Met Ser Arg Asp Pro Tyr Cys Gly
            500                 505                 510

Trp Asp Gln Gly Arg Cys Ile Ser Ile Tyr Ser Ser Glu Arg Ser Val
        515                 520                 525

Leu Gln Ser Ile Asn Pro Ala Glu Pro His Lys Glu Cys Pro Asn Pro
    530                 535                 540

Lys Pro Asp Lys Ala Pro Leu Gln Lys Val Ser Leu Ala Pro Asn Ser
545                 550                 555                 560

Arg Tyr Tyr Leu Ser Cys Pro Met Glu Ser Arg His Ala Thr Tyr Ser
                565                 570                 575

Trp Arg His Lys Glu Asn Val Glu Gln Ser Cys Glu Pro Gly His Gln
            580                 585                 590

Ser Pro Asn Cys Ile Leu Phe Ile Glu Asn Leu Thr Ala Gln Gln Tyr
        595                 600                 605

Gly His Tyr Phe Cys Glu Ala Gln Glu Gly Ser Tyr Phe Arg Glu Ala
    610                 615                 620

Gln His Trp Gln Leu Leu Pro Glu Asp Gly Ile Met Ala Glu His Leu
625                 630                 635                 640

Leu Gly His Ala Cys Ala Leu Ala Ala Ser Leu Trp Leu Gly Val Leu
                645                 650                 655

Pro Thr Leu Thr Leu Gly Leu Leu Val His
            660                 665

(2) INFORMATION ZU SEQ ID NO: 4:

(i) SEQUENZ CHARAKTERISTIKA:
(A) LÄNGE: 394 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..394

(xi) SEQUENZBESCHREIBUNG: SEQ ID NO: 4:

Met Thr Pro Pro Pro Pro Gly Arg Ala Ala Pro Ser Ala Pro Arg Ala
1               5                   10                  15

Arg Val Leu Ser Leu Pro Ala Arg Phe Gly Leu Pro Leu Arg Leu Arg
            20                  25                  30

Leu Leu Leu Val Phe Trp Val Ala Ala Ala Ser Ala Gln Gly His Ser
        35                  40                  45

Arg Ser Gly Pro Arg Ile Ser Ala Val Trp Lys Gly Gln Asp His Val
    50                  55                  60

Asp Phe Ser Gln Pro Glu Pro His Thr Val Leu Phe His Glu Pro Gly
65                  70                  75                  80

Ser Phe Ser Val Trp Val Gly Gly Arg Gly Lys Val Tyr His Phe Asn
                85                  90                  95

Phe Pro Glu Gly Lys Asn Ala Ser Val Arg Thr Val Asn Ile Gly Ser
            100                 105                 110

Thr Lys Gly Ser Cys Gln Asp Lys Gln Asp Cys Gly Asn Tyr Ile Thr
        115                 120                 125

Leu Leu Glu Arg Arg Gly Asn Gly Leu Leu Val Cys Gly Thr Asn Ala
    130                 135                 140

Arg Lys Pro Ser Cys Trp Asn Leu Val Asn Asp Ser Val Val Met Ser
145                 150                 155                 160

Leu Gly Glu Met Lys Gly Tyr Ala Pro Phe Ser Pro Asp Glu Asn Ser
                165                 170                 175

Leu Val Leu Phe Glu Gly Asp Glu Val Tyr Ser Thr Ile Arg Lys Gln
            180                 185                 190

Glu Tyr Asn Gly Lys Ile Pro Arg Phe Arg Arg Ile Arg Gly Glu Ser
        195                 200                 205

Glu Leu Tyr Thr Ser Asp Thr Val Met Gln Asn Pro Gln Phe Ile Lys
    210                 215                 220

Ala Thr Ile Val His Gln Asp Gln Ala Tyr Asp Asp Lys Ile Tyr Tyr
225                 230                 235                 240

Phe Phe Arg Glu Asp Asn Pro Asp Lys Asn Pro Glu Ala Pro Leu Asn
                245                 250                 255

Val Ser Arg Val Ala Gln Leu Cys Arg Gly Asp Gln Gly Gly Glu Ser
            260                 265                 270

Ser Leu Ser Val Ser Lys Trp Asn Thr Phe Leu Lys Ala Met Leu Val
        275                 280                 285

Cys Ser Asp Ala Ala Thr Asn Arg Asn Phe Asn Arg Leu Gln Asp Val
    290                 295                 300

Phe Leu Leu Pro Asp Pro Ser Gly Gln Trp Arg Asp Thr Arg Val Tyr
305                 310                 315                 320

Gly Val Phe Ser Asn Pro Trp Asn Tyr Ser Ala Val Cys Val Tyr Ser
                325                 330                 335

Leu Gly Asp Ile Asp Arg Val Phe Arg Thr Ser Ser Leu Lys Gly Tyr
            340                 345                 350

His Met Gly Leu Ser Asn Pro Arg Pro Gly Met Cys Leu Pro Lys Lys
        355                 360                 365

Gln Pro Ile Pro Thr Glu Thr Phe Gln Val Ala Asp Ser His Pro Glu
    370                 375                 380

Val Ala Gln Arg Val Glu Pro Met Gly Pro
385                 390

(2) INFORMATION ZU SEQ ID NO: 5:

(i) SEQUENZ CHARAKTERISTIKA:
(A) LÄNGE: 23 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..23

(xi) SEQUENZBESCHREIBUNG: SEQ ID NO: 5:

ACTCACTATA GGGCTCGAGC GGC 23

(2) INFORMATION ZU SEQ ID NO: 6:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..20

(xi) SEQUENZBESCHREIBUNG: SEQ ID NO: 6:

AGCCGCACAC GGTGCTTTTC 20

(2) INFORMATION ZU SEQ ID NO: 7:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..20

(xi) SEQUENZBESCHREIBUNG: SEQ ID NO: 7:

GCACAGATGC GTTCTTGCCC 20

(2) INFORMATION ZU SEQ ID NO: 8:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..20

(xi) SEQUENZBESCHREIBUNG: SEQ ID NO: 8:

ACCATAGACC CTGGTGTCCC 20

(2) INFORMATION ZU SEQ ID NO: 9:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..20

(xi) SEQUENZBESCHREIBUNG: SEQ ID NO: 9:

GCAGTGATGC TGCCACCAAC 20

(2) INFORMATION ZU SEQ ID NO: 10:

(i) SEQUENZ CHARAKTERISTIKA:
(A) LÄNGE: 20 Basenpaare
(B) ART: Nukleinsäure

(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..20

(xi) SEQUENZBESCHREIBUNG: SEQ ID NO: 10:

CCAGACCATG TCGCTGGATG 20

(2) INFORMATION ZU SEQ ID NO: 11:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..20

(xi) SEQUENZBESCHREIBUNG: SEQ ID NO: 11:

ACATGAGGCA ACCGTGGCAG 20

(2) INFORMATION ZU SEQ ID NO: 12:

(i) SEQUENZ CHARAKTERISTIKA:
(A) LÄNGE: 27 Basenpaare
(B) ART: Nukleinsäure
(C) STRANGFORM: Einzel
(D) TOPOLOGIE: linear

(ii) ART DES MOLEKÜLS: DNS (genomisch)

(xi) SEQUENZBESCHREIBUNG: SEQ ID NO: 12:

CCATCCTAAT ACGACTCACT ATAGGGC 27

(2) INFORMATION ZU SEQ ID NO: 13:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..20

(xi) SEQUENZBESCHREIBUNG: SEQ ID NO: 13:

AGGTAGACCT TGCCACGTCC 20

(2) INFORMATION ZU SEQ ID NO: 14:

(i) SEQUENZ CHARAKTERISTIKA:
(A) LÄNGE: 23 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..23

(xi) SEQUENZBESCHREIBUNG: SEQ ID NO: 14:

GAACTTCAAC AGGCTGCAAG ACG 23

(2) INFORMATION ZU SEQ ID NO: 15:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..20

(xi) SEQUENZBESCHREIBUNG: SEQ ID NO: 15:

ATGCTGAGCG GAGGAAGCTG 20

(2) INFORMATION ZU SEQ ID NO: 16:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)

(xi) SEQUENZBESCHREIBUNG: SEQ ID NO: 16:

CCGCCATACA CCTCACACAG 20

(2) INFORMATION ZU SEQ ID NO: 17:

(i) SEQUENZ CHARAKTERISTIKA:
(A) LÄNGE: 28 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..28

(xi) SEQUENZBESCHREIBUNG: SEQ ID NO: 17:

CTGGAAGCTT TCTGTGGGTA TCGGCTGC 28

(2) INFORMATION ZU SEQ ID NO: 18:

(i) SEQUENZ CHARAKTERISTIKA:
(A) LÄNGE: 25 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..25

(xi) SEQUENZBESCHREIBUNG: SEQ ID NO: 18:

TTTGGATCCC TGGTTCTGTT TGAAG 25

(2) INFORMATION ZU SEQ ID NO: 19:

(i) SEQUENZ CHARAKTERISTIKA:
(A) LÄNGE: 50 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..50

(xi) SEQUENZBESCHREIBUNG: SEQ ID NO: 19:

TTCTAGAATT CAGCGGCCGC TTTTTTTTTT TTTTTTTTTT TTTTTTTTTT 50

(2) INFORMATION ZU SEQ ID NO: 20:

(i) SEQUENZ CHARAKTERISTIKA:
(A) LÄNGE: 27 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..27

(xi) SEQUENZBESCHREIBUNG: SEQ ID NO: 20:

GGGGAAAGTT CACTGTCAGT CTCCAAG 27

(2) INFORMATION ZU SEQ ID NO: 21:

(i) SEQUENZ CHARAKTERISTIKA:
(A) LÄNGE: 26 Basenpaare
(B) ART: Nukleinsäure
(C) STRANGFORM: Einzel
(D) TOPOLOGIE: linear

(ii) ART DES MOLEKÜLS: DNS (genomisch)

(xi) SEQUENZBESCHREIBUNG: SEQ ID NO: 21:

GGGAATACAC ACAGACGGCT GAGTAG 26

(2) INFORMATION ZU SEQ ID NO: 22:

(i) SEQUENZ CHARAKTERISTIKA:
(A) LÄNGE: 22 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..22

(xi) SEQUENZBESCHREIBUNG: SEQ ID NO: 22:

AGCAAGTTCA GCCTGGTTAA GT 22

(2) INFORMATION ZU SEQ ID NO: 23:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..21

(xi) SEQUENZBESCHREIBUNG: SEQ ID NO: 23:

TTATGAGTAT TTCTTCCAGG G 21

(2) INFORMATION ZU SEQ ID NO: 24:

(i) SEQUENZ CHARAKTERISTIKA:
(A) LÄNGE: 26 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..26

(xi) SEQUENZBESCHREIBUNG: SEQ ID NO: 24:

CCATTAATCC AGCCGAGCCA CACAAG 26

(2) INFORMATION ZU SEQ ID NO: 25:

(i) SEQUENZ CHARAKTERISTIKA:
(A) LÄNGE: 25 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..25

(xi) SEQUENZBESCHREIBUNG: SEQ ID NO: 25:

CATCTACAGC TCCGAACGGT CAGTG 25

(2) INFORMATION ZU SEQ ID NO: 26:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..20

(xi) SEQUENZBESCHREIBUNG: SEQ ID NO: 26:

CAGCGGAAGC CCCAACCGAG 20

(2) INFORMATION ZU SEQ ID NO: 27:

(i) SEQUENZ CHARAKTERISTIKA:
(A) LÄNGE: 23 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..23

(xi) SEQUENZBESCHREIBUNG: SEQ ID NO: 27:

GGGATGACGC CTCCTCCGCC CGG 23

(2) INFORMATION ZU SEQ ID NO: 28:

(i) SEQUENZ CHARAKTERISTIKA:
(A) LÄNGE: 31 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..31

(xi) SEQUENZBESCHREIBUNG: SEQ ID NO: 28:

AAGCTTCACG TGGACCAGCA AGCCAAGAGT G 31

(2) INFORMATION ZU SEQ ID NO: 29:

(i) SEQUENZ CHARAKTERISTIKA:
(A) LÄNGE: 25 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..25

(xi) SEQUENZBESCHREIBUNG: SEQ ID NO: 29:

AAGCTTTTTC CGTCCTTCCG TCCGG 25

(2) INFORMATION ZU SEQ ID NO: 30:

(i) SEQUENZ CHARAKTERISTIKA:
(A) LÄNGE: 24 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..24

(xi) SEQUENZBESCHREIBUNG: SEQ ID NO: 30:

ATGGTGAGCA AGGGCGAGGA GCTG 24

(2) INFORMATION ZU SEQ ID NO: 31:

(i) SEQUENZ CHARAKTERISTIKA:
(A) LÄNGE: 24 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..24

(xi) SEQUENZBESCHREIBUNG: SEQ ID NO: 31:

CTTGTACAGC TCGTCCATGC CGAG 24

(2) INFORMATION ZU SEQ ID NO: 32:

(i) SEQUENZ CHARAKTERISTIKA:
(A) LÄNGE: 25 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..25

(xi) SEQUENZBESCHREIBUNG: SEQ ID NO: 32:

GGGTGGTGAG AGTTCGTTGT CTGTC 25

(2) INFORMATION ZU SEQ ID NO: 33:

(i) SEQUENZ CHARAKTERISTIKA:
(A) LÄNGE: 25 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..25

(xi) SEQUENZBESCHREIBUNG: SEQ ID NO: 33:

GAGCGATGAG GTACGGAAGA CTCTG 25

(2) INFORMATION ZU SEQ ID NO: 34:

(i) SEQUENZ CHARAKTERISTIKA:
(A) LÄNGE: 5856 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..5856

(xi) SEQUENZBESCHREIBUNG: SEQ ID NO: 34:

AGCGCCCAAT ACGCAAACCG CCTCTCCCCG CGCGTTGGCC GATTCATTAA TGCAGCTGGC 60

ACGACAGGTT TCCCGACTGG AAAGCGGGCA GTGAGCGCAA CGCAATTAAT GTGAGTTAGC 120

TCACTCATTA GGCACCCCAG GCTTTACACT TTATGCTTCC GGCTCGTATG TTGTGTGGAA 180

TTGTGAGCGG ATAACAATTT CACACAGGAA ACAGCTATGA CCATGATTAC GCCAAGCTTC 240

ACGTGGACCA GCAAGCCAAG AGTGAGTGTG GGCAGCACCC CCAGCCAGAG GGAGGCAGCC 300

AGGGCACAGG CATGACCCAG CAGGTGCTCG GCCATGATGC CGTCCTCGGG CAGCAGCTGC 360

CAGTGCTGAG CCTCGCGGAA GTAGGAGCCC TCCTGGGCCT CGCAGAAGTA GTGGCCGTAC 420

TGCTGCGCCG TGAGGTTCTC GATGAACAGG ATGCAGTTGG GGCTCTGGTG ACCAGGTTCG 480

CAGCTCTGCT CCACGTTCTC CTTGTGGCGC CATGAGTAGG TGGCGTGGCG GGATTCCATG 540

GGGCAGCTCA GGTAGTAGCG AGAGTTTGGG GCCAGGGAAA CCTTCTGCAG TGGGGCCTTG 600

TCTGGTTTGG GGTTGGGACA CTCCTTGTGT GGCTCGGCTG GATTAATGGA TTGCAGCACT 660

GACCGTTCGG AGCTGTAGAT GGAGATGCAG CGGCCCTGGT CCCAGCCGCA GTAGGGGTCT 720

CGGGACATGA GGCAACCGTG GCAGCCCCCG CCATAGACCT CACACAGGTC CAGGGGCACC 780

TGGCTCACCT CCCACTGGGA GCTCACATAC AGCTTCCTCC GCTCAGCATC CAGCGACATG 840

GTCTGGATGG CAGCCGCGCG GCGGAAGGGC TGGATCTCCA TGATGTTGAA GGCGAAGCTG 900

TGCTCCTGCT CCCCCGGTTC CACCACCTTG TGGATAGTGC CCCTGTCTGT AGTTAGGTAA 960

AGCACATGAA AGGTCTCCCC GTGGCTGGCT TGCATGCGGT GAACGGCCAC TTTCTGGTAG 1020

TGGTATTTAG AGTGGAACAA TGGCGTCTTC AGAGGCCCCA TGGGCTCCAC CCTCTGCGCC 1080

ACCTCTGGGT GACGGTCAGC CACCTGGAAG GTCTCTGTGG GTATCGGCTG CTGGTCTGGG 1140

AGGCACTTGC CAGGCCGCGG GTTGGGAAGG CTTGAGTGGT AGCCCTTGAG TGAGGAGGTA 1200

CGGAAGACCT TGTCAATGTC ACCGAGGGAA TACACACAGA CGGCTGAGTA GTTCCAGGGG 1260

TTGGAGAAAA CACCATAGAC CCTGGTGTCC CTCCACTGGC CGCTGGGGTC AGGGAGCAGG 1320

AAGACGTCTT GCAGCCTGTT GAAGTTCTTG TTGGTGGCAG CATCACTGCA TACCAGCATG 1380

GCTTTCAGAA AAGTGTTCCA CTTGGAGACT GACAGTGAAC TTTCCCCACC CTGGTCCCCC 1440

CTGCACAACT GGGCCACACG GGACACATTG AGAGGAGCCT CAGGATTCTT GTCAGGATTG 1500

TCCTCTCGGA AGAAGTAGTA GATCTTGTCA TCGTAAGCCT GGTCTTGGTG CACGATGGTG 1560

GCTTTGATGA ACTGTGGGTT CTGCATGACA GTATCACTGG TGTACAGCTC ACTCTCGCCC 1620

CGGATGCGGC GGAACCGAGG GATCTTCCCA TTGTATTCCT GCTTCCGGAT GGTGGAATAC 1680

ACCTCGTCCC CTTCAAACAG AACCAGGGAG TTCTCGTCCG GGCTGAAGGG GGCGTAGCCT 1740

CTCATCTCGC CAAGTGGCAC CACAGTGCCA TTCACCAGGT TCCAGCAGCT GGGGTGCCGG 1800

GCGTTGGTGC CACAGGCCAG CAGCCCCTCA CTCCGCCTCT CCAGGAGAGT GATGTAGTTC 1860

TCGCAGTCCC GCTTATCCAG ACAGGACCCC TTTGTGGAGC CGATATTCAC CGTGCGCACA 1920

GATGCGTTCT TGCCCTCGGG GAAGTCAAAG AGGTAGACCT TGCCACGTCC TCCCACCCAC 1980

ACAGAGGAGC TGCCTGGCTC GTGGAAAAGC ACCGTGTGCG GCTCAGTCTG GCCAAAGTCC 2040

ACCCGGTCCT GCCCTACATG GCCTTTCCAG ACGGCGAAGA TGCGGGGTCC GCTCCTTAGG 2100

TGGCCCTGGG CGGAGGCGGC GGCCGCCCAG AGCAGCAGCA GCAGCCGCAG CCGCAGCGGA 2160

AGCCCCAACC GAGCCGGCGG GCCAGGGACG CGGGCGCGCG GTGCGCTGGG GGCGGCACGT 2220

CCGGGCGGAG GAGGCGTCAT CCCAAGCCGA ATTCTGCAGA TATCCATCAC ACTGGCGGCC 2280

GCTCGAGCAT GCATCTAGAG GGCCCAATTC GCCCTATAGT GAGTCGTATT ACAATTCACT 2340

GGCCGTCGTT TTACAACGTC GTGACTGGGA AAACCCTGGC GTTACCCAAC TTAATCGCCT 2400

TGCAGCACAT CCCCCTTTCG CCAGCTGGCG TAATAGCGAA GAGGCCCGCA CCGATCGCCC 2460

TTCCCAACAG TTGCGCAGCC TGAATGGCGA ATGGGACGCG CCCTGTAGCG GCGCATTAAG 2520

CGCGGCGGGT GTGGTGGTTA CGCGCAGCGT GACCGCTACA CTTGCCAGCG CCCTAGCGCC 2580

CGCTCCTTTC GCTTTCTTCC CTTCCTTTCT CGCCACGTTC GCCGGCTTTC CCCGTCAAGC 2640

TCTAAATCGG GGGCTCCCTT TAGGGTTCCG ATTTAGAGCT TTACGGCACC TCGACCGCAA 2700

AAAACTTGAT TTGGGTGATG GTTCACGTAG TGGGCCATCG CCCTGATAGA CGGTTTTTCG 2760

CCCTTTGACG TTGGAGTCCA CGTTCTTTAA TAGTGGACTC TTGTTCCAAA CTGGAACAAC 2820

ACTCAACCCT ATCGCGGTCT ATTCTTTTGA TTTATAAGGG ATTTTGCCGA TTTCGGCCTA 2880

TTGGTTAAAA AATGAGCTGA TTTAACAAAT TCAGGGCGCA AGGGCTGCTA AAGGAACCGG 2940

AACACGTAGA AAGCCAGTCC GCAGAAACGG TGCTGACCCC GGATGAATGT CAGCTACTGG 3000

GCTATCTGGA CAAGGGAAAA CGCAAGCGCA AAGAGAAAGC AGGTAGCTTG CAGTGGGCTT 3060

ACATGGCGAT AGCTAGACTG GGCGGTTTTA TGGACAGCAA GCGAACCGGA ATTGCCAGCT 3120

GGGGCGCCCT CTGGTAAGGT TGGGAAGCCC TGCAAAGTAA ACTGGATGGC TTTCTTGCCG 3180

CCAAGGATCT GATGGCGCAG GGGATCAAGA TCTGATCAAG AGACAGGATG AGGATCGTTT 3240

CGCATGATTG AACAAGATGG ATTGCACGCA GGTTCTCCGG CCGCTTGGGT GGAGAGGCTA 3300

TTCGGCTATG ACTGGGCACA ACAGACAATC GGCTGCTCTG ATGCCGCCGT GTTCCGGCTG 3360

TCAGCGCAGG GGCGCCCGGT TCTTTTTGTC AAGACCGACC TGTCCGGTGC CCTGAATGAA 3420

CTGCAGGACG AGGCAGCGCG GCTATCGTGG CTGGCCACGA CGGGCGTTCC TTGCGCAGCT 3480

GTGCTCGACG TTGTCACTGA AGCGGGAAGG GACTGGCTGC TATTGGGCGA AGTGCCGGGG 3540

CAGGATCTCC TGTCATCTCG CCTTGCTCCT GCCGAGAAAG TATCCATCAT GGCTGATGCA 3600

ATGCGGCGGC TGCATACGCT TGATCCGGCT ACCTGCCCAT TCGACCACCA AGCGAAACAT 3660

CGCATCGAGC GAGCACGTAC TCGGATGGAA GCCGGTCTTG TCGATCAGGA TGATCTGGAC 3720

GAAGAGCATC AGGGGCTCGC GCCAGCCGAA CTGTTCGCCA GGCTCAAGGC GCGCATGCCC 3780

GACGGCGAGG ATCTCGTCGT GATCCATGGC GATGCCTGCT TGCCGAATAT CATGGTGGAA 3840

AATGGCCGCT TTTCTGGATT CAACGACTGT GGCCGGCTGG GTGTGGCGGA CCGCTATCAG 3900

GACATAGCGT TGGATACCCG TGATATTGCT GAAGAGCTTG GCGGCGAATG GGCTGACCGC 3960

TTCCTCGTGC TTTACGGTAT CGCCGCTCCC GATTCGCAGC GCATCGCCTT CTATCGCCTT 4020

CTTGACGAGT TCTTCTGAAT TGAAAAAGGA AGAGTATGAG TATTCAACAT TTCCGTGTCG 4080

CCCTTATTCC CTTTTTTGCG GCATTTTGCC TTCCTGTTTT TGCTCACCCA GAAACGCTGG 4140

TGAAAGTAAA AGATGCTGAA GATCAGTTGG GTGCACGAGT GGGTTACATC GAACTGGATC 4200

TCAACAGCGG TAAGATCCTT GAGAGTTTTC GCCCCGAAGA ACGTTTTCCA ATGATGAGCA 4260

CTTTTAAAGT TCTGCTATGT CATACACTAT TATCCCGTAT TGACGCCGGG CAAGAGCAAC 4320

TCGGTCGCCG GGCGCGGTAT TCTCAGAATG ACTTGGTTGA GTACTCACCA GTCACAGAAA 4380

AGCATCTTAC GGATGGCATG ACAGTAAGAG AATTATGCAG TGCTGCCATA ACCATGAGTG 4440

ATAACACTGC GGCCAACTTA CTTCTGACAA CGATCGGAGG ACCGAAGGAG CTAACCGCTT 4500

TTTTGCACAA CATGGGGGAT CATGTAACTC GCCTTGATCG TTGGGAACCG GAGCTGAATG 4560

AAGCCATACC AAACGACGAG AGTGACACCA CGATGCCTGT AGCAATGCCA ACAACGTTGC 4620

GCAAACTATT AACTGGCGAA CTACTTACTC TAGCTTCCCG GCAACAATTA ATAGACTGGA 4680

TGGAGGCGGA TAAAGTTGCA GGACCACTTC TGCGCTCGGC CCTTCCGGCT GGCTGGTTTA 4740

TTGCTGATAA ATCTGGAGCC GGTGAGCGTG GGTCTCGCGG TATCATTGCA GCACTGGGGC 4800

CAGATGGTAA GCCCTCCCGT ATCGTAGTTA TCTACACGAC GGGGAGTCAG GCAACTATGG 4860

ATGAACGAAA TAGACAGATC GCTGAGATAG GTGCCTCACT GATTAAGCAT TGGTAACTGT 4920

CAGACCAAGT TTACTCATAT ATACTTTAGA TTGATTTAAA ACTTCATTTT TAATTTAAAA 4980

GGATCTAGGT GAAGATCCTT TTTGATAATC TCATGACCAA AATCCCTTAA CGTGAGTTTT 5040

CGTTCCACTG AGCGTCAGAC CCCGTAGAAA AGATCAAAGG ATCTTCTTGA GATCCTTTTT 5100

TTCTGCGCGT AATCTGCTGC TTGCAAACAA AAAAACCACC GCTACCAGCG GTGGTTTGTT 5160

TGCCGGATCA AGAGCTACCA ACTCTTTTTC CGAAGGTAAC TGGCTTCAGC AGAGCGCAGA 5220

TACCAAATAC TGTCCTTCTA GTGTAGCCGT AGTTAGGCCA CCACTTCAAG AACTCTGTAG 5280

CACCGCCTAC ATACCTCGCT CTGCTAATCC TGTTACCAGT GGCTGCTGCC AGTGGCGATA 5340

AGTCGTGTCT TACCGGGTTG GACTCAAGAC GATAGTTACC GGATAAGGCG CAGCGGTCGG 5400

GCTGAACGGG GGGTTCGTGC ACACAGCCCA GCTTGGAGCG AACGACCTAC ACCGAACTGA 5460

GATACCTACA GCGTGAGCAT TGAGAAAGCG CCACGCTTCC CGAAGGGAGA AAGGCGGACA 5520

GGTATCCGGT AAGCGGCAGG GTCGGAACAG GAGAGCGCAC GAGGGAGCTT CCAGGGGGAA 5580

ACGCCTGGTA TCTTTATAGT CCTGTCGGGT TTCGCCACCT CTGACTTGAG CGTCGATTTT 5640

TGTGATGCTC GTCAGGGGGG CGGAGCCTAT GGAAAAACGC CAGCAACGCG GCCTTTTTAC 5700

GGTTCCTGGC CTTTTGCTGG CCTTTTGCTC ACATGTTCTT TCCTGCGTTA TCCCCTGATT 5760

CTGTGGATAA CCGTATTACC GCCTTTGAGT GAGCTGATAC CGCTCGCCGC AGCCGAACGA 5820

CCGAGCGCAG CGAGTCAGTG AGCGAGGAAG CGGAAG 5856

(2) INFORMATION ZU SEQ ID NO: 35:

(i) SEQUENZ CHARAKTERISTIKA:
(A) LÄNGE: 7475 Basenpaare
(B) ART: Nukleinsäure

(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..7475

(xi) SEQUENZBESCHREIBUNG: SEQ ID NO: 35:

GACGGATCGG GAGATCTCCC GATCCCCTAT GGTCGACTCT CAGTACAATC TGCTCTGATG 60

CCGCATAGTT AAGCCAGTAT CTGCTCCCTG CTTGTGTGTT GGAGGTCGCT GAGTAGTGCG 120

CGAGCAAAAT TTAAGCTACA ACAAGGCAAG GCTTGACCGA CAATTGCATG AAGAATCTGC 180

TTAGGGTTAG GCGTTTTGCG CTGCTTCGCG ATGTACGGGC CAGATATACG CGTTGACATT 240

GATTATTGAC TAGTTATTAA TAGTAATCAA TTACGGGGTC ATTAGTTCAT AGCCCATATA 300

TGGAGTTCCG CGTTACATAA CTTACGGTAA ATGGCCCGCC TGGCTGACCG CCCAACGACC 360

CCCGCCCATT GACGTCAATA ATGACGTATG TTCCCATAGT AACGCCAATA GGGACTTTCC 420

ATTGACGTCA ATGGGTGGAC TATTTACGGT AAACTGCCCA CTTGGCAGTA CATCAAGTGT 480

ATCATATGCC AAGTACGCCC CCTATTGACG TCAATGACGG TAAATGGCCC GCCTGGCATT 540

ATGCCCAGTA CATGACCTTA TGGGACTTTC CTACTTGGCA GTACATCTAC GTATTAGTCA 600

TCGCTATTAC CATGGTGATG CGGTTTTGGC AGTACATCAA TGGGCGTGGA TAGCGGTTTG 660

ACTCACGGGG ATTTCCAAGT CTCCACCCCA TTGACGTCAA TGGGAGTTTG TTTTGGCACC 720

AAAATCAACG GGACTTTCCA AAATGTCGTA ACAACTCCGC CCCATTGACG CAAATGGGCG 780

GTAGGCGTGT ACGGTGGGAG GTCTATATAA GCAGAGCTCT CTGGCTAACT AGAGAACCCA 840

CTGCTTACTG GCTTATCGAA ATTAATACGA CTCACTATAG GGAGACCCAA GCTGGCTAGC 900

GTTTAAACGG GCCCTCTAGA CTCGAGCGGC CGCCACTGTG CTGGATATCT GCAGAATTCG 960

GCTTGGGATG ACGCCTCCTC CGCCCGGACG TGCCGCCCCC AGCGCACCGC GCGCCCGCGT 1020

CCCTGGCCCG CCGGCTCGGT TGGGGCTTCC GCTGCGGCTG CGGCTGCTGC TGCTGCTCTG 1080

GGCGGCCGCC GCCTCCGCCC AGGGCCACCT AAGGAGCGGA CCCCGCATCT TCGCCGTCTG 1140

GAAAGGCCAT GTAGGGCAGG ACCGGGTGGA CTTTGGCCAG ACTGAGCCGC ACACGGTGCT 1200

TTTCCACGAG CCAGGCAGCT CCTCTGTGTG GGTGGGAGGA CGTGGCAAGG TCTACCTCTT 1260

TGACTTCCCC GAGGGCAAGA ACGCATCTGT GCGCACGGTG AATATCGGCT CCACAAAGGG 1320

GTCCTGTCTG GATAAGCGGG ACTGCGAGAA CTACATCACT CTCCTGGAGA GGCGGAGTGA 1380

GGGGCTGCTG GCCTGTGGCA CCAACGCCCG GCACCCCAGC TGCTGGAACC TGGTGAATGG 1440

CACTGTGGTG CCACTTGGCG AGATGAGAGG CTACGCCCCC TTCAGCCCGG ACGAGAACTC 1500

CCTGGTTCTG TTTGAAGGGG ACGAGGTGTA TTCCACCATC CGGAAGCAGG AATACAATGG 1560

GAAGATCCCT CGGTTCCGCC GCATCCGGGG CGAGAGTGAG CTGTACACCA GTGATACTGT 1620

CATGCAGAAC CCACAGTTCA TCAAAGCCAC CATCGTGCAC CAAGACCAGG CTTACGATGA 1680

CAAGATCTAC TACTTCTTCC GAGAGGACAA TCCTGACAAG AATCCTGAGG CTCCTCTCAA 1740

TGTGTCCCGT GTGGCCCAGT TGTGCAGGGG GGACCAGGGT GGGGAAAGTT CACTGTCAGT 1800

CTCCAAGTGG AACACTTTTC TGAAAGCCAT GCTGGTATGC AGTGATGCTG CCACCAACAA 1860

GAACTTCAAC AGGCTGCAAG ACGTCTTCCT GCTCCCTGAC CCCAGCGGCC AGTGGAGGGA 1920

CACCAGGGTC TATGGTGTTT TCTCCAACCC CTGGAACTAC TCAGCCGTCT GTGTGTATTC 1980

CCTCGGTGAC ATTGACAAGG TCTTCCGTAC CTCCTCACTC AAGGGCTACC ACTCAAGCCT 2040

TCCCAACCCG CGGCCTGGCA AGTGCCTCCC AGACCAGCAG CCGATACCCA CAGAGACCTT 2100

CCAGGTGGCT GACCGTCACC CAGAGGTGGC GCAGAGGGTG GAGCCCATGG GGCCTCTGAA 2160

GACGCCATTG TTCCACTCTA AATACCACTA CCAGAAAGTG GCCGTTCACC GCATGCAAGC 2220

CAGCCACGGG GAGACCTTTC ATGTGCTTTA CCTAACTACA GACAGGGGCA CTATCCACAA 2280

GGTGGTGGAA CCGGGGGAGC AGGAGCACAG CTTCGCCTTC AACATCATGG AGATCCAGCC 2340

CTTCCGCCGC GCGGCTGCCA TCCAGACCAT GTCGCTGGAT GCTGAGCGGA GGAAGCTGTA 2400

TGTGAGCTCC CAGTGGGAGG TGAGCCAGGT GCCCCTGGAC CTGTGTGAGG TCTATGGCGG 2460

GGGCTGCCAC GGTTGCCTCA TGTCCCGAGA CCCCTACTGC GGCTGGGACC AGGGCCGCTG 2520

CATCTCCATC TACAGCTCCG AACGGTCAGT GCTGCAATCC ATTAATCCAG CCGAGCCACA 2580

CAAGGAGTGT CCCAACCCCA AACCAGACAA GGCCCCACTG CAGAAGGTTT CCCTGGCCCC 2640

AAACTCTCGC TACTACCTGA GCTGCCCCAT GGAATCCCGC CACGCCACCT ACTCATGGCG 2700

CCACAAGGAG AACGTGGAGC AGAGCTGCGA ACCTGGTCAC CAGAGCCCCA ACTGCATCCT 2760

GTTCATCGAG AACCTCACGG CGCAGCAGTA CGGCCACTAC TTCTGCGAGG CCCAGGAGGG 2820

CTCCTACTTC CGCGAGGCTC AGCACTGGCA GCTGCTGCCC GAGGACGGCA TCATGGCCGA 2880

GCACCTGCTG GGTCATGCCT GTGCCCTGGC TGCCTCCCTC TGGCTGGGGG TGCTGCCCAC 2940

ACTCACTCTT GGCTTGCTGG TCCACGTGAA GCTTGGGCCC GAACAAAAAC TCATCTCAGA 3000

AGAGGATCTG AATAGCGCCG TCGACCATCA TCATCATCAT CATTGAGTTT AAACCGCTGA 3060

TCAGCCTCGA CTGTGCCTTC TAGTTGCCAG CCATCTGTTG TTTGCCCCTC CCCCGTGCCT 3120

TCCTTGACCC TGGAAGGTGC CACTCCCACT GTCCTTTCCT AATAAAATGA GGAAATTGCA 3180

TCGCATTGTC TGAGTAGGTG TCATTCTATT CTGGGGGGTG GGGTGGGGCA GGACAGCAAG 3240

GGGGAGGATT GGGAAGACAA TAGCAGGCAT GCTGGGGATG CGGTGGGCTC TATGGCTTCT 3300

GAGGCGGAAA GAACCAGCTG GGGCTCTAGG GGGTATCCCC ACGCGCCCTG TAGCGGCGCA 3360

TTAAGCGCGG CGGGTGTGGT GGTTACGCGC AGCGTGACCG CTACACTTGC CAGCGCCCTA 3420

GCGCCCGCTC CTTTCGCTTT CTTCCCTTCC TTTCTCGCCA CGTTCGCCGG CTTTCCCCGT 3480

CAAGCTCTAA ATCGGGGCAT CCCTTTAGGG TTCCGATTTA GTGCTTTACG GCACCTCGAC 3540

CCCAAAAAAC TTGATTAGGG TGATGGTTCA CGTAGTGGGC CATCGCCCTG ATAGACGGTT 3600

TTTCGCCCTT TGACGTTGGA GTCCACGTTC TTTAATAGTG GACTCTTGTT CCAAACTGGA 3660

ACAACACTCA ACCCTATCTC GGTCTATTCT TTTGATTTAT AAGGGATTTT GGGGATTTCG 3720

GCCTATTGGT TAAAAAATGA GCTGATTTAA CAAAAATTTA ACGCGAATTA ATTCTGTGGA 3780

ATGTGTGTCA GTTAGGGTGT GGAAAGTCCC CAGGCTCCCC AGGCAGGCAG AAGTATGCAA 3840

AGCATGCATC TCAATTAGTC AGCAACCAGG TGTGGAAAGT CCCCAGGCTC CCCAGCAGGC 3900

AGAAGTATGC AAAGCATGCA TCTCAATTAG TCAGCAACCA TAGTCCCGCC CCTAACTCCG 3960

CCCATCCCGC CCCTAACTCC GCCCAGTTCC GCCCATTCTC CGCCCCATGG CTGACTAATT 4020

TTTTTTATTT ATGCAGAGGC CGAGGCCGCC TCTGCCTCTG AGCTATTCCA GAAGTAGTGA 4080

GGAGGCTTTT TTGGAGGCCT AGGCTTTTGC AAAAAGCTCC CGGGAGCTTG TATATCCATT 4140

TTCGGATCTG ATCAAGAGAC AGGATGAGGA TCGTTTCGCA TGATTGAACA AGATGGATTG 4200

CACGCAGGTT CTCCGGCCGC TTGGGTGGAG AGGCTATTCG GCTATGACTG GGCACAACAG 4260

ACAATCGGCT GCTCTGATGC CGCCGTGTTC CGGCTGTCAG CGCAGGGGCG CCCGGTTCTT 4320

TTTGTCAAGA CCGACCTGTC CGGTGCCCTG AATGAACTGC AGGACGAGGC AGCGCGGCTA 4380

TCGTGGCTGG CCACGACGGG CGTTCCTTGC GCAGCTGTGC TCGACGTTGT CACTGAAGCG 4440

GGAAGGGACT GGCTGCTATT GGGCGAAGTG CCGGGGCAGG ATCTCCTGTC ATCTCACCTT 4500

GCTCCTGCCG AGAAAGTATC CATCATGGCT GATGCAATGC GGCGGCTGCA TACGCTTGAT 4560

CCGGCTACCT GCCCATTCGA CCACCAAGCG AAACATCGCA TCGAGCGAGC ACGTACTCGG 4620

ATGGAAGCCG GTCTTGTCGA TCAGGATGAT CTGGACGAAG AGCATCAGGG GCTCGCGCCA 4680

GCCGAACTGT TCGCCAGGCT CAAGGCGCGC ATGCCCGACG GCGAGGATCT CGTCGTGACC 4740

CATGGCGATG CCTGCTTGCC GAATATCATG GTGGAAAATG GCCGCTTTTC TGGATTCATC 4800

GACTGTGGCC GGCTGGGTGT GGCGGACCGC TATCAGGACA TAGCGTTGGC TACCCGTGAT 4860

ATTGCTGAAG AGCTTGGCGG CGAATGGGCT GACCGCTTCC TCGTGCTTTA CGGTATCGCC 4920

GCTCCCGATT CGCAGCGCAT CGCCTTCTAT CGCCTTCTTG ACGAGTTCTT CTGAGCGGGA 4980

CTCTGGGGTT CGAAATGACC GACCAAGCGA CGCCCAACCT GCCATCACGA GATTTCGATT 5040

CCACCGCCGC CTTCTATGAA AGGTTGGGCT TCGGAATCGT TTTCCGGGAC GCCGGCTGGA 5100

TGATCCTCCA GCGCGGGGAT CTCATGCTGG AGTTCTTCGC CCACCCCAAC TTGTTTATTG 5160

CAGCTTATAA TGGTTACAAA TAAAGCAATA GCATCACAAA TTTCACAAAT AAAGCATTTT 5220

TTTCACTGCA TTCTAGTTGT GGTTTGTCCA AACTCATCAA TGTATCTTAT CATGTCTGTA 5280

TACCGTCGAC CTCTAGCTAG AGCTTGGCGT AATCATGGTC ATAGCTGTTT CCTGTGTGAA 5340

ATTGTTATCC GCTCACAATT CCACACAACA TACGAGCCGG AAGCATAAAG TGTAAAGCCT 5400

GGGGTGCCTA ATGAGTGAGC TAACTCACAT TAATTGCGTT GCGCTCACTG CCCGCTTTCC 5460

AGTCGGGAAA CCTGTCGTGC CAGCTGCATT AATGAATCGG CCAACGCGCG GGGAGAGGCG 5520

GTTTGCGTAT TGGGCGCTCT TCCGCTTCCT CGCTCACTGA CTCGCTGCGC TCGGTCGTTC 5580

GGCTGCGGCG AGCGGTATCA GCTCACTCAA AGGCGGTAAT ACGGTTATCC ACAGAATCAG 5640

GGGATAACGC AGGAAAGAAC ATGTGAGCAA AAGGCCAGCA AAAGGCCAGG AACCGTAAAA 5700

AGGCCGCGTT GCTGGCGTTT TTCCATAGGC TCCGCCCCCC TGACGAGCAT CACAAAAATC 5760

GACGCTCAAG TCAGAGGTGG CGAAACCCGA CAGGACTATA AAGATACCAG GCGTTTCCCC 5820

CTGGAAGCTC CCTCGTGCGC TCTCCTGTTC CGACCCTGCC GCTTACCGGA TACCTGTCCG 5880

CCTTTCTCCC TTCGGGAAGC GTGGCGCTTT CTCAATGCTC ACGCTGTAGG TATCTCAGTT 5940

CGGTGTAGGT CGTTCGCTCC AAGCTGGGCT GTGTGCACGA ACCCCCCGTT CAGCCCGACC 6000

GCTGCGCCTT ATCCGGTAAC TATCGTCTTG AGTCCAACCC GGTAAGACAC GACTTATCGC 6060

CACTGGCAGC AGCCACTGGT AACAGGATTA GCAGAGCGAG GTATGTAGGC GGTGCTACAG 6120

AGTTCTTGAA GTGGTGGCCT AACTACGGCT ACACTAGAAG GACAGTATTT GGTATCTGCG 6180

CTCTGCTGAA GCCAGTTACC TTCGGAAAAA GAGTTGGTAG CTCTTGATCC GGCAAACAAA 6240

CCACCGCTGG TAGCGGTGGT TTTTTTGTTT GCAAGCAGCA GATTACGCGC AGAAAAAAAG 6300

GATCTCAAGA AGATCCTTTG ATCTTTTCTA CGGGGTCTGA CGCTCAGTGG AACGAAAACT 6360

CACGTTAAGG GATTTTGGTC ATGAGATTAT CAAAAAGGAT CTTCACCTAG ATCCTTTTAA 6420

ATTAAAAATG AAGTTTTAAA TCAATCTAAA GTATATATGA GTAAACTTGG TCTGACAGTT 6480

ACCAATGCTT AATCAGTGAG GCACCTATCT CAGCGATCTG TCTATTTCGT TCATCCATAG 6540

TTGCCTGACT CCCCGTCGTG TAGATAACTA CGATACGGGA GGGCTTACCA TCTGGCCCCA 6600

GTGCTGCAAT GATACCGCGA GACCCACGCT CACCGGCTCC AGATTTATCA GCAATAAACC 6660

AGCCAGCCGG AAGGGCCGAG CGCAGAAGTG GTCCTGCAAC TTTATCCGCC TCCATCCAGT 6720

CTATTAATTG TTGCCGGGAA GCTAGAGTAA GTAGTTCGCC AGTTAATAGT TTGCGCAACG 6780

TTGTTGCCAT TGCTACAGGC ATCGTGGTGT CACGCTCGTC GTTTGGTATG GCTTCATTCA 6840

GCTCCGGTTC CCAACGATCA AGGCGAGTTA CATGATCCCC CATGTTGTGC AAAAAAGCGG 6900

TTAGCTCCTT CGGTCCTCCG ATCGTTGTCA GAAGTAAGTT GGCCGCAGTG TTATCACTCA 6960

TGGTTATGGC AGCACTGCAT AATTCTCTTA CTGTCATGCC ATCCGTAAGA TGCTTTTCTG 7020

TGACTGGTGA GTACTCAACC AAGTCATTCT GAGAATAGTG TATGCGGCGA CCGAGTTGCT 7080

CTTGCCCGGC GTCAATACGG GATAATACCG CGCCACATAG CAGAACTTTA AAAGTGCTCA 7140

TCATTGGAAA ACGTTCTTCG GGGCGAAAAC TCTCAAGGAT CTTACCGCTG TTGAGATCCA 7200

GTTCGATGTA ACCCACTCGT GCACCCAACT GATCTTCAGC ATCTTTTACT TTCACCAGCG 7260

TTTCTGGGTG AGCAAAAACA GGAAGGCAAA ATGCCGCAAA AAAGGGAATA AGGGCGACAC 7320

GGAAATGTTG AATACTCATA CTCTTCCTTT TTCAATATTA TTGAAGCATT TATCAGGGTT 7380

ATTGTCTCAT GAGCGGATAC ATATTTGAAT GTATTTAGAA AAATAAACAA ATAGGGGTTC 7440

CGCGCACATT TCCCCGAAAA GTGCCACCTG ACGTC 7475

(2) INFORMATION ZU SEQ ID NO: 36:

(i) SEQUENZ CHARAKTERISTIKA:
(A) LÄNGE: 8192 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..8192

(xi) SEQUENZBESCHREIBUNG: SEQ ID NO: 36:

GACGGATCGG GAGATCTCCC GATCCCCTAT GGTCGACTCT CAGTACAATC TGCTCTGATG 60

CCGCATAGTT AAGCCAGTAT CTGCTCCCTG CTTGTGTGTT GGAGGTCGCT GAGTAGTGCG 120

CGAGCAAAAT TTAAGCTACA ACAAGGCAAG GCTTGACCGA CAATTGCATG AAGAATCTGC 180

TTAGGGTTAG GCGTTTTGCG CTGCTTCGCG ATGTACGGGC CAGATATACG CGTTGACATT 240

GATTATTGAC TAGTTATTAA TAGTAATCAA TTACGGGGTC ATTAGTTCAT AGCCCATATA 300

TGGAGTTCCG CGTTACATAA CTTACGGTAA ATGGCCCGCC TGGCTGACCG CCCAACGACC 360

CCCGCCCATT GACGTCAATA ATGACGTATG TTCCCATAGT AACGCCAATA GGGACTTTCC 420

ATTGACGTCA ATGGGTGGAC TATTTACGGT AAACTGCCCA CTTGGCAGTA CATCAAGTGT 480

ATCATATGCC AAGTACGCCC CCTATTGACG TCAATGACGG TAAATGGCCC GCCTGGCATT 540

ATGCCCAGTA CATGACCTTA TGGGACTTTC CTACTTGGCA GTACATCTAC GTATTAGTCA 600

TCGCTATTAC CATGGTGATG CGGTTTTGGC AGTACATCAA TGGGCGTGGA TAGCGGTTTG 660

ACTCACGGGG ATTTCCAAGT CTCCACCCCA TTGACGTCAA TGGGAGTTTG TTTTGGCACC 720

AAAATCAACG GGACTTTCCA AAATGTCGTA ACAACTCCGC CCCATTGACG CAAATGGGCG 780

GTAGGCGTGT ACGGTGGGAG GTCTATATAA GCAGAGCTCT CTGGCTAACT AGAGAACCCA 840

CTGCTTACTG GCTTATCGAA ATTAATACGA CTCACTATAG GGAGACCCAA GCTGGCTAGC 900

GTTTAAACGG GCCCTCTAGA CTCGAGCGGC CGCCACTGTG CTGGATATCT GCAGAATTCG 960

GCTTGGGATG ACGCCTCCTC CGCCCGGACG TGCCGCCCCC AGCGCACCGC GCGCCCGCGT 1020

CCCTGGCCCG CCGGCTCGGT TGGGGCTTCC GCTGCGGCTG CGGCTGCTGC TGCTGCTCTG 1080

GGCGGCCGCC GCCTCCGCCC AGGGCCACCT AAGGAGCGGA CCCCGCATCT TCGCCGTCTG 1140

GAAAGGCCAT GTAGGGCAGG ACCGGGTGGA CTTTGGCCAG ACTGAGCCGC ACACGGTGCT 1200

TTTCCACGAG CCAGGCAGCT CCTCTGTGTG GGTGGGAGGA CGTGGCAAGG TCTACCTCTT 1260

TGACTTCCCC GAGGGCAAGA ACGCATCTGT GCGCACGGTG AATATCGGCT CCACAAAGGG 1320

GTCCTGTCTG GATAAGCGGG ACTGCGAGAA CTACATCACT CTCCTGGAGA GGCGGAGTGA 1380

GGGGCTGCTG GCCTGTGGCA CCAACGCCCG GCACCCCAGC TGCTGGAACC TGGTGAATGG 1440

CACTGTGGTG CCACTTGGCG AGATGAGAGG CTACGCCCCC TTCAGCCCGG ACGAGAACTC 1500

CCTGGTTCTG TTTGAAGGGG ACGAGGTGTA TTCCACCATC CGGAAGCAGG AATACAATGG 1560

GAAGATCCCT CGGTTCCGCC GCATCCGGGG CGAGAGTGAG CTGTACACCA GTGATACTGT 1620

CATGCAGAAC CCACAGTTCA TCAAAGCCAC CATCGTGCAC CAAGACCAGG CTTACGATGA 1680

CAAGATCTAC TACTTCTTCC GAGAGGACAA TCCTGACAAG AATCCTGAGG CTCCTCTCAA 1740

TGTGTCCCGT GTGGCCCAGT TGTGCAGGGG GGACCAGGGT GGGGAAAGTT CACTGTCAGT 1800

CTCCAAGTGG AACACTTTTC TGAAAGCCAT GCTGGTATGC AGTGATGCTG CCACCAACAA 1860

GAACTTCAAC AGGCTGCAAG ACGTCTTCCT GCTCCCTGAC CCCAGCGGCC AGTGGAGGGA 1920

CACCAGGGTC TATGGTGTTT TCTCCAACCC CTGGAACTAC TCAGCCGTCT GTGTGTATTC 1980

CCTCGGTGAC ATTGACAAGG TCTTCCGTAC CTCCTCACTC AAGGGCTACC ACTCAAGCCT 2040

TCCCAACCCG CGGCCTGGCA AGTGCCTCCC AGACCAGCAG CCGATACCCA CAGAGACCTT 2100

CCAGGTGGCT GACCGTCACC CAGAGGTGGC GCAGAGGGTG GAGCCCATGG GGCCTCTGAA 2160

GACGCCATTG TTCCACTCTA AATACCACTA CCAGAAAGTG GCCGTTCACC GCATGCAAGC 2220

CAGCCACGGG GAGACCTTTC ATGTGCTTTA CCTAACTACA GACAGGGGCA CTATCCACAA 2280

GGTGGTGGAA CCGGGGGAGC AGGAGCACAG CTTCGCCTTC AACATCATGG AGATCCAGCC 2340

CTTCCGCCGC GCGGCTGCCA TCCAGACCAT GTCGCTGGAT GCTGAGCGGA GGAAGCTGTA 2400

TGTGAGCTCC CAGTGGGAGG TGAGCCAGGT GCCCCTGGAC CTGTGTGAGG TCTATGGCGG 2460

GGGCTGCCAC GGTTGCCTCA TGTCCCGAGA CCCCTACTGC GGCTGGGACC AGGGCCGCTG 2520

CATCTCCATC TACAGCTCCG AACGGTCAGT GCTGCAATCC ATTAATCCAG CCGAGCCACA 2580

CAAGGAGTGT CCCAACCCCA AACCAGACAA GGCCCCACTG CAGAAGGTTT CCCTGGCCCC 2640

AAACTCTCGC TACTACCTGA GCTGCCCCAT GGAATCCCGC CACGCCACCT ACTCATGGCG 2700

CCACAAGGAG AACGTGGAGC AGAGCTGCGA ACCTGGTCAC CAGAGCCCCA ACTGCATCCT 2760

GTTCATCGAG AACCTCACGG CGCAGCAGTA CGGCCACTAC TTCTGCGAGG CCCAGGAGGG 2820

CTCCTACTTC CGCGAGGCTC AGCACTGGCA GCTGCTGCCC GAGGACGGCA TCATGGCCGA 2880

GCACCTGCTG GGTCATGCCT GTGCCCTGGC TGCCTCCCTC TGGCTGGGGG TGCTGCCCAC 2940

ACTCACTCTT GGCTTGCTGG TCCACATGGT GAGCAAGGGC GAGGAGCTGT TCACCGGGGT 3000

GGTGCCCATC CTGGTCGAGC TGGACGGCGA CGTAAACGGC CACAAGTTCA GCGTGTCCGG 3060

CGAGGGCGAG GGCGATGCCA CCTACGGCAA GCTGACCCTG AAGTTCATCT GCACCACCGG 3120

CAAGCTGCCC GTGCCCTGGC CCACCCTCGT GACCACCCTG ACCTACGGCG TGCAGTGCTT 3180

CAGCCGCTAC CCCGACCACA TGAAGCAGCA CGACTTCTTC AAGTCCGCCA TGCCCGAAGG 3240

CTACGTCCAG GAGCGCACCA TCTTCTTCAA GGACGACGGC AACTACAAGA CCCGCGCCGA 3300

GGTGAAGTTC GAGGGCGACA CCCTGGTGAA CCGCATCGAG CTGAAGGGCA TCGACTTCAA 3360

GGAGGACGGC AACATCCTGG GGCACAAGCT GGAGTACAAC TACAACAGCC ACAACGTCTA 3420

TATCATGGCC GACAAGCAGA AGAACGGCAT CAAGGTGAAC TTCAAGATCC GCCACAACAT 3480

CGAGGACGGC AGCGTGCAGC TCGCCGACCA CTACCAGCAG AACACCCCCA TCGGCGACGG 3540

CCCCGTGCTG CTGCCCGACA ACCACTACCT GAGCACCCAG TCCGCCCTGA GCAAAGACCC 3600

CAACGAGAAG CGCGATCACA TGGTCCTGCT GGAGTTCGTG ACCGCCGCCG GGATCACTCT 3660

CGGCATGGAC GAGCTGTACA AGGTGAAGCT TGGGCCCGAA CAAAAACTCA TCTCAGAAGA 3720

GGATCTGAAT AGCGCCGTCG ACCATCATCA TCATCATCAT TGAGTTTAAA CCGCTGATCA 3780

GCCTCGACTG TGCCTTCTAG TTGCCAGCCA TCTGTTGTTT GCCCCTCCCC CGTGCCTTCC 3840

TTGACCCTGG AAGGTGCCAC TCCCACTGTC CTTTCCTAAT AAAATGAGGA AATTGCATCG 3900

CATTGTCTGA GTAGGTGTCA TTCTATTCTG GGGGGTGGGG TGGGGCAGGA CAGCAAGGGG 3960

GAGGATTGGG AAGACAATAG CAGGCATGCT GGGGATGCGG TGGGCTCTAT GGCTTCTGAG 4020

GCGGAAAGAA CCAGCTGGGG CTCTAGGGGG TATCCCCACG CGCCCTGTAG CGGCGCATTA 4080

AGCGCGGCGG GTGTGGTGGT TACGCGCAGC GTGACCGCTA CACTTGCCAG CGCCCTAGCG 4140

CCCGCTCCTT TCGCTTTCTT CCCTTCCTTT CTCGCCACGT TCGCCGGCTT TCCCCGTCAA 4200

GCTCTAAATC GGGGCATCCC TTTAGGGTTC CGATTTAGTG CTTTACGGCA CCTCGACCCC 4260

AAAAAACTTG ATTAGGGTGA TGGTTCACGT AGTGGGCCAT CGCCCTGATA GACGGTTTTT 4320

CGCCCTTTGA CGTTGGAGTC CACGTTCTTT AATAGTGGAC TCTTGTTCCA AACTGGAACA 4380

ACACTCAACC CTATCTCGGT CTATTCTTTT GATTTATAAG GGATTTTGGG GATTTCGGCC 4440

TATTGGTTAA AAAATGAGCT GATTTAACAA AAATTTAACG CGAATTAATT CTGTGGAATG 4500

TGTGTCAGTT AGGGTGTGGA AAGTCCCCAG GCTCCCCAGG CAGGCAGAAG TATGCAAAGC 4560

ATGCATCTCA ATTAGTCAGC AACCAGGTGT GGAAAGTCCC CAGGCTCCCC AGCAGGCAGA 4620

AGTATGCAAA GCATGCATCT CAATTAGTCA GCAACCATAG TCCCGCCCCT AACTCCGCCC 4680

ATCCCGCCCC TAACTCCGCC CAGTTCCGCC CATTCTCCGC CCCATGGCTG ACTAATTTTT 4740

TTTATTTATG CAGAGGCCGA GGCCGCCTCT GCCTCTGAGC TATTCCAGAA GTAGTGAGGA 4800

GGCTTTTTTG GAGGCCTAGG CTTTTGCAAA AAGCTCCCGG GAGCTTGTAT ATCCATTTTC 4860

GGATCTGATC AAGAGACAGG ATGAGGATCG TTTCGCATGA TTGAACAAGA TGGATTGCAC 4920

GCAGGTTCTC CGGCCGCTTG GGTGGAGAGG CTATTCGGCT ATGACTGGGC ACAACAGACA 4980

ATCGGCTGCT CTGATGCCGC CGTGTTCCGG CTGTCAGCGC AGGGGCGCCC GGTTCTTTTT 5040

GTCAAGACCG ACCTGTCCGG TGCCCTGAAT GAACTGCAGG ACGAGGCAGC GCGGCTATCG 5100

TGGCTGGCCA CGACGGGCGT TCCTTGCGCA GCTGTGCTCG ACGTTGTCAC TGAAGCGGGA 5160

AGGGACTGGC TGCTATTGGG CGAAGTGCCG GGGCAGGATC TCCTGTCATC TCACCTTGCT 5220

CCTGCCGAGA AAGTATCCAT CATGGCTGAT GCAATGCGGC GGCTGCATAC GCTTGATCCG 5280

GCTACCTGCC CATTCGACCA CCAAGCGAAA CATCGCATCG AGCGAGCACG TACTCGGATG 5340

GAAGCCGGTC TTGTCGATCA GGATGATCTG GACGAAGAGC ATCAGGGGCT CGCGCCAGCC 5400

GAACTGTTCG CCAGGCTCAA GGCGCGCATG CCCGACGGCG AGGATCTCGT CGTGACCCAT 5460

GGCGATGCCT GCTTGCCGAA TATCATGGTG GAAAATGGCC GCTTTTCTGG ATTCATCGAC 5520

TGTGGCCGGC TGGGTGTGGC GGACCGCTAT CAGGACATAG CGTTGGCTAC CCGTGATATT 5580

GCTGAAGAGC TTGGCGGCGA ATGGGCTGAC CGCTTCCTCG TGCTTTACGG TATCGCCGCT 5640

CCCGATTCGC AGCGCATCGC CTTCTATCGC CTTCTTGACG AGTTCTTCTG AGCGGGACTC 5700

TGGGGTTCGA AATGACCGAC CAAGCGACGC CCAACCTGCC ATCACGAGAT TTCGATTCCA 5760

CCGCCGCCTT CTATGAAAGG TTGGGCTTCG GAATCGTTTT CCGGGACGCC GGCTGGATGA 5820

TCCTCCAGCG CGGGGATCTC ATGCTGGAGT TCTTCGCCCA CCCCAACTTG TTTATTGCAG 5880

CTTATAATGG TTACAAATAA AGCAATAGCA TCACAAATTT CACAAATAAA GCATTTTTTT 5940

CACTGCATTC TAGTTGTGGT TTGTCCAAAC TCATCAATGT ATCTTATCAT GTCTGTATAC 6000

CGTCGACCTC TAGCTAGAGC TTGGCGTAAT CATGGTCATA GCTGTTTCCT GTGTGAAATT 6060

GTTATCCGCT CACAATTCCA CACAACATAC GAGCCGGAAG CATAAAGTGT AAAGCCTGGG 6120

GTGCCTAATG AGTGAGCTAA CTCACATTAA TTGCGTTGCG CTCACTGCCC GCTTTCCAGT 6180

CGGGAAACCT GTCGTGCCAG CTGCATTAAT GAATCGGCCA ACGCGCGGGG AGAGGCGGTT 6240

TGCGTATTGG GCGCTCTTCC GCTTCCTCGC TCACTGACTC GCTGCGCTCG GTCGTTCGGC 6300

TGCGGCGAGC GGTATCAGCT CACTCAAAGG CGGTAATACG GTTATCCACA GAATCAGGGG 6360

ATAACGCAGG AAAGAACATG TGAGCAAAAG GCCAGCAAAA GGCCAGGAAC CGTAAAAAGG 6420

CCGCGTTGCT GGCGTTTTTC CATAGGCTCC GCCCCCCTGA CGAGCATCAC AAAAATCGAC 6480

GCTCAAGTCA GAGGTGGCGA AACCCGACAG GACTATAAAG ATACCAGGCG TTTCCCCCTG 6540

GAAGCTCCCT CGTGCGCTCT CCTGTTCCGA CCCTGCCGCT TACCGGATAC CTGTCCGCCT 6600

TTCTCCCTTC GGGAAGCGTG GCGCTTTCTC AATGCTCACG CTGTAGGTAT CTCAGTTCGG 6660

TGTAGGTCGT TCGCTCCAAG CTGGGCTGTG TGCACGAACC CCCCGTTCAG CCCGACCGCT 6720

GCGCCTTATC CGGTAACTAT CGTCTTGAGT CCAACCCGGT AAGACACGAC TTATCGCCAC 6780

TGGCAGCAGC CACTGGTAAC AGGATTAGCA GAGCGAGGTA TGTAGGCGGT GCTACAGAGT 6840

TCTTGAAGTG GTGGCCTAAC TACGGCTACA CTAGAAGGAC AGTATTTGGT ATCTGCGCTC 6900

TGCTGAAGCC AGTTACCTTC GGAAAAAGAG TTGGTAGCTC TTGATCCGGC AAACAAACCA 6960

CCGCTGGTAG CGGTGGTTTT TTTGTTTGCA AGCAGCAGAT TACGCGCAGA AAAAAAGGAT 7020

CTCAAGAAGA TCCTTTGATC TTTTCTACGG GGTCTGACGC TCAGTGGAAC GAAAACTCAC 7080

GTTAAGGGAT TTTGGTCATG AGATTATCAA AAAGGATCTT CACCTAGATC CTTTTAAATT 7140

AAAAATGAAG TTTTAAATCA ATCTAAAGTA TATATGAGTA AACTTGGTCT GACAGTTACC 7200

AATGCTTAAT CAGTGAGGCA CCTATCTCAG CGATCTGTCT ATTTCGTTCA TCCATAGTTG 7260

CCTGACTCCC CGTCGTGTAG ATAACTACGA TACGGGAGGG CTTACCATCT GGCCCCAGTG 7320

CTGCAATGAT ACCGCGAGAC CCACGCTCAC CGGCTCCAGA TTTATCAGCA ATAAACCAGC 7380

CAGCCGGAAG GGCCGAGCGC AGAAGTGGTC CTGCAACTTT ATCCGCCTCC ATCCAGTCTA 7440

TTAATTGTTG CCGGGAAGCT AGAGTAAGTA GTTCGCCAGT TAATAGTTTG CGCAACGTTG 7500

TTGCCATTGC TACAGGCATC GTGGTGTCAC GCTCGTCGTT TGGTATGGCT TCATTCAGCT 7560

CCGGTTCCCA ACGATCAAGG CGAGTTACAT GATCCCCCAT GTTGTGCAAA AAAGCGGTTA 7620

GCTCCTTCGG TCCTCCGATC GTTGTCAGAA GTAAGTTGGC CGCAGTGTTA TCACTCATGG 7680

TTATGGCAGC ACTGCATAAT TCTCTTACTG TCATGCCATC CGTAAGATGC TTTTCTGTGA 7740

CTGGTGAGTA CTCAACCAAG TCATTCTGAG AATAGTGTAT GCGGCGACCG AGTTGCTCTT 7800

GCCCGGCGTC AATACGGGAT AATACCGCGC CACATAGCAG AACTTTAAAA GTGCTCATCA 7860

TTGGAAAACG TTCTTCGGGG CGAAAACTCT CAAGGATCTT ACCGCTGTTG AGATCCAGTT 7920

CGATGTAACC CACTCGTGCA CCCAACTGAT CTTCAGCATC TTTTACTTTC ACCAGCGTTT 7980

CTGGGTGAGC AAAAACAGGA AGGCAAAATG CCGCAAAAAA GGGAATAAGG GCGACACGGA 8040

AATGTTGAAT ACTCATACTC TTCCTTTTTC AATATTATTG AAGCATTTAT CAGGGTTATT 8100

GTCTCATGAG CGGATACATA TTTGAATGTA TTTAGAAAAA TAAACAAATA GGGGTTCCGC 8160

GCACATTTCC CCGAAAAGTG CCACCTGACG TC 8192

(2) INFORMATION ZU SEQ ID NO: 37:

(i) SEQUENZ CHARAKTERISTIKA:
(A) LÄNGE: 7000 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..7000

(xi) SEQUENZBESCHREIBUNG: SEQ ID NO: 37:

AGATCTCGGC CGCATATTAA GTGCATTGTT CTCGATACCG CTAAGTGCAT TGTTCTCGTT 60

AGCTCGATGG ACAAGTGCAT TGTTCTCTTG CTGAAAGCTC GATGGACAAG TGCATTGTTC 120

TCTTGCTGAA AGCTCGATGG ACAAGTGCAT TGTTCTCTTG CTGAAAGCTC AGTACCCGGG 180

AGTACCCTCG ACCGCCGGAG TATAAATAGA GGCGCTTCGT CTACGGAGCG ACAATTCAAT 240

TCAAACAAGC AAAGTGAACA CGTCGCTAAG CGAAAGCTAA GCAAATAAAC AAGCGCAGCT 300

GAACAAGCTA AACAATCTGC AGTAAAGTGC AAGTTAAAGT GAATCAATTA AAAGTAACCA 360

GCAACCAAGT AAATCAACTG CAACTACTGA AATCTGCCAA GAAGTAATTA TTGAATACAA 420

GAAGAGAACT CTGAATACTT TCAACAAGTT ACCGAGAAAG AAGAACTCAC ACACAGCTAG 480

CGTTTAAACT TAAGCTTGGT ACCGAGCTCG GATCCACTAG TCCAGTGTGG TGGAATTCGG 540

CTTGGGATGA CGCCTCCTCC GCCCGGACGT GCCGCCCCCA GCGCACCGCG CGCCCGCGTC 600

CCTGGCCCGC CGGCTCGGTT GGGGCTTCCG CTGCGGCTGC GGCTGCTGCT GCTGCTCTGG 660

GCGGCCGCCG CCTCCGCCCA GGGCCACCTA AGGAGCGGAC CCCGCATCTT CGCCGTCTGG 720

AAAGGCCATG TAGGGCAGGA CCGGGTGGAC TTTGGCCAGA CTGAGCCGCA CACGGTGCTT 780

TTCCACGAGC CAGGCAGCTC CTCTGTGTGG GTGGGAGGAC GTGGCAAGGT CTACCTCTTT 840

GACTTCCCCG AGGGCAAGAA CGCATCTGTG CGCACGGTGA ATATCGGCTC CACAAAGGGG 900

TCCTGTCTGG ATAAGCGGGA CTGCGAGAAC TACATCACTC TCCTGGAGAG GCGGAGTGAG 960

GGGCTGCTGG CCTGTGGCAC CAACGCCCGG CACCCCAGCT GCTGGAACCT GGTGAATGGC 1020

ACTGTGGTGC CACTTGGCGA GATGAGAGGC TACGCCCCCT TCAGCCCGGA CGAGAACTCC 1080

CTGGTTCTGT TTGAAGGGGA CGAGGTGTAT TCCACCATCC GGAAGCAGGA ATACAATGGG 1140

AAGATCCCTC GGTTCCGCCG CATCCGGGGC GAGAGTGAGC TGTACACCAG TGATACTGTC 1200

ATGCAGAACC CACAGTTCAT CAAAGCCACC ATCGTGCACC AAGACCAGGC TTACGATGAC 1260

AAGATCTACT ACTTCTTCCG AGAGGACAAT CCTGACAAGA ATCCTGAGGC TCCTCTCAAT 1320

GTGTCCCGTG TGGCCCAGTT GTGCAGGGGG GACCAGGGTG GGGAAAGTTC ACTGTCAGTC 1380

TCCAAGTGGA ACACTTTTCT GAAAGCCATG CTGGTATGCA GTGATGCTGC CACCAACAAG 1440

AACTTCAACA GGCTGCAAGA CGTCTTCCTG CTCCCTGACC CCAGCGGCCA GTGGAGGGAC 1500

ACCAGGGTCT ATGGTGTTTT CTCCAACCCC TGGAACTACT CAGCCGTCTG TGTGTATTCC 1560

CTCGGTGACA TTGACAAGGT CTTCCGTACC TCCTCACTCA AGGGCTACCA CTCAAGCCTT 1620

CCCAACCCGC GGCCTGGCAA GTGCCTCCCA GACCAGCAGC CGATACCCAC AGAGACCTTC 1680

CAGGTGGCTG ACCGTCACCC AGAGGTGGCG CAGAGGGTGG AGCCCATGGG GCCTCTGAAG 1740

ACGCCATTGT TCCACTCTAA ATACCACTAC CAGAAAGTGG CCGTTCACCG CATGCAAGCC 1800

AGCCACGGGG AGACCTTTCA TGTGCTTTAC CTAACTACAG ACAGGGGCAC TATCCACAAG 1860

GTGGTGGAAC CGGGGGAGCA GGAGCACAGC TTCGCCTTCA ACATCATGGA GATCCAGCCC 1920

TTCCGCCGCG CGGCTGCCAT CCAGACCATG TCGCTGGATG CTGAGCGGAG GAAGCTGTAT 1980

GTGAGCTCCC AGTGGGAGGT GAGCCAGGTG CCCCTGGACC TGTGTGAGGT CTATGGCGGG 2040

GGCTGCCACG GTTGCCTCAT GTCCCGAGAC CCCTACTGCG GCTGGGACCA GGGCCGCTGC 2100

ATCTCCATCT ACAGCTCCGA ACGGTCAGTG CTGCAATCCA TTAATCCAGC CGAGCCACAC 2160

AAGGAGTGTC CCAACCCCAA ACCAGACAAG GCCCCACTGC AGAAGGTTTC CCTGGCCCCA 2220

AACTCTCGCT ACTACCTGAG CTGCCCCATG GAATCCCGCC ACGCCACCTA CTCATGGCGC 2280

CACAAGGAGA ACGTGGAGCA GAGCTGCGAA CCTGGTCACC AGAGCCCCAA CTGCATCCTG 2340

TTCATCGAGA ACCTCACGGC GCAGCAGTAC GGCCACTACT TCTGCGAGGC CCAGGAGGGC 2400

TCCTACTTCC GCGAGGCTCA GCACTGGCAG CTGCTGCCCG AGGACGGCAT CATGGCCGAG 2460

CACCTGCTGG GTCATGCCTG TGCCCTGGCT GCCTCCCTCT GGCTGGGGGT GCTGCCCACA 2520

CTCACTCTTG GCTTGCTGGT CCACGTGAAG CTTGGGCCCG TTTAAACCCG CTGATCAGCC 2580

TCGACTGTGC CTTCTAGTTG CCAGCCATCT GTTGTTTGCC CCTCCCCCGT GCCTTCCTTG 2640

ACCCTGGAAG GTGCCACTCC CACTGTCCTT TCCTAATAAA ATGAGGAAAT TGCATCGCAT 2700

TGTCTGAGTA GGTGTCATTC TATTCTGGGG GGTGGGGTGG GGCAGGACAG CAAGGGGGAG 2760

GATTGGGAAG ACAATAGCAG GCATGCTGGG GATGCGGTGG GCTCTATGGC TTCTGAGGCG 2820

GAAAGAACCA GCTGGGGCTC TAGGGGGTAT CCCCACGCGC CCTGTAGCGG CGCATTAAGC 2880

GCGGCGGGTG TGGTGGTTAC GCGCAGCGTG ACCGCTACAC TTGCCAGCGC CCTAGCGCCC 2940

GCTCCTTTCG CTTTCTTCCC TTCCTTTCTC GCCACGTTCG CCGGCTTTCC CCGTCAAGCT 3000

CTAAATCGGG GCATCCCTTT AGGGTTCCGA TTTAGTGCTT TACGGCACCT CGACCCCAAA 3060

AAACTTGATT AGGGTGATGG TTCACGTAGT GGGCCATCGC CCTGATAGAC GGTTTTTCGC 3120

CCTTTGACGT TGGAGTCCAC GTTCTTTAAT AGTGGACTCT TGTTCCAAAC TGGAACAACA 3180

CTCAACCCTA TCTCGGTCTA TTCTTTTGAT TTATAAGGGA TTTTGGGGAT TTCGGCCTAT 3240

TGGTTAAAAA ATGAGCTGAT TTAACAAAAA TTTAACGCGA ATTAATTCTG TGGAATGTGT 3300

GTCAGTTAGG GTGTGGAAAG TCCCCAGGCT CCCCAGGCAG GCAGAAGTAT GCAAAGCATG 3360

CATCTCAATT AGTCAGCAAC CAGGTGTGGA AAGTCCCCAG GCTCCCCAGC AGGCAGAAGT 3420

ATGCAAAGCA TGCATCTCAA TTAGTCAGCA ACCATAGTCC CGCCCCTAAC TCCGCCCATC 3480

CCGCCCCTAA CTCCGCCCAG TTCCGCCCAT TCTCCGCCCC ATGGCTGACT AATTTTTTTT 3540

ATTTATGCAG AGGCCGAGGC CGCCTCTGCC TCTGAGCTAT TCCAGAAGTA GTGAGGAGGC 3600

TTTTTTGGAG GCCTAGGCTT TTGCAAAAAG CTCCCGGGAG CTTGTATATC CATTTTCGGA 3660

TCTGATCAAG AGACAGGATG AGGATCGTTT CGCATGATTG AACAAGATGG ATTGCACGCA 3720

GGTTCTCCGG CCGCTTGGGT GGAGAGGCTA TTCGGCTATG ACTGGGCACA ACAGACAATC 3780

GGCTGCTCTG ATGCCGCCGT GTTCCGGCTG TCAGCGCAGG GGCGCCCGGT TCTTTTTGTC 3840

AAGACCGACC TGTCCGGTGC CCTGAATGAA CTGCAGGACG AGGCAGCGCG GCTATCGTGG 3900

CTGGCCACGA CGGGCGTTCC TTGCGCAGCT GTGCTCGACG TTGTCACTGA AGCGGGAAGG 3960

GACTGGCTGC TATTGGGCGA AGTGCCGGGG CAGGATCTCC TGTCATCTCA CCTTGCTCCT 4020

GCCGAGAAAG TATCCATCAT GGCTGATGCA ATGCGGCGGC TGCATACGCT TGATCCGGCT 4080

ACCTGCCCAT TCGACCACCA AGCGAAACAT CGCATCGAGC GAGCACGTAC TCGGATGGAA 4140

GCCGGTCTTG TCGATCAGGA TGATCTGGAC GAAGAGCATC AGGGGCTCGC GCCAGCCGAA 4200

CTGTTCGCCA GGCTCAAGGC GCGCATGCCC GACGGCGAGG ATCTCGTCGT GACCCATGGC 4260
GATGCCTGCT TGCCGAATAT CATGGTGGAA AATGGCCGCT TTTCTGGATT CATCGACTGT 4320
GGCCGGCTGG GTGTGGCGGA CCGCTATCAG GACATAGCGT TGGCTACCCG TGATATTGCT 4380
GAAGAGCTTG GCGGCGAATG GGCTGACCGC TTCCTCGTGC TTTACGGTAT CGCCGCTCCC 4440
GATTCGCAGC GCATCGCCTT CTATCGCCTT CTTGACGAGT TCTTCTGAGC GGGACTCTGG 4500
GGTTCGAAAT GACCGACCAA GCGACGCCCA ACCTGCCATC ACGAGATTTC GATTCCACCG 4560
CCGCCTTCTA TGAAAGGTTG GGCTTCGGAA TCGTTTTCCG GGACGCCGGC TGGATGATCC 4620
TCCAGCGCGG GGATCTCATG CTGGAGTTCT TCGCCCACCC CAACTTGTTT ATTGCAGCTT 4680
ATAATGGTTA CAAATAAAGC AATAGCATCA CAAATTTCAC AAATAAAGCA TTTTTTTCAC 4740
TGCATTCTAG TTGTGGTTTG TCCAAACTCA TCAATGTATC TTATCATGTC TGTATACCGT 4800
CGACCTCTAG CTAGAGCTTG GCGTAATCAT GGTCATAGCT GTTTCCTGTG TGAAATTGTT 4860
ATCCGCTCAC AATTCCACAC AACATACGAG CCGGAAGCAT AAAGTGTAAA GCCTGGGGTG 4920
CCTAATGAGT GAGCTAACTC ACATTAATTG CGTTGCGCTC ACTGCCCGCT TTCCAGTCGG 4980
GAAACCTGTC GTGCCAGCTG CATTAATGAA TCGGCCAACG CGCGGGGAGA GGCGGTTTGC 5040
GTATTGGGCG CTCTTCCGCT TCCTCGCTCA CTGACTCGCT GCGCTCGGTC GTTCGGCTGC 5100
GGCGAGCGGT ATCAGCTCAC TCAAAGGCGG TAATACGGTT ATCCACAGAA TCAGGGGATA 5160
ACGCAGGAAA GAACATGTGA GCAAAAGGCC AGCAAAAGGC CAGGAACCGT AAAAAGGCCG 5220
CGTTGCTGGC GTTTTTCCAT AGGCTCCGCC CCCCTGACGA GCATCACAAA AATCGACGCT 5280
CAAGTCAGAG GTGGCGAAAC CCGACAGGAC TATAAAGATA CCAGGCGTTT CCCCCTGGAA 5340
GCTCCCTCGT GCGCTCTCCT GTTCCGACCC TGCCGCTTAC CGGATACCTG TCCGCCTTTC 5400
TCCCTTCGGG AAGCGTGGCG CTTTCTCAAT GCTCACGCTG TAGGTATCTC AGTTCGGTGT 5460
AGGTCGTTCG CTCCAAGCTG GGCTGTGTGC ACGAACCCCC CGTTCAGCCC GACCGCTGCG 5520
CCTTATCCGG TAACTATCGT CTTGAGTCCA ACCCGGTAAG ACACGACTTA TCGCCACTGG 5580
CAGCAGCCAC TGGTAACAGG ATTAGCAGAG CGAGGTATGT AGGCGGTGCT ACAGAGTTCT 5640
TGAAGTGGTG GCCTAACTAC GGCTACACTA GAAGGACAGT ATTTGGTATC TGCGCTCTGC 5700
TGAAGCCAGT TACCTTCGGA AAAAGAGTTG GTAGCTCTTG ATCCGGCAAA CAAACCACCG 5760
CTGGTAGCGG TGGTTTTTTT GTTTGCAAGC AGCAGATTAC GCGCAGAAAA AAAGGATCTC 5820
AAGAAGATCC TTTGATCTTT TCTACGGGGT CTGACGCTCA GTGGAACGAA AACTCACGTT 5880

AAGGGATTTT GGTCATGAGA TTATCAAAAA GGATCTTCAC CTAGATCCTT TTAAATTAAA 5940

AATGAAGTTT TAAATCAATC TAAAGTATAT ATGAGTAAAC TTGGTCTGAC AGTTACCAAT 6000

GCTTAATCAG TGAGGCACCT ATCTCAGCGA TCTGTCTATT TCGTTCATCC ATAGTTGCCT 6060

GACTCCCCGT CGTGTAGATA ACTACGATAC GGGAGGGCTT ACCATCTGGC CCCAGTGCTG 6120

CAATGATACC GCGAGACCCA CGCTCACCGG CTCCAGATTT ATCAGCAATA AACCAGCCAG 6180

CCGGAAGGGC CGAGCGCAGA AGTGGTCCTG CAACTTTATC CGCCTCCATC CAGTCTATTA 6240

ATTGTTGCCG GGAAGCTAGA GTAAGTAGTT CGCCAGTTAA TAGTTTGCGC AACGTTGTTG 6300

CCATTGCTAC AGGCATCGTG GTGTCACGCT CGTCGTTTGG TATGGCTTCA TTCAGCTCCG 6360

GTTCCCAACG ATCAAGGCGA GTTACATGAT CCCCCATGTT GTGCAAAAAA GCGGTTAGCT 6420

CCTTCGGTCC TCCGATCGTT GTCAGAAGTA AGTTGGCCGC AGTGTTATCA CTCATGGTTA 6480

TGGCAGCACT GCATAATTCT CTTACTGTCA TGCCATCCGT AAGATGCTTT TCTGTGACTG 6540

GTGAGTACTC AACCAAGTCA TTCTGAGAAT AGTGTATGCG GCGACCGAGT TGCTCTTGCC 6600

CGGCGTCAAT ACGGGATAAT ACCGCGCCAC ATAGCAGAAC TTTAAAAGTG CTCATCATTG 6660

GAAAACGTTC TTCGGGGCGA AAACTCTCAA GGATCTTACC GCTGTTGAGA TCCAGTTCGA 6720

TGTAACCCAC TCGTGCACCC AACTGATCTT CAGCATCTTT TACTTTCACC AGCGTTTCTG 6780

GGTGAGCAAA AACAGGAAGG CAAAATGCCG CAAAAAAGGG AATAAGGGCG ACACGGAAAT 6840

GTTGAATACT CATACTCTTC CTTTTTCAAT ATTATTGAAG CATTTATCAG GGTTATTGTC 6900

TCATGAGCGG ATACATATTT GAATGTATTT AGAAAAATAA ACAAATAGGG GTTCCGCGCA 6960

CATTTCCCCG AAAAGTGCCA CCTGACGTCG ACGGATCGGG 7000

(2) INFORMATION ZU SEQ ID NO: 38:

(i) SEQUENZ CHARAKTERISTIKA:
(A) LÄNGE: 7108 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..7108

(xi) SEQUENZBESCHREIBUNG: SEQ ID NO: 38:

AGATCTCGGC CGCATATTAA GTGCATTGTT CTCGATACCG CTAAGTGCAT TGTTCTCGTT 60

AGCTCGATGG ACAAGTGCAT TGTTCTCTTG CTGAAAGCTC GATGGACAAG TGCATTGTTC 120

TCTTGCTGAA AGCTCGATGG ACAAGTGCAT TGTTCTCTTG CTGAAAGCTC AGTACCCGGG 180

AGTACCCTCG ACCGCCGGAG TATAAATAGA GGCGCTTCGT CTACGGAGCG ACAATTCAAT 240

TCAAACAAGC AAAGTGAACA CGTCGCTAAG CGAAAGCTAA GCAAATAAAC AAGCGCAGCT 300

GAACAAGCTA AACAATCTGC AGTAAAGTGC AAGTTAAAGT GAATCAATTA AAAGTAACCA 360

GCAACCAAGT AAATCAACTG CAACTACTGA AATCTGCCAA GAAGTAATTA TTGAATACAA 420

GAAGAGAACT CTGAATACTT TCAACAAGTT ACCGAGAAAG AAGAACTCAC ACACAGCTAG 480

CGTTTAAACT TAAGCTTGGT ACCGAGCTCG GATCCACTAG TCCAGTGTGG TGGAATTCGG 540

CTTGGGATGA CGCCTCCTCC GCCCGGACGT GCCGCCCCCA GCGCACCGCG CGCCCGCGTC 600

CCTGGCCCGC CGGCTCGGTT GGGGCTTCCG CTGCGGCTGC GGCTGCTGCT GCTGCTCTGG 660

GCGGCCGCCG CCTCCGCCCA GGGCCACCTA AGGAGCGGAC CCCGCATCTT CGCCGTCTGG 720

AAAGGCCATG TAGGGCAGGA CCGGGTGGAC TTTGGCCAGA CTGAGCCGCA CACGGTGCTT 780

TTCCACGAGC CAGGCAGCTC CTCTGTGTGG GTGGGAGGAC GTGGCAAGGT CTACCTCTTT 840

GACTTCCCCG AGGGCAAGAA CGCATCTGTG CGCACGGTGA ATATCGGCTC CACAAAGGGG 900

TCCTGTCTGG ATAAGCGGGA CTGCGAGAAC TACATCACTC TCCTGGAGAG GCGGAGTGAG 960

GGGCTGCTGG CCTGTGGCAC CAACGCCCGG CACCCCAGCT GCTGGAACCT GGTGAATGGC 1020

ACTGTGGTGC CACTTGGCGA GATGAGAGGC TACGCCCCCT TCAGCCCGGA CGAGAACTCC 1080

CTGGTTCTGT TTGAAGGGGA CGAGGTGTAT TCCACCATCC GGAAGCAGGA ATACAATGGG 1140

AAGATCCCTC GGTTCCGCCG CATCCGGGGC GAGAGTGAGC TGTACACCAG TGATACTGTC 1200

ATGCAGAACC CACAGTTCAT CAAAGCCACC ATCGTGCACC AAGACCAGGC TTACGATGAC 1260

AAGATCTACT ACTTCTTCCG AGAGGACAAT CCTGACAAGA ATCCTGAGGC TCCTCTCAAT 1320

GTGTCCCGTG TGGCCCAGTT GTGCAGGGGG GACCAGGGTG GGGAAAGTTC ACTGTCAGTC 1380

TCCAAGTGGA ACACTTTTCT GAAAGCCATG CTGGTATGCA GTGATGCTGC CACCAACAAG 1440

AACTTCAACA GGCTGCAAGA CGTCTTCCTG CTCCCTGACC CCAGCGGCCA GTGGAGGGAC 1500

ACCAGGGTCT ATGGTGTTTT CTCCAACCCC TGGAACTACT CAGCCGTCTG TGTGTATTCC 1560

CTCGGTGACA TTGACAAGGT CTTCCGTACC TCCTCACTCA AGGGCTACCA CTCAAGCCTT 1620

CCCAACCCGC GGCCTGGCAA GTGCCTCCCA GACCAGCAGC CGATACCCAC AGAGACCTTC 1680

CAGGTGGCTG ACCGTCACCC AGAGGTGGCG CAGAGGGTGG AGCCCATGGG GCCTCTGAAG 1740

ACGCCATTGT TCCACTCTAA ATACCACTAC CAGAAAGTGG CCGTTCACCG CATGCAAGCC 1800

AGCCACGGGG AGACCTTTCA TGTGCTTTAC CTAACTACAG ACAGGGGCAC TATCCACAAG 1860

GTGGTGGAAC CGGGGGAGCA GGAGCACAGC TTCGCCTTCA ACATCATGGA GATCCAGCCC 1920

TTCCGCCGCG CGGCTGCCAT CCAGACCATG TCGCTGGATG CTGAGCGGAG GAAGCTGTAT 1980

GTGAGCTCCC AGTGGGAGGT GAGCCAGGTG CCCCTGGACC TGTGTGAGGT CTATGGCGGG 2040

GGCTGCCACG GTTGCCTCAT GTCCCGAGAC CCCTACTGCG GCTGGGACCA GGGCCGCTGC 2100

ATCTCCATCT ACAGCTCCGA ACGGTCAGTG CTGCAATCCA TTAATCCAGC CGAGCCACAC 2160

AAGGAGTGTC CCAACCCCAA ACCAGACAAG GCCCCACTGC AGAAGGTTTC CCTGGCCCCA 2220

AACTCTCGCT ACTACCTGAG CTGCCCCATG GAATCCCGCC ACGCCACCTA CTCATGGCGC 2280

CACAAGGAGA ACGTGGAGCA GAGCTGCGAA CCTGGTCACC AGAGCCCCAA CTGCATCCTG 2340

TTCATCGAGA ACCTCACGGC GCAGCAGTAC GGCCACTACT TCTGCGAGGC CCAGGAGGGC 2400

TCCTACTTCC GCGAGGCTCA GCACTGGCAG CTGCTGCCCG AGGACGGCAT CATGGCCGAG 2460

CACCTGCTGG GTCATGCCTG TGCCCTGGCT GCCTCCCTCT GGCTGGGGGT GCTGCCCACA 2520

CTCACTCTTG GCTTGCTGGT CCACGTGAAG CTTGGGCCCG AACAAAAACT CATCTCAGAA 2580

GAGGATCTGA ATAGCGCCGT CGACCATCAT CATCATCATC ATTGAGTTTA TCCAGCACAG 2640

TGGCGGCCGC TCGAGTCTAG AGGGCCCGTT TAAACCCGCT GATCAGCCTC GACTGTGCCT 2700

TCTAGTTGCC AGCCATCTGT TGTTTGCCCC TCCCCCGTGC CTTCCTTGAC CCTGGAAGGT 2760

GCCACTCCCA CTGTCCTTTC CTAATAAAAT GAGGAAATTG CATCGCATTG TCTGAGTAGG 2820

TGTCATTCTA TTCTGGGGGG TGGGGTGGGG CAGGACAGCA AGGGGGAGGA TTGGGAAGAC 2880

AATAGCAGGC ATGCTGGGGA TGCGGTGGGC TCTATGGCTT CTGAGGCGGA AAGAACCAGC 2940

TGGGGCTCTA GGGGGTATCC CCACGCGCCC TGTAGCGGCG CATTAAGCGC GGCGGGTGTG 3000

GTGGTTACGC GCAGCGTGAC CGCTACACTT GCCAGCGCCC TAGCGCCCGC TCCTTTCGCT 3060

TTCTTCCCTT CCTTTCTCGC CACGTTCGCC GGCTTTCCCC GTCAAGCTCT AAATCGGGGC 3120

ATCCCTTTAG GGTTCCGATT TAGTGCTTTA CGGCACCTCG ACCCCAAAAA ACTTGATTAG 3180

GGTGATGGTT CACGTAGTGG GCCATCGCCC TGATAGACGG TTTTTCGCCC TTTGACGTTG 3240

GAGTCCACGT TCTTTAATAG TGGACTCTTG TTCCAAACTG GAACAACACT CAACCCTATC 3300

TCGGTCTATT CTTTTGATTT ATAAGGGATT TTGGGGATTT CGGCCTATTG GTTAAAAAAT 3360

GAGCTGATTT AACAAAAATT TAACGCGAAT TAATTCTGTG GAATGTGTGT CAGTTAGGGT 3420

GTGGAAAGTC CCCAGGCTCC CCAGGCAGGC AGAAGTATGC AAAGCATGCA TCTCAATTAG 3480

TCAGCAACCA GGTGTGGAAA GTCCCCAGGC TCCCCAGCAG GCAGAAGTAT GCAAAGCATG 3540

CATCTCAATT AGTCAGCAAC CATAGTCCCG CCCCTAACTC CGCCCATCCC GCCCCTAACT 3600

CCGCCCAGTT CCGCCCATTC TCCGCCCCAT GGCTGACTAA TTTTTTTTAT TTATGCAGAG 3660

GCCGAGGCCG CCTCTGCCTC TGAGCTATTC CAGAAGTAGT GAGGAGGCTT TTTTGGAGGC 3720

CTAGGCTTTT GCAAAAAGCT CCCGGGAGCT TGTATATCCA TTTTCGGATC TGATCAAGAG 3780

ACAGGATGAG GATCGTTTCG CATGATTGAA CAAGATGGAT TGCACGCAGG TTCTCCGGCC 3840

GCTTGGGTGG AGAGGCTATT CGGCTATGAC TGGGCACAAC AGACAATCGG CTGCTCTGAT 3900

GCCGCCGTGT TCCGGCTGTC AGCGCAGGGG CGCCCGGTTC TTTTTGTCAA GACCGACCTG 3960

TCCGGTGCCC TGAATGAACT GCAGGACGAG GCAGCGCGGC TATCGTGGCT GGCCACGACG 4020

GGCGTTCCTT GCGCAGCTGT GCTCGACGTT GTCACTGAAG CGGGAAGGGA CTGGCTGCTA 4080

TTGGGCGAAG TGCCGGGGCA GGATCTCCTG TCATCTCACC TTGCTCCTGC CGAGAAAGTA 4140

TCCATCATGG CTGATGCAAT GCGGCGGCTG CATACGCTTG ATCCGGCTAC CTGCCCATTC 4200

GACCACCAAG CGAAACATCG CATCGAGCGA GCACGTACTC GGATGGAAGC CGGTCTTGTC 4260

GATCAGGATG ATCTGGACGA AGAGCATCAG GGGCTCGCGC CAGCCGAACT GTTCGCCAGG 4320

CTCAAGGCGC GCATGCCCGA CGGCGAGGAT CTCGTCGTGA CCCATGGCGA TGCCTGCTTG 4380

CCGAATATCA TGGTGGAAAA TGGCCGCTTT TCTGGATTCA TCGACTGTGG CCGGCTGGGT 4440

GTGGCGGACC GCTATCAGGA CATAGCGTTG GCTACCCGTG ATATTGCTGA AGAGCTTGGC 4500

GGCGAATGGG CTGACCGCTT CCTCGTGCTT TACGGTATCG CCGCTCCCGA TTCGCAGCGC 4560

ATCGCCTTCT ATCGCCTTCT TGACGAGTTC TTCTGAGCGG GACTCTGGGG TTCGAAATGA 4620

CCGACCAAGC GACGCCCAAC CTGCCATCAC GAGATTTCGA TTCCACCGCC GCCTTCTATG 4680

AAAGGTTGGG CTTCGGAATC GTTTTCCGGG ACGCCGGCTG GATGATCCTC CAGCGCGGGG 4740

ATCTCATGCT GGAGTTCTTC GCCCACCCCA ACTTGTTTAT TGCAGCTTAT AATGGTTACA 4800

AATAAAGCAA TAGCATCACA AATTTCACAA ATAAAGCATT TTTTTCACTG CATTCTAGTT 4860

GTGGTTTGTC CAAACTCATC AATGTATCTT ATCATGTCTG TATACCGTCG ACCTCTAGCT 4920
AGAGCTTGGC GTAATCATGG TCATAGCTGT TTCCTGTGTG AAATTGTTAT CCGCTCACAA 4980
TTCCACACAA CATACGAGCC GGAAGCATAA AGTGTAAAGC CTGGGGTGCC TAATGAGTGA 5040
GCTAACTCAC ATTAATTGCG TTGCGCTCAC TGCCCGCTTT CCAGTCGGGA AACCTGTCGT 5100
GCCAGCTGCA TTAATGAATC GGCCAACGCG CGGGGAGAGG CGGTTTGCGT ATTGGGCGCT 5160
CTTCCGCTTC CTCGCTCACT GACTCGCTGC GCTCGGTCGT TCGGCTGCGG CGAGCGGTAT 5220
CAGCTCACTC AAAGGCGGTA ATACGGTTAT CCACAGAATC AGGGGATAAC GCAGGAAAGA 5280
ACATGTGAGC AAAAGGCCAG CAAAAGGCCA GGAACCGTAA AAAGGCCGCG TTGCTGGCGT 5340
TTTTCCATAG GCTCCGCCCC CCTGACGAGC ATCACAAAAA TCGACGCTCA AGTCAGAGGT 5400
GGCGAAACCC GACAGGACTA TAAAGATACC AGGCGTTTCC CCCTGGAAGC TCCCTCGTGC 5460
GCTCTCCTGT TCCGACCCTG CCGCTTACCG GATACCTGTC CGCCTTTCTC CCTTCGGGAA 5520
GCGTGGCGCT TTCTCAATGC TCACGCTGTA GGTATCTCAG TTCGGTGTAG GTCGTTCGCT 5580
CCAAGCTGGG CTGTGTGCAC GAACCCCCCG TTCAGCCCGA CCGCTGCGCC TTATCCGGTA 5640
ACTATCGTCT TGAGTCCAAC CCGGTAAGAC ACGACTTATC GCCACTGGCA GCAGCCACTG 5700
GTAACAGGAT TAGCAGAGCG AGGTATGTAG GCGGTGCTAC AGAGTTCTTG AAGTGGTGGC 5760
CTAACTACGG CTACACTAGA AGGACAGTAT TTGGTATCTG CGCTCTGCTG AAGCCAGTTA 5820
CCTTCGGAAA AAGAGTTGGT AGCTCTTGAT CCGGCAAACA AACCACCGCT GGTAGCGGTG 5880
GTTTTTTTGT TTGCAAGCAG CAGATTACGC GCAGAAAAAA AGGATCTCAA GAAGATCCTT 5940
TGATCTTTTC TACGGGGTCT GACGCTCAGT GGAACGAAAA CTCACGTTAA GGGATTTTGG 6000
TCATGAGATT ATCAAAAAGG ATCTTCACCT AGATCCTTTT AAATTAAAAA TGAAGTTTTA 6060
AATCAATCTA AAGTATATAT GAGTAAACTT GGTCTGACAG TTACCAATGC TTAATCAGTG 6120
AGGCACCTAT CTCAGCGATC TGTCTATTTC GTTCATCCAT AGTTGCCTGA CTCCCCGTCG 6180
TGTAGATAAC TACGATACGG GAGGGCTTAC CATCTGGCCC CAGTGCTGCA ATGATACCGC 6240
GAGACCCACG CTCACCGGCT CCAGATTTAT CAGCAATAAA CCAGCCAGCC GGAAGGGCCG 6300
AGCGCAGAAG TGGTCCTGCA ACTTTATCCG CCTCCATCCA GTCTATTAAT TGTTGCCGGG 6360
AAGCTAGAGT AAGTAGTTCG CCAGTTAATA GTTTGCGCAA CGTTGTTGCC ATTGCTACAG 6420
GCATCGTGGT GTCACGCTCG TCGTTTGGTA TGGCTTCATT CAGCTCCGGT TCCCAACGAT 6480
CAAGGCGAGT TACATGATCC CCCATGTTGT GCAAAAAAGC GGTTAGCTCC TTCGGTCCTC 6540

CGATCGTTGT CAGAAGTAAG TTGGCCGCAG TGTTATCACT CATGGTTATG GCAGCACTGC 6600

ATAATTCTCT TACTGTCATG CCATCCGTAA GATGCTTTTC TGTGACTGGT GAGTACTCAA 6660

CCAAGTCATT CTGAGAATAG TGTATGCGGC GACCGAGTTG CTCTTGCCCG GCGTCAATAC 6720

GGGATAATAC CGCGCCACAT AGCAGAACTT TAAAAGTGCT CATCATTGGA AAACGTTCTT 6780

CGGGGCGAAA ACTCTCAAGG ATCTTACCGC TGTTGAGATC CAGTTCGATG TAACCCACTC 6840

GTGCACCCAA CTGATCTTCA GCATCTTTTA CTTTCACCAG CGTTTCTGGG TGAGCAAAAA 6900

CAGGAAGGCA AAATGCCGCA AAAAAGGGAA TAAGGGCGAC ACGGAAATGT TGAATACTCA 6960

TACTCTTCCT TTTTCAATAT TATTGAAGCA TTTATCAGGG TTATTGTCTC ATGAGCGGAT 7020

ACATATTTGA ATGTATTTAG AAAAATAAAC AAATAGGGGT TCCGCGCACA TTTCCCCGAA 7080

AAGTGCCACC TGACGTCGAC GGATCGGG 7108

(2) INFORMATION ZU SEQ ID NO: 39:

(i) SEQUENZ CHARAKTERISTIKA:
(A) LÄNGE: 4019 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..4019

(xi) SEQUENZBESCHREIBUNG: SEQ ID NO: 39:

CTCGAGAAAT CATAAAAAAT TTATTTGCTT TGTGAGCGGA TAACAATTAT AATAGATTCA 60

ATTGTGAGCG GATAACAATT TCACACAGAA TTCATTAAAG AGGAGAAATT AACTATGAGA 120

GGATCGCATC ACCATCACCA TCACGGATCC CTGGTTCTGT TTGAAGGGGA CGAGGTGTAT 180

TCCACCATCC GGAAGCAGGA ATACAATGGG AAGATCCCTC GGTTCCGCCG CATCCGGGGC 240

GAGAGTGAGC TGTACACCAG TGATACTGTC ATGCAGAACC CACAGTTCAT CAAAGCCACC 300

ATCGTGCACC AAGACCAGGC TTACGATGAC AAGATCTACT ACTTCTTCCG AGAGGACAAT 360

CCTGACAAGA ATCCTGAGGC TCCTCTCAAT GTGTCCCGTG TGGCCCAGTT GTGCAGGGGG 420

GACCAGGGTG GGGAAAGTTC ACTGTCAGTC TCCAAGTGGA ACACTTTTCT GAAAGCCATG 480

CTGGTATGCA GTGATGCTGC CACCAACAAG AACTTCAACA GGCTGCAAGA CGTCTTCCTG 540
CTCCCTGACC CCAGCGGCCA GTGGAGGGAC ACCAGGGTCT ATGGTGTTTT CTCCAACCCC 600
TGGAACTACT CAGCCGTCTG TGTGTATTCC CTCGGTGACA TTGACAAGGT CTTCCGTACC 660
TCCTCACTCA AGGGCTACCA CTCAAGCCTT CCCAACCCGC GGCCTGGCAA GTGCCTCCCA 720
GACCAGCAGC CGATACCCAC AGAAAGCTTA ATTAGCTGAG CTTGGACTCC TGTTGATAGA 780
TCCAGTAATG ACCTCAGAAC TCCATCTGGA TTTGTTCAGA ACGCTCGGTT GCCGCCGGGC 840
GTTTTTTATT GGTGAGAATC CAAGCTAGCT TGGCGAGATT TTCAGGAGCT AAGGAAGCTA 900
AAATGGAGAA AAAAATCACT GGATATACCA CCGTTGATAT ATCCCAATGG CATCGTAAAG 960
AACATTTTGA GGCATTTCAG TCAGTTGCTC AATGTACCTA TAACCAGACC GTTCAGCTGG 1020
ATATTACGGC CTTTTTAAAG ACCGTAAAGA AAAATAAGCA CAAGTTTTAT CCGGCCTTTA 1080
TTCACATTCT TGCCCGCCTG ATGAATGCTC ATCCGGAATT TCGTATGGCA ATGAAAGACG 1140
GTGAGCTGGT GATATGGGAT AGTGTTCACC CTTGTTACAC CGTTTTCCAT GAGCAAACTG 1200
AAACGTTTTC ATCGCTCTGG AGTGAATACC ACGACGATTT CCGGCAGTTT CTACACATAT 1260
ATTCGCAAGA TGTGGCGTGT TACGGTGAAA ACCTGGCCTA TTTCCCTAAA GGGTTTATTG 1320
AGAATATGTT TTTCGTCTCA GCCAATCCCT GGGTGAGTTT CACCAGTTTT GATTTAAACG 1380
TGGCCAATAT GGACAACTTC TTCGCCCCCG TTTTCACCAT GGGCAAATAT TATACGCAAG 1440
GCGACAAGGT GCTGATGCCG CTGGCGATTC AGGTTCATCA TGCCGTCTGT GATGGCTTCC 1500
ATGTCGGCAG AATGCTTAAT GAATTACAAC AGTACTGCGA TGAGTGGCAG GGCGGGGCGT 1560
AATTTTTTTA AGGCAGTTAT TGGTGCCCTT AAACGCCTGG GGTAATGACT CTCTAGCTTG 1620
AGGCATCAAA TAAAACGAAA GGCTCAGTCG AAAGACTGGG CCTTTCGTTT TATCTGTTGT 1680
TTGTCGGTGA ACGCTCTCCT GAGTAGGACA AATCCGCCGC TCTAGAGCTG CCTCGCGCGT 1740
TTCGGTGATG ACGGTGAAAA CCTCTGACAC ATGCAGCTCC CGGAGACGGT CACAGCTTGT 1800
CTGTAAGCGG ATGCCGGGAG CAGACAAGCC CGTCAGGGCG CGTCAGCGGG TGTTGGCGGG 1860
TGTCGGGGCG CAGCCATGAC CCAGTCACGT AGCGATAGCG GAGTGTATAC TGGCTTAACT 1920
ATGCGGCATC AGAGCAGATT GTACTGAGAG TGCACCATAT GCGGTGTGAA ATACCGCACA 1980
GATGCGTAAG GAGAAAATAC CGCATCAGGC GCTCTTCCGC TTCCTCGCTC ACTGACTCGC 2040
TGCGCTCGGT CTGTCGGCTG CGGCGAGCGG TATCAGCTCA CTCAAAGGCG GTAATACGGT 2100

TATCCACAGA ATCAGGGGAT AACGCAGGAA AGAACATGTG AGCAAAAGGC CAGCAAAAGG 2160

CCAGGAACCG TAAAAAGGCC GCGTTGCTGG CGTTTTTCCA TAGGCTCCGC CCCCCTGACG 2220

AGCATCACAA AAATCGACGC TCAAGTCAGA GGTGGCGAAA CCCGACAGGA CTATAAAGAT 2280

ACCAGGCGTT TCCCCCTGGA AGCTCCCTCG TGCGCTCTCC TGTTCCGACC CTGCCGCTTA 2340

CCGGATACCT GTCCGCCTTT CTCCCTTCGG GAAGCGTGGC GCTTTCTCAA TGCTCACGCT 2400

GTAGGTATCT CAGTTCGGTG TAGGTCGTTC GCTCCAAGCT GGGCTGTGTG CACGAACCCC 2460

CCGTTCAGCC CGACCGCTGC GCCTTATCCG GTAACTATCG TCTTGAGTCC AACCCGGTAA 2520

GACACGACTT ATCGCCACTG GCAGCAGCCA CTGGTAACAG GATTAGCAGA GCGAGGTATG 2580

TAGGCGGTGC TACAGAGTTC TTGAAGTGGT GGCCTAACTA CGGCTACACT AGAAGGACAG 2640

TATTTGGTAT CTGCGCTCTG CTGAAGCCAG TTACCTTCGG AAAAAGAGTT GGTAGCTCTT 2700

GATCCGGCAA ACAAACCACC GCTGGTAGCG GTGGTTTTTT TGTTTGCAAG CAGCAGATTA 2760

CGCGCAGAAA AAAAGGATCT CAAGAAGATC CTTTGATCTT TTCTACGGGG TCTGACGCTC 2820

AGTGGAACGA AAACTCACGT TAAGGGATTT TGGTCATGAG ATTATCAAAA AGGATCTTCA 2880

CCTAGATCCT TTTAAATTAA AAATGAAGTT TTAAATCAAT CTAAAGTATA TATGAGTAAA 2940

CTTGGTCTGA CAGTTACCAA TGCTTAATCA GTGAGGCACC TATCTCAGCG ATCTGTCTAT 3000

TTCGTTCATC CATAGCTGCC TGACTCCCCG TCGTGTAGAT AACTACGATA CGGGAGGGCT 3060

TACCATCTGG CCCCAGTGCT GCAATGATAC CGCGAGACCC ACGCTCACCG GCTCCAGATT 3120

TATCAGCAAT AAACCAGCCA GCCGGAAGGG CCGAGCGCAG AAGTGGTCCT GCAACTTTAT 3180

CCGCCTCCAT CCAGTCTATT AATTGTTGCC GGGAAGCTAG AGTAAGTAGT TCGCCAGTTA 3240

ATAGTTTGCG CAACGTTGTT GCCATTGCTA CAGGCATCGT GGTGTCACGC TCGTCGTTTG 3300

GTATGGCTTC ATTCAGCTCC GGTTCCCAAC GATCAAGGCG AGTTACATGA TCCCCCATGT 3360

TGTGCAAAAA AGCGGTTAGC TCCTTCGGTC CTCCGATCGT TGTCAGAAGT AAGTTGGCCG 3420

CAGTGTTATC ACTCATGGTT ATGGCAGCAC TGCATAATTC TCTTACTGTC ATGCCATCCG 3480

TAAGATGCTT TTCTGTGACT GGTGAGTACT CAACCAAGTC ATTCTGAGAA TAGTGTATGC 3540

GGCGACCGAG TTGCTCTTGC CCGGCGTCAA TACGGGATAA TACCGCGCCA CATAGCAGAA 3600

CTTTAAAAGT GCTCATCATT GGAAAACGTT CTTCGGGGCG AAAACTCTCA AGGATCTTAC 3660

CGCTGTTGAG ATCCAGTTCG ATGTAACCCA CTCGTGCACC CAACTGATCT TCAGCATCTT 3720

TTACTTTCAC CAGCGTTTCT GGGTGAGCAA AAACAGGAAG GCAAAATGCC GCAAAAAAGG 3780

GAATAAGGGC GACACGGAAA TGTTGAATAC TCATACTCTT CCTTTTTCAA TATTATTGAA 3840

GCATTTATCA GGGTTATTGT CTCATGAGCG GATACATATT TGAATGTATT TAGAAAAATA 3900

AACAAATAGG GGTTCCGCGC ACATTTCCCC GAAAAGTGCC ACCTGACGTC TAAGAAACCA 3960

TTATTATCAT GACATTAACC TATAAAAATA GGCGTATCAC GAGGCCCTTT CGTCTTCAC 4019

(2) INFORMATION ZU SEQ ID NO: 40:

(i) SEQUENZ CHARAKTERISTIKA:
(A) LÄNGE: 3999 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..3999

(xi) SEQUENZBESCHREIBUNG: SEQ ID NO: 40:

CTCGAGAAAT CATAAAAAAT TTATTTGCTT TGTGAGCGGA TAACAATTAT AATAGATTCA 60

ATTGTGAGCG GATAACAATT TCACACAGAA TTCATTAAAG AGGAGAAATT AACTATGAGA 120

GGATCGCATC ACCATCACCA TCACACGGAT CCGCATGCGA GCTCCCAGTG GGAGGTGAGC 180

CAGGTGCCCC TGGACCTGTG TGAGGTCTAT GGCGGGGGCT GCCACGGTTG CCTCATGTCC 240

CGAGACCCCT ACTGCGGCTG GGACCAGGGC CGCTGCATCT CCATCTACAG CTCCGAACGG 300

TCAGTGCTGC AATCCATTAA TCCAGCCGAG CCACACAAGG AGTGTCCCAA CCCCAAACCA 360

GACAAGGCCC CACTGCAGAA GGTTTCCCTG GCCCCAAACT CTCGCTACTA CCTGAGCTGC 420

CCCATGGAAT CCCGCCACGC CACCTACTCA TGGCGCCACA AGGAGAACGT GGAGCAGAGC 480

TGCGAACCTG GTCACCAGAG CCCCAACTGC ATCCTGTTCA TCGAGAACCT CACGGCGCAG 540

CAGTACGGCC ACTACTTCTG CGAGGCCCAG GAGGGCTCCT ACTTCCGCGA GGCTCAGCAC 600

TGGCAGCTGC TGCCCGAGGA CGGCATCATG GCCGAGCACC TGCTGGGTCA TGCCTGTGCC 660

CTGGCTGCCT CCCTCTGGCT GGGGGTGCTG CCCACACTCA CTCTTGGCTT GCTGGTCCAC 720

GTGAAGCTTA ATTAGCTGAG CTTGGACTCC TGTTGATAGA TCCAGTAATG ACCTCAGAAC 780

TCCATCTGGA TTTGTTCAGA ACGCTCGGTT GCCGCCGGGC GTTTTTTATT GGTGAGAATC 840

CAAGCTAGCT TGGCGAGATT TTCAGGAGCT AAGGAAGCTA AAATGGAGAA AAAAATCACT 900

GGATATACCA CCGTTGATAT ATCCCAATGG CATCGTAAAG AACATTTTGA GGCATTTCAG 960

TCAGTTGCTC AATGTACCTA TAACCAGACC GTTCAGCTGG ATATTACGGC CTTTTTAAAG 1020

ACCGTAAAGA AAAATAAGCA CAAGTTTTAT CCGGCCTTTA TTCACATTCT TGCCCGCCTG 1080

ATGAATGCTC ATCCGGAATT TCGTATGGCA ATGAAAGACG GTGAGCTGGT GATATGGGAT 1140

AGTGTTCACC CTTGTTACAC CGTTTTCCAT GAGCAAACTG AAACGTTTTC ATCGCTCTGG 1200

AGTGAATACC ACGACGATTT CCGGCAGTTT CTACACATAT ATTCGCAAGA TGTGGCGTGT 1260

TACGGTGAAA ACCTGGCCTA TTTCCCTAAA GGGTTTATTG AGAATATGTT TTTCGTCTCA 1320

GCCAATCCCT GGGTGAGTTT CACCAGTTTT GATTTAAACG TGGCCAATAT GGACAACTTC 1380

TTCGCCCCCG TTTTCACCAT GGGCAAATAT TATACGCAAG GCGACAAGGT GCTGATGCCG 1440

CTGGCGATTC AGGTTCATCA TGCCGTCTGT GATGGCTTCC ATGTCGGCAG AATGCTTAAT 1500

GAATTACAAC AGTACTGCGA TGAGTGGCAG GGCGGGGCGT AATTTTTTTA AGGCAGTTAT 1560

TGGTGCCCTT AAACGCCTGG GGTAATGACT CTCTAGCTTG AGGCATCAAA TAAAACGAAA 1620

GGCTCAGTCG AAAGACTGGG CCTTTCGTTT TATCTGTTGT TTGTCGGTGA ACGCTCTCCT 1680

GAGTAGGACA AATCCGCCGC TCTAGAGCTG CCTCGCGCGT TTCGGTGATG ACGGTGAAAA 1740

CCTCTGACAC ATGCAGCTCC CGGAGACGGT CACAGCTTGT CTGTAAGCGG ATGCCGGGAG 1800

CAGACAAGCC CGTCAGGGCG CGTCAGCGGG TGTTGGCGGG TGTCGGGGCG CAGCCATGAC 1860

CCAGTCACGT AGCGATAGCG GAGTGTATAC TGGCTTAACT ATGCGGCATC AGAGCAGATT 1920

GTACTGAGAG TGCACCATAT GCGGTGTGAA ATACCGCACA GATGCGTAAG GAGAAAATAC 1980

CGCATCAGGC GCTCTTCCGC TTCCTCGCTC ACTGACTCGC TGCGCTCGGT CTGTCGGCTG 2040

CGGCGAGCGG TATCAGCTCA CTCAAAGGCG GTAATACGGT TATCCACAGA ATCAGGGGAT 2100

AACGCAGGAA AGAACATGTG AGCAAAAGGC CAGCAAAAGG CCAGGAACCG TAAAAAGGCC 2160

GCGTTGCTGG CGTTTTTCCA TAGGCTCCGC CCCCCTGACG AGCATCACAA AAATCGACGC 2220

TCAAGTCAGA GGTGGCGAAA CCCGACAGGA CTATAAAGAT ACCAGGCGTT TCCCCCTGGA 2280

AGCTCCCTCG TGCGCTCTCC TGTTCCGACC CTGCCGCTTA CCGGATACCT GTCCGCCTTT 2340

CTCCCTTCGG GAAGCGTGGC GCTTTCTCAA TGCTCACGCT GTAGGTATCT CAGTTCGGTG 2400

TAGGTCGTTC GCTCCAAGCT GGGCTGTGTG CACGAACCCC CCGTTCAGCC CGACCGCTGC 2460

GCCTTATCCG GTAACTATCG TCTTGAGTCC AACCCGGTAA GACACGACTT ATCGCCACTG 2520

GCAGCAGCCA CTGGTAACAG GATTAGCAGA GCGAGGTATG TAGGCGGTGC TACAGAGTTC 2580

TTGAAGTGGT GGCCTAACTA CGGCTACACT AGAAGGACAG TATTTGGTAT CTGCGCTCTG 2640

CTGAAGCCAG TTACCTTCGG AAAAAGAGTT GGTAGCTCTT GATCCGGCAA ACAAACCACC 2700

GCTGGTAGCG GTGGTTTTTT TGTTTGCAAG CAGCAGATTA CGCGCAGAAA AAAAGGATCT 2760

CAAGAAGATC CTTTGATCTT TTCTACGGGG TCTGACGCTC AGTGGAACGA AAACTCACGT 2820

TAAGGGATTT TGGTCATGAG ATTATCAAAA AGGATCTTCA CCTAGATCCT TTTAAATTAA 2880

AAATGAAGTT TTAAATCAAT CTAAAGTATA TATGAGTAAA CTTGGTCTGA CAGTTACCAA 2940

TGCTTAATCA GTGAGGCACC TATCTCAGCG ATCTGTCTAT TTCGTTCATC CATAGCTGCC 3000

TGACTCCCCG TCGTGTAGAT AACTACGATA CGGGAGGGCT TACCATCTGG CCCCAGTGCT 3060

GCAATGATAC CGCGAGACCC ACGCTCACCG GCTCCAGATT TATCAGCAAT AAACCAGCCA 3120

GCCGGAAGGG CCGAGCGCAG AAGTGGTCCT GCAACTTTAT CCGCCTCCAT CCAGTCTATT 3180

AATTGTTGCC GGGAAGCTAG AGTAAGTAGT TCGCCAGTTA ATAGTTTGCG CAACGTTGTT 3240

GCCATTGCTA CAGGCATCGT GGTGTCACGC TCGTCGTTTG GTATGGCTTC ATTCAGCTCC 3300

GGTTCCCAAC GATCAAGGCG AGTTACATGA TCCCCCATGT TGTGCAAAAA AGCGGTTAGC 3360

TCCTTCGGTC CTCCGATCGT TGTCAGAAGT AAGTTGGCCG CAGTGTTATC ACTCATGGTT 3420

ATGGCAGCAC TGCATAATTC TCTTACTGTC ATGCCATCCG TAAGATGCTT TTCTGTGACT 3480

GGTGAGTACT CAACCAAGTC ATTCTGAGAA TAGTGTATGC GGCGACCGAG TTGCTCTTGC 3540

CCGGCGTCAA TACGGGATAA TACCGCGCCA CATAGCAGAA CTTTAAAAGT GCTCATCATT 3600

GGAAAACGTT CTTCGGGGCG AAAACTCTCA AGGATCTTAC CGCTGTTGAG ATCCAGTTCG 3660

ATGTAACCCA CTCGTGCACC CAACTGATCT TCAGCATCTT TTACTTTCAC CAGCGTTTCT 3720

GGGTGAGCAA AAACAGGAAG GCAAAATGCC GCAAAAAAGG GAATAAGGGC GACACGGAAA 3780

TGTTGAATAC TCATACTCTT CCTTTTTCAA TATTATTGAA GCATTTATCA GGGTTATTGT 3840

CTCATGAGCG GATACATATT TGAATGTATT TAGAAAAATA AACAAATAGG GGTTCCGCGC 3900

ACATTTCCCC GAAAAGTGCC ACCTGACGTC TAAGAAACCA TTATTATCAT GACATTAACC 3960

TATAAAAATA GGCGTATCAC GAGGCCCTTT CGTCTTCAC 3999

(2) ANGABEN ZU SEQ ID NO: 41:

(i) SEQUENZKENNZEICHEN:
(A) LÄNGE: 8888 Basenpaare
(B) ART: Nucleotid
(C) STRANGFORM: Einzelstrang
(D) TOPOLOGIE: linear

(ii) ART DES MOLEKÜLS: Genom-DNA

(ix) MERKMAL:
(A) NAME/SCHLÜSSEL: exon
(B) LAGE:1..8888

(xi) SEQUENZBESCHREIBUNG: SEQ ID NO: 41:

GAGCCGCACA CGGTGCTTTT CCACGAGCCA GGCAGCTCCT CTGTGTGGGT GGGAGGACGT 60

GGCAAGGTCT ACCTCTTTGA CTTCCCCGAG GGCAAGAACG CATCTGTGCG CACGGTGAGC 120

CTCTCTCTTC CCCCAACACC CCCCCTACCC TCTTATCTCC CCTCTGGCCC TGCCAAGGGT 180

CCTCAGGGAA TCCGAGGGAG CTGGCTTCTC TTCCTAAACT GCCCCCACCT CCGTATCCTA 240

TAAATGGCTC CTGGGGGAGG CTCCCTAAAG GTAGTCCAGA TTGGAGTGGG GAGCTGGGGC 300

GGTGTGGAGA AAAACAGGAG CTAATGGGCC TGGCCAGCTG GGCAGCGCTG CTGCGGAAAG 360

CCCAGGCTGG AAGCTGGGCC CCAGAGCCCA TGCCTGGTCT TCTGAACCCT CTGGGCCTCA 420

GCTCTGGATA TGAGACCCTG TTTGACCTCA GGTAGATCAC TCACCCTCTC AGAGCCCCAG 480

TTGCTCATCT GTCAGATGAG AATAATGGTT GCTTCCTTTG GGGCTTATCC TGAGGCTGTG 540

TGGAAAGCAT TTCAGGGGTA CCTCACCCCT GGCAGATTGA ACTAATGCTT CTCCCCTTCC 600

CCAGGTGAAT ATCGGCTCCA CAAAGGGGTC CTGTCTGGAT AAGCGGGTGA GCGGGGGAGG 660

GATCTGGAGG GGTCTGAGCC ACTTGGTAAA GGGAGAGGAG ACCCTGAGGG TCTAAGGAAG 720

GAAGCATGGC CCTGCCCCAC GAGTCCCAGA CTGATGGGGA GACGTGGTCC TCTGTGCTTA 780

GGGGATGGCG TCAGCTGCAC ACACTCTGGG CTGTCCCGGG AGGCTGTCAC CTATGCTAAG 840

CCCTTCTGAC ACCTTCTTCC CTGATCCTGG GGGTCCTAGT GCTAGGCTTG CCAGGGCCTT 900

CCAGCAACCA ATTTCTCTCC TCCCTTCTCT CTTCCCCGGG CAGGACTGCG AGAACTACAT 960

CACTCTCCTG GAGAGGCGGA GTGAGGGGCT GCTGGCCTGT GGCACCAACG CCCGGCACCC 1020

CAGCTGCTGG AACCTGGTGA GAAGGCTGCT CCCCATGTGC CTGATCAGCT CACCTTCTAC 1080

TGCGTGGGCT TCTGCCCCTC ATGGTGGGAA GGAGATGGCG AGACTCCAAT GCTGGCCTTG 1140

CCCTGGGAGG ATGGGGCTCC TGGCCGAGAA ACTGGCCGTC ATGGGAGGCA GTGGCTGTGG 1200
GATTATGTGG CCATCCAACC CTCTGGATCT CCCACAGGTG AATGGCACTG TGGTGCCACT 1260
TGGCGAGATG AGAGGCTACG CCCCCTTCAG CCCGGACGAG AACTCCCTGG TTCTGTTTGA 1320
AGGTTGGGGC ATGCTTCGGA ACTGGGCTGG GAGCAGGATG GTCAGCTCTT TGTCCAGTGT 1380
CCGGAGGAGG GACTTCCAGG AGCTGCCTGC CCTTACTCAT TTCTCCCTCC CACTGACCCC 1440
AGGGGACGAG GTGTATTCCA CCATCCGGAA GCAGGAATAC AATGGGAAGA TCCCTCGGTT 1500
CCGCCGCATC CGGGGCGAGA GTGAGCTGTA CACCAGTGAT ACTGTCATGC AGAGTGAGTC 1560
AGGCTCCGGC TGGGCTGAGG GTGGGCAAGG GGGTGTGAGC ACTTAAGGTG GCAGATGGGA 1620
TCCTGATGTT TCTGGGAGGG CTCCCTGAGG GCCGCTGGGG CCATGCAGGA AAGCAGGACC 1680
TTGGTATAGG CCTGAGAAGT TAGGGTTGGC TGGGAGCAGA GGAACAGACA AGGTATAGCA 1740
GTGGGATGGG CCCAGCCCTC TTCAGGAACA CAAACAGAGG GAGCCCCAGA CCCAGTGCAG 1800
GGTCCCCAGG AGCCAAAGTT TATCCTCTGC TGAGTTCACG TGGAGGCAGC CCCCCAACTC 1860
CCTCCTCATC AGGGCTCTGC CAATTGAGCA GAAGTGACAT AGGGGCCCCC AGGGACCTTC 1920
CCCCACTCCC CAGGCATGAA GTCATTGCTC CTGGGCCGAT GACATCTTTG TAGGAAGAGG 1980
GCAAAACAGG TGTGGGGTGG AGGTGCAGGG TCTAGGGCCC CTCGGGGAGT TGGACCTGAT 2040
GTTATGAGTC CTATTCCAGA TCTGATTTGC CATGGTTTGT GCAGACCCGA AGGAGGGAGG 2100
AGAGTGTGCA GGGTTGGAAT GGTCTCCCGG GCAAGCTTCC CAGCCTTACG CCCATTCGCT 2160
TCTGTGCCCT GGCAGACCCA CAGTTCATCA AAGCCACCAT CGTGCACCAA GACCAGGCTT 2220
ACGATGACAA GATCTACTAC TTCTTCCGAG AGGACAATCC TGACAAGAAT CCTGAGGCTC 2280
CTCTCAATGT GTCCCGTGTG GCCCAGTTGT GCAGGGTGAA CACGGGCGTG AGGGCTGCTG 2340
GCTACGTGTC TGTGCATGAA TAGGCCTGAG TGAGGGTGAG TTCTGTGTGT CCGTGTGCAT 2400
GTAGAAGTTG TGTGGATGTA TGAGTGGGTC TGTGTCAGGG ACTGTGGGAG CAGCTGTGTG 2460
TGCATGGAGC ATCATGTGTC TGTGTGTGGG TAAAGGTGGC TGAGCTCCTG TGCACGTATG 2520
ATGGCGTGTG AGCGTGTGTA TGATGGGGTG TGTGTGTGTG TGTGTGTGTG TGTTTTGCCT 2580
GTGTGAATGT GCTGTGCCAC GTATGTGGGT GCGTGAGTCA GTAAATGTGT GTCTGAGTCC 2640
GTCTGCTCTG TGGGGACCTG GCACTCTCAC CTGCCCTGAC CCTGGGCACT GCTGGCCCTG 2700
GGCTCTGGAT CAGCCAGGCC TGCTTGCAGG AGTCTCATCT GGAGACCTGC CCTGAGTCCT 2760
GGGGCACCCC CGGCAGGTCC TGGCCCCTCG CAGCCTGCCT TCCTCCTCTG GGCCCAGGTG 2820

TTGATATTGC TGGCAGTGGT TTCCTGGGGT GTGTGGGGAA GCCCGGGCAG GTGCTGAGGG 2880

GCCTCTTCTC CCCTCTACCC TTCCAGGGGG ACCAGGGTGG GGAAAGTTCA CTGTCAGTCT 2940

CCAAGTGGAA CACTTTTCTG AAAGCCATGC TGGTATGCAG TGATGCTGCC ACCAACAAGA 3000

ACTTCAACAG GCTGCAAGAC GTCTTCCTGC TCCCTGACCC CAGCGGCCAG TGGAGGGACA 3060

CCAGGGTCTA TGGTGTTTTC TCCAACCCCT GGTGAGTGGC CCTTGTCCTG GGGCCGGGGC 3120

TGGCATTGGT TCAGTGTCCA GTAGGGACAG GAGGCCTTGG GCCCTGCTGA GGGCCTCCCT 3180

GGTGTGGCAG GAGCAGGGGC TGCAGGCTCA AGAGGCTGGG CTGTTGCTGG GTGTGGGGTG 3240

GGGGGACAGC CAGTGCGATG TATGTACTGT TGTGTGAGTG AGTCTGCACT CATGGGTGTG 3300

TGTGCATGCC CTATATGCAC ACTCATGACT GCACTTGTGC CTGTGTGTCC CACCACCTGC 3360

TTGTGCCGAG AGTGGACACT GGGCCCAGGA GGAAGCTGCT GAAGCATCTC TCGGGGAGCT 3420

GGGTGCTATT ACACCTGCTC AGGCACTGCC TGAGCCCGAT AATTCACACT TCTTAATCAC 3480

TCTCATTGAT TGAACACACG GCAGGCGGAA GTGTTGGGTG TGTGTGGGGA GAGTTAGGGA 3540

TAGAGTGGAG GAAGCCAAGA CCCTGCTCTG TGGCTCCTGG GTGAGTGGGT CCCCCAGGCT 3600

GGGAAGGGGT TGGGGGTCTG GCCTCCTGGG GCATCAGCAC CCCACAGCCT GTGCCCAGGG 3660

AGGGCTAGAG AACTGCTCAG CCTATGATGG GGTTCCTCCT GCCTTGGGGT TGGGTAGAGC 3720

AGATGGCCTC TAGACTCAGT GATTCTGTAA CAGGATACAA GTTTGTGGTT TTAAATTGCA 3780

GCACAAAGAA ATTAGGCTGA ACTCCTCTCC TTCCTCCTCT CCATCCCTCC CCATTTTCAG 3840

TGGTGGTTGG CAACTCAGTG CCAGGCACAA GGCTGGCCTG GGTGAGTGGA GGTGGATGGG 3900

TGGGTTCTGG GCCCCCCATT GAGCTGGTCT CCATGTCACT GCAGGAACTA CTCAGCCGTC 3960

TGTGTGTATT CCCTCGGTGA CATTGACAAG GTCTTCCGTA CCTCCTCACT CAAGGGCTAC 4020

CACTCAAGCC TTCCCAACCC GCGGCCTGGC AAGGTGAGCG TGACACCAGC CGTGGCCCAG 4080

GCCCAGCCCT CCTTCTGCCT CACCTCCCAC CACCCCACTG ACCTGGGCCT GCTCTCCTTG 4140

CCCAGTGCCT CCCAGACCAG CAGCCGATAC CCACAGAGAC CTTCCAGGTG GCTGACCGTC 4200

ACCCAGAGGT GGCGCAGAGG GTGGAGCCCA TGGGGCCTCT GAAGACGCCA TTGTTCCACT 4260

CTAAATACCA CTACCAGAAA GTGGCCGTCC ACCGCATGCA AGCCAGCCAC GGGGAGACCT 4320

TTCATGTGCT TTACCTAACT ACAGGTGAGA GGCTACCCCG GGACCCTCAG TTTGCTTTGT 4380

AAAAACGGGC ATGAAAGGTG TAAGGAATAA TGTAGTTAAC ATCTGGTTGG ATCTTTACAT 4440

GTGGAAGGAA TAATTGAGTG ACTGGAGTTG TCAGGGGTTA ATGTGTGTGG GTGTGGAAGA 4500

GCCAGGCAGG GAGAGCTTCC TGGAGGAGGT AGGGGCAAGA GGGAAAGGGG GATGGGAGAA 4560

AAGCAAGCAC TGGGATTTGG AGGCGGAAAT CTGGAGAGTC TGAGCAAAGC CAGGTGCACC 4620

TTTGGTCCAG ATGTCTGACT CAGGGAAGAA GATGGTAGGA AGAGACGTGG CAAATGAGGA 4680

GGAGGGGCCT GAACCACAGG GATACTGGCC TCTGCCAGGC AGAATGAGGG AGTCAGGCCC 4740

TGCGCCTGTC TTTGGGATTG TGCAGGTGAG AAGAAACATT TGAGGAGTTG ATGGGGCACA 4800

AATTAGGTAT GGGGAAGGAG TTCCAGGGGG CAGAACCTTT GCCATCTCAC AGAGGACAGG 4860

GGCAGCTTCT CTTCTTCCCT GGAGTAGGCC CTGCTGGGGG AAGCTGGGTG GAATGCCGTG 4920

GGAGATGCTC CTGCTTTCTG GAAAGCCACA GGACACGGAG GAGCCAGTCC TGAGTTGGGT 4980

TTGTCGCAGC TTCCCATGCC AGCTGCCTTC CTTGAGACTG GAAAGGGCCT CTAGCACCCC 5040

TGGGGCCATT CAATTCAGGC CCAGGCGCCC AACCTCAGTT GTTCACATTC CCCATGTGAT 5100

CTCCTGTTGC TGCTTCACCT TGGGACTGTC TCGGCTTTGG TGACCTTGTA GGAAACTGGA 5160

ACCCCAGCAC CATTGTTTGG CTCCTGGAAG CCTTGGGGAG AGGAATTTCC CACAGGGCAG 5220

GGCCTGGGTC CTGATTCCCT GCCTCTTTAC TCCCTATTCA TCCCGGCTAC ACCCTTGGGC 5280

CCCCATCCTT GCTTGGCTCC AGTACTGGCT GGCACAGCTG TTGTGGTCAT CCAGGGATGG 5340

CAGGGCACTG GGGAACAGAA GAGAGAGGTC ACACAGTGCG GAACTGGGAG CAGGAGCTAG 5400

GACAAGGAAG GCTGGACTTG GGCCATGGAT TCCCTTCCTG CAGACTTGGG AAGTGAGCAC 5460

ACTTGAGTGA TTAGAGAAGG TGTCTTCGTT CTAAGGGCAG TGGAGGAGGC ACCATTTTGG 5520

AGCCTGCATC ATTCGTATTT GGGCTAGATT GAAAAATAGA GCTTTCTAAG TCCTCTGCAG 5580

AGAATGGGAG GCTCTCACAA CTGGGAGAAG TATTGGCTCT TTTCCTGAGA ATTTTGCCAA 5640

GGGTATGCTG TTACTGGGGC TGGTTTGGAA GGAGTATAGG GCATTATGTC TGTGAAGGCA 5700

GTGGCTGGGG TGGGGCCTTA TCAGGCCCAA GGAGCATCTG GCCACATCTC AGAGTCCACA 5760

GATGAGGATC ACGGATGTGT AGAGGAAACA TCCTAGGCAG GCAATCATCT GACTGCTTTT 5820

TTGGGGCAGG TGATGCCCTG GGAAATTGGG AGGGAGGGAG AGAGGGAGGT AGGCTATTCT 5880

AGAAACTGGG AGAGCAGGTG AGGTAGGATT GGGAGGACCA GGGGTCAGGG TCCCCATTGG 5940

TCCCTAATTG AGAACGGAGA GAGCATTGGT CTAGGAGGCA GGCAGCTCGG TTATAAGACC 6000

TTGGGAACTC TTGATTTAGA ATCCAAGATC CTTTTTAGAT CTAGGATTTT ATAAAATTAA 6060

GATATCCCCT AAGATCAAAT GCAACGTGGA GTCCTGAATT GGATCCTAGA ACAGAAGAAG 6120

GACATTTGTG GAAAAACTAG TGAAATCCAA ATAAAGTCTG TAGTTTTGTT AATAGTAATG 6180

CACCAATGTC AGTTGCCTAG TTGTGACAAA TATACCGTGG TTATGTAAGA TGGTAACATT 6240

AGGGGGAACT GGAGAAGGGT AGATTGGAGC TCTCTGTACT ATCTTTGCAA CTTTTCTGGG 6300

AATCTAAAAT TACTCCAAAA TAAAAAAAAA ATGTATTTAA AGTAAATATA TTCCCTAAGA 6360

GTCCAGGAGG CAGGGGAGTT GTAGAAGCAG CTGAGTGGTT GGGTTCTGAC AGATTTGGTT 6420

CCAACTCGGT CTCTGCTGCT CACCAGCTGT GTGACCTTGA GCAAGTGGCT TAGCCTTTCT 6480

GAGCCTGATT TCCTTATCTG TGGAGTGGGG AAGATGACAG CCACCTCGCA GGGCTGTGGA 6540

GGGTTAAACG AGGTGATGCA TGGACAGCAG CCGCACTGAC CTTGCTGGTG TGGGGCTCCT 6600

GCTTCTGTTC TTCCCGTGCA GCCTTGGGAA TGTTGGAGGC CGTATCCAGG GACCCCTGGG 6660

CCTCCTGGGA TGGCCTCTCT GGATCAGCCT TGGAAGGTTC CAGGCTGCCC TTAGGCTCCC 6720

ACATTCTTCC CCAGTCACGC TCTCCTCGCC CTGCCCACAC CAGTCCTGTG ACCCTTGCCT 6780

GAGTTGTGAC TTCCCACCCC TCCCCGGCCT AGAGGAAAGC TGCCTGGCCC CTCAGTGGGA 6840

CTCCCGCCCA CTGACCCTCT GTCCACCATA CACAGACAGG GGCACTATCC ACAAGGTGGT 6900

GGAACCGGGG GAGCAGGAGC ACAGCTTCGC CTTCAACATC ATGGAGATCC AGCCCTTCCG 6960

CCGCGCGGCT GCCATCCAGA CCATGTCGCT GGATGCTGAG CGGGTGAGCC TTCCCCCACT 7020

GCGTCCCATG GGCTATGCAG TGACTGCAGC TGAGGACAGG GCTCCTTTGC ATGTGATTTG 7080

TGTGTTCTTT TAAGAGCTTC TAGGCCTTAG GGCCTGGACA TTTAGGACTG AGTGTGGGGT 7140

GGGGCCCGGG CCTGACCCAA TCCTGCTGTC CTTCCAGAGG AAGCTGTATG TGAGCTCCCA 7200

GTGGGAGGTG AGCCAGGTGC CCCTGGACCT GTGTGAGGTC TATGGCGGGG GCTGCCACGG 7260

TTGCCTCATG TCCCGAGACC CCTACTGCGG CTGGGACCAG GGCCGCTGCA TCTCCATCTA 7320

CAGCTCCGAA CGGTACGTTG GCCGGGATCC CTCCGTCCCT GGGACAAGGT GGGCATGGGA 7380

CAGGGGGAGG TGTTGTCGGG CTGGAAGAGG TGGCGGTACT GGGCCTTTCT TGTGGGACCT 7440

CCTCTCTACT GGAACTGCAC TAGGGGTAAG GATATGAGGG TCAGGTCTGC AGCCTTGTAT 7500

CTGCTGATCC TCTTTCGTCC TTCCCACTCC AGGTCAGTGC TGCAATCCAT TAATCCAGCC 7560

GAGCCACACA AGGAGTGTCC CAACCCCAAA CCAGGTACCT GATCTGGCCC TGCTGGCGGC 7620

TGTGGCCCAA TGAGTGGGGT ACTGCCCTGC CCTGATTGTC CTGGTCTGAG GGAAACATGG 7680

CCTTGTCCTG TGGGCCCCAG GTACATGGGG CAGGATACAG TCCTGCAGAG GGAGCCCTCT 7740

TGGTGGGATG AGCGAGACGG GAGAAAAAAG GAGGACGCTG AGGGCTGGGT TCCCCACGTT 7800

CATTCAGAAG CCTTGTCCTG GGATCCCAGT CGGTGGGGAG GACACATCCT CCCCTGGGAG 7860

CTCTTTGTCC CTCCTCACGG CTGCTTCCCC ACTGCCTCCC CAGACAAGGC CCCACTGCAG 7920

AAGGTTTCCC TGGCCCCAAA CTCTCGCTAC TACCTGAGCT GCCCCATGGA ATCCCGCCAC 7980

GCCACCTACT CATGGCGCCA CAAGGAGAAC GTGGAGCAGA GCTGCGAACC TGGTCACCAG 8040

AGCCCCAACT GCATCCTGTT CATCGAGAAC CTCACGGCGC AGCAGTACGG CCACTACTTC 8100

TGCGAGGCCC AGGAGGGCTC CTACTTCCGC GAGGCTCAGC ACTGGCAGCT GCTGCCCGAG 8160

GACGGCATCA TGGCCGAGCA CCTGCTGGGT CATGCCTGTG CCCTGGCCGC CTCCCTCTGG 8220

CTGGGGGTGC TGCCCACACT CACTCTTGGC TTGCTGGTCC ACTAGGGCCT CCCGAGGCTG 8280

GGCATGCCTC AGGCTTCTGC AGCCCAGGGC ACTAGAACGT CTCACACTCA GAGCCGGCTG 8340

GCCCGGGAGC TCCTTGCCTG CCACTTCTTC CAGGGGACAG AATAACCCAG TGGAGGATGC 8400

CAGGCCTGGA GACGTCCAGC CGCAGGCGGC TGCTGGGCCC CAGGTGGCGC ACGGATGGTG 8460

AGGGGCTGAG AATGAGGGCA CCGACTGTGA AGCTGGGGCA TCGATGACCC AAGACTTTAT 8520

CTTCTGGAAA ATATTTTTCA GACTCCTCAA ACTTGACTAA ATGCAGCGAT GCTCCCAGCC 8580

CAAGAGCCCA TGGGTCGGGG AGTGGGTTTG GATAGGAGAG CTGGGACTCC ATCTCGACCC 8640

TGGGGCTGAG GCCTGAGTCC TTCTGGACTC TTGGTACCCA CATTGCCTCC TTCCCCTCCC 8700

TCTCTCATGG CTGGGTGGCT GGTGTTCCTG AAGACCCAGG GCTACCCTCT GTCCAGCCCT 8760

GTCCTCTGCA GCTCCCTCTC TGGTCCTGGG TCCCACAGGA CAGCCGCCTT GCATGTTTAT 8820

TGAAGGATGT TTGCTTTCCG GACGGAAGGA CGGAAAAAGC TCTGAAAAAA AAAAAAAAAA 8880

AAAAAAAA 8888

(2) ANGABEN ZU SEQ ID NO: 42:

(i) SEQUENZKENNZEICHEN:
(A) LÄNGE: 6622 Basenpaare
(B) ART: Nucleotid
(C) STRANGFORM: Einzelstrang
(D) TOPOLOGIE: linear

(ii) ART DES MOLEKÜLS: Genom-DNA

(ix) MERKMAL:
(A) NAME/SCHLÜSSEL: exon
(B) LAGE:1..6622

(xi) SEQUENZBESCHREIBUNG: SEQ ID NO: 42:

GATATCATGG AGATAATTAA AATGATAACC ATCTCGCAAA TAAATAAGTA TTTTACTGTT 60

TTCGTAACAG TTTTGTAATA AAAAAACCTA TAAATATGAA ATTCTTAGTC AACGTTGCCC 120

TTGTTTTTAT GGTCGTATAC ATTTCTTACA TCTATGCGGA TCGATGGGGA TCCGCCCAGG 180

GCCACCTAAG GAGCGGACCC CGCATCTTCG CCGTCTGGAA AGGCCATGTA GGGCAGGACC 240

GGGTGGACTT TGGCCAGACT GAGCCGCACA CGGTGCTTTT CCACGAGCCA GGCAGCTCCT 300

CTGTGTGGGT GGGAGGACGT GGCAAGGTCT ACCTCTTTGA CTTCCCCGAG GGCAAGAACG 360

CATCTGTGCG CACGGTGAAT ATCGGCTCCA CAAAGGGGTC CTGTCTGGAT AAGCGGGACT 420

GCGAGAACTA CATCACTCTC CTGGAGAGGC GGAGTGAGGG GCTGCTGGCC TGTGGCACCA 480

ACGCCCGGCA CCCCAGCTGC TGGAACCTGG TGAATGGCAC TGTGGTGCCA CTTGGCGAGA 540

TGAGAGGCTA TGCCCCCTTC AGCCCGGACG AGAACTCCCT GGTTCTGTTT GAAGGGGACG 600

AGGTGTATTC CACCATCCGG AAGCAGGAAT ACAATGGGAA GATCCCTCGG TTCCGCCGCA 660

TCCGGGGCGA GAGTGAGCTG TACACCAGTG ATACTGTCAT GCAGAACCCA CAGTTCATCA 720

AAGCCACCAT CGTGCACCAA GACCAGGCTT ACGATGACAA GATCTACTAC TTCTTCCGAG 780

AGGACAATCC TGACAAGAAT CCTGAGGCTC CTCTCAATGT GTCCCGTGTG GCCCAGTTGT 840

GCAGGGGGGA CCAGGGTGGG GAAAGTTCAC TGTCAGTCTC CAAGTGGAAC ACTTTTCTGA 900

AAGCCATGCT GGTATGCAGT GATGCTGCCA CCAACAAGAA CTTCAACAGG CTGCAAGACG 960

TCTTCCTGCT CCCTGACCCC AGCGGCCAGT GGAGGGACAC CAGGGTCTAT GGTGTTTTCT 1020

CCAACCCCTG GAACTACTCA GCCGTCTGTG TGTATTCCCT CGGTGACATT GACAAGGTCT 1080

TCCGTACCTC CTCACTCAAG GGCTACCACT CAAGCCTTCC CAACCCGCGG CCTGGCAAGT 1140

GCCTCCCAGA CCAGCAGCCG ATACCCACAG AGACCTTCCA GGTGGCTGAC CGTCACCCAG 1200

AGGTGGCGCA GAGGGTGGAG CCCATGGGGC CTCTGAAGAC GCCATTGTTC CACTCTAAAT 1260

ACCACTACCA GAAAGTGGCC GTTCACCGCA TGCAAGCCAG CCACGGGGAG ACCTTTCATG 1320

TGCTTTACCT AACTACAGAC AGGGGCACTA TCCACAAGGT GGTGGAACCG GGGGAGCAGG 1380

AGCACAGCTT CGCCTTCAAC ATCATGGAGA TCCAGCCCTT CCGCCGCGCG GCTGCCATCC 1440

AGACCATGTC GCTGGATGCT GAGCGGAGGA AGCTGTATGT GAGCTCCCAG TGGGAGGTGA 1500

GCCAGGTGCC CCTGGACCTG TGTGAGGTCT ATGGCGGGGG CTGCCACGGT TGCCTCATGT 1560

CCCGAGACCC CTACTGCGGC TGGGACCAGG GCCGCTGCAT CTCCATCTAC AGCTCCGAAC 1620

GGTCAGTGCT GCAATCCATT AATCCAGCCG AGCCACACAA GGAGTGTCCC AACCCCAAAC 1680

CAGACAAGGC CCCACTGCAG AAGGTTTCCC TGGCCCCAAA CTCTCGCTAC TACCTGAGCT 1740

GCCCCATGGA ATCCCGCCAC GCCACCTACT CATGGCGCCA CAAGGAGAAC GTGGAGCAGA 1800

GCTGCGAACC TGGTCACCAG AGCCCCAACT GCATCCTGTT CATCGAGAAC CTCACGGCGC 1860

AGCAGTACGG CCACTACTTC TGCGAGGCCC AGGAGGGCTC CTACTTCCGC GAGGCTCAGC 1920

ACTGGCAGCT GCTGCCCGAG GACGGCATCA TGGCCGAGCA CCTGCTGGGT CATGCCTGTG 1980

CCCTGGCTGC CTGAATTCGA AGCTTGGAGT CGACTCTGCT GAAGAGGAGG AAATTCTCCT 2040

TGAAGTTTCC CTGGTGTTCA AAGTAAAGGA GTTTGCACCA GACGCACCTC TGTTCACTGG 2100

TCCGGCGTAT TAAAACACGA TACATTGTTA TTAGTACATT TATTAAGCGC TAGATTCTGT 2160

GCGTTGTTGA TTTACAGACA ATTGTTGTAC GTATTTTAAT AATTCATTAA ATTTATAATC 2220

TTTAGGGTGG TATGTTAGAG CGAAAATCAA ATGATTTTCA GCGTCTTTAT ATCTGAATTT 2280

AAATATTAAA TCCTCAATAG ATTTGTAAAA TAGGTTTCGA TTAGTTTCAA ACAAGGGTTG 2340

TTTTTCCGAA CCGATGGCTG GACTATCTAA TGGATTTTCG CTCAACGCCA CAAAACTTGC 2400

CAAATCTTGT AGCAGCAATC TAGCTTTGTC GATATTCGTT TGTGTTTTGT TTTGTAATAA 2460

AGGTTCGACG TCGTTCAAAA TATTATGCGC TTTTGTATTT CTTTCATCAC TGTCGTTAGT 2520

GTACAATTGA CTCGACGTAA ACACGTTAAA TAAAGCCTGG ACATATTTAA CATCGGGCGT 2580

GTTAGCTTTA TTAGGCCGAT TATCGTCGTC GTCCCAACCC TCGTCGTTAG AAGTTGCTTC 2640

CGAAGACGAT TTTGCCATAG CCACACGACG CCTATTAATT GTGTCGGCTA ACACGTCCGC 2700

GATCAAATTT GTAGTTGAGC TTTTTGGAAT TATTTCTGAT TGCGGGCGTT TTTGGGCGGG 2760

TTTCAATCTA ACTGTGCCCG ATTTTAATTC AGACAACACG TTAGAAAGCG ATGGTGCAGG 2820

CGGTGGTAAC ATTTCAGACG GCAAATCTAC TAATGGCGGC GGTGGTGGAG CTGATGATAA 2880

ATCTACCATC GGTGGAGGCG CAGGCGGGGC TGGCGGCGGA GGCGGAGGCG GAGGTGGTGG 2940

CGGTGATGCA GACGGCGGTT TAGGCTCAAA TTGTCTCTTT CAGGCAACAC AGTCGGCACC 3000

TCAACTATTG TACTGGTTTC GGGCGTATGG TGCACTCTCA GTACAATCTG CTCTGATGCC 3060

GCATAGTTAA GCCAGCCCCG ACACCCGCCA ACACCCGCTG ACGCGCCCTG ACGGGCTTGT 3120

CTGCTCCCGG CATCCGCTTA CAGACAAGCT GTGACCGTCT CCGGGAGCTG CATGTGTCAG 3180

AGGTTTTCAC CGTCATCACC GAAACGCGCG AGACGAAAGG GCCTCGTGAT ACGCCTATTT 3240

TTATAGGTTA ATGTCATGAT AATAATGGTT TCTTAGACGT CAGGTGGCAC TTTTCGGGGA 3300

AATGTGCGCG GAACCCCTAT TTGTTTATTT TTCTAAATAC ATTCAAATAT GTATCCGCTC 3360

ATGAGACAAT AACCCTGATA AATGCTTCAA TAATATTGAA AAAGGAAGAG TATGAGTATT 3420

CAACATTTCC GTGTCGCCCT TATTCCCTTT TTTGCGGCAT TTTGCCTTCC TGTTTTTGCT 3480

CACCCAGAAA CGCTGGTGAA AGTAAAAGAT GCTGAAGATC AGTTGGGTGC ACGAGTGGGT 3540

TACATCGAAC TGGATCTCAA CAGCGGTAAG ATCCTTGAGA GTTTTCGCCC CGAAGAACGT 3600

TTTCCAATGA TGAGCACTTT TAAAGTTCTG CTATGTGGCG CGGTATTATC CCGTATTGAC 3660

GCCGGGCAAG AGCAACTCGG TCGCCGCATA CACTATTCTC AGAATGACTT GGTTGAGTAC 3720

TCACCAGTCA CAGAAAAGCA TCTTACGGAT GGCATGACAG TAAGAGAATT ATGCAGTGCT 3780

GCCATAACCA TGAGTGATAA CACTGCGGCC AACTTACTTC TGACAACGAT CGGAGGACCG 3840

AAGGAGCTAA CCGCTTTTTT GCACAACATG GGGGATCATG TAACTCGCCT TGATCGTTGG 3900

GAACCGGAGC TGAATGAAGC CATACCAAAC GACGAGCGTG ACACCACGAT GCCTGTAGCA 3960

ATGGCAACAA CGTTGCGCAA ACTATTAACT GGCGAACTAC TTACTCTAGC TTCCCGGCAA 4020

CAATTAATAG ACTGGATGGA GGCGGATAAA GTTGCAGGAC CACTTCTGCG CTCGGCCCTT 4080

CCGGCTGGCT GGTTTATTGC TGATAAATCT GGAGCCGGTG AGCGTGGGTC TCGCGGTATC 4140

ATTGCAGCAC TGGGGCCAGA TGGTAAGCCC TCCCGTATCG TAGTTATCTA CACGACGGGG 4200

AGTCAGGCAA CTATGGATGA ACGAAATAGA CAGATCGCTG AGATAGGTGC CTCACTGATT 4260

AAGCATTGGT AACTGTCAGA CCAAGTTTAC TCATATATAC TTTAGATTGA TTTAAAACTT 4320

CATTTTTAAT TTAAAAGGAT CTAGGTGAAG ATCCTTTTTG ATAATCTCAT GACCAAAATC 4380

CCTTAACGTG AGTTTTCGTT CCACTGAGCG TCAGACCCCG TAGAAAAGAT CAAAGGATCT 4440

TCTTGAGATC CTTTTTTTCT GCGCGTAATC TGCTGCTTGC AAACAAAAAA ACCACCGCTA 4500

CCAGCGGTGG TTTGTTTGCC GGATCAAGAG CTACCAACTC TTTTTCCGAA GGTAACTGGC 4560

TTCAGCAGAG CGCAGATACC AAATACTGTT CTTCTAGTGT AGCCGTAGTT AGGCCACCAC 4620

TTCAAGAACT CTGTAGCACC GCCTACATAC CTCGCTCTGC TAATCCTGTT ACCAGTGGCT 4680

GCTGCCAGTG GCGATAAGTC GTGTCTTACC GGGTTGGACT CAAGACGATA GTTACCGGAT 4740

AAGGCGCAGC GGTCGGGCTG AACGGGGGGT TCGTGCACAC AGCCCAGCTT GGAGCGAACG 4800

ACCTACACCG AACTGAGATA CCTACAGCGT GAGCTATGAG AAAGCGCCAC GCTTCCCGAA 4860

GGGAGAAAGG CGGACAGGTA TCCGGTAAGC GGCAGGGTCG GAACAGGAGA GCGCACGAGG 4920

GAGCTTCCAG GGGGAAACGC CTGGTATCTT TATAGTCCTG TCGGGTTTCG CCACCTCTGA 4980

CTTGAGCGTC GATTTTTGTG ATGCTCGTCA GGGGGGCGGA GCCTATGGAA AAACGCCAGC 5040

AACGCGGCCT TTTTACGGTT CCTGGCCTTT TGCTGGCCTT TTGCTCACAT GTTCTTTCCT 5100

GCGTTATCCC CTGATTCTGT GGATAACCGT ATTACCGCCT TTGAGTGAGC TGATACCGCT 5160

CGCCGCAGCC GAACGACCGA GCGCAGCGAG TCAGTGAGCG AGGAAGCATC CTGCACCATC 5220

GTCTGCTCAT CCATGACCTG ACCATGCAGA GGATGATGCT CGTGACGGTT AACGCCTCGA 5280

ATCAGCAACG GCTTGCCGTT CAGCAGCAGC AGACCATTTT CAATCCGCAC CTCGCGGAAA 5340

CCGACATCGC AGGCTTCTGC TTCAATCAGC GTGCCGTCGG CGGTGTGCAG TTCAACCACC 5400

GCACGATAGA GATTCGGGAT TTCGGCGCTC CACAGTTTCG GGTTTTCGAC GTTCAGACGT 5460

AGTGTGACGC GATCGGTATA ACCACCACGC TCATCGATAA TTTCACCGCC GAAAGGCGCG 5520

GTGCCGCTGG CGACCTGCGT TTCACCCTGC CATAAAGAAA CTGTTACCCG TAGGTAGTCA 5580

CGCAACTCGC CGCACATCTG AACTTCAGCC TCCAGTACAG CGCGGCTGAA ATCATCATTA 5640

AAGCGAGTGG CAACATGGAA ATCGCTGATT TGTGTAGTCG GTTTATGCAG CAACGAGACG 5700

TCACGGAAAA TGCCGCTCAT CCGCCACATA TCCTGATCTT CCAGATAACT GCCGTCACTC 5760

CAACGCAGCA CCATCACCGC GAGGCGGTTT TCTCCGGCGC GTAAAAATGC GCTCAGGTCA 5820

AATTCAGACG GCAAACGACT GTCCTGGCCG TAACCGACCC AGCGCCCGTT GCACCACAGA 5880

TGAAACGCCG AGTTAACGCC ATCAAAAATA ATTCGCGTCT GGCCTTCCTG TAGCCAGCTT 5940

TCATCAACAT TAAATGTGAG CGAGTAACAA CCCGTCGGAT TCTCCGTGGG AACAAACGGC 6000

GGATTGACCG TAATGGGATA GGTCACGTTG GTGTAGATGG GCGCATCGTA ACCGTGCATC 6060

TGCCAGTTTG AGGGGACGAC GACAGTATCG GCCTCAGGAA GATCGCACTC CAGCCAGCTT 6120

TCCGGCACCG CTTCTGGTGC CGGAAACCAG GCAAAGCGCC ATTCGCCATT CAGGCTGCGC 6180

AACTGTTGGG AAGGGCGATC GGTGCGGGCC TCTTCGCTAT TACGCCAGCT GGCGAAAGGG 6240

GGATGTGCTG CAAGGCGATT AAGTTGGGTA ACGCCAGGGT TTTCCCAGTC ACGACGTTGT 6300

AAAACGACGG GATCTATCAT TTTTAGCAGT GATTCTAATT GCAGCTGCTC TTTGATACAA 6360

CTAATTTTAC GACGACGATG CGAGCTTTTA TTCAACCGAG CGTGCATGTT TGCAATCGTG 6420

CAAGCGTTAT CAATTTTTCA TTATCGTATT GTTGCACATC AACAGGCTGG ACACCACGTT 6480

GAACTCGCCG CAGTTTTGCG GCAAGTTGGA CCCGCCGCGC ATCCAATGCA AACTTTCCGA 6540

CATTCTGTTG CCTACGAACG ATTGATTCTT TGTCCATTGA TCGAAGCGAG TGCCTTCGAC 6600

TTTTTCGTGT CCAGTGTGGC TT 6622

(2) ANGABEN ZU SEQ ID NO: 43:

(i) SEQUENZKENNZEICHEN:
(A) LÄNGE: 31 Basenpaare
(B) ART: Nucleotid
(C) STRANGFORM: Einzelstrang
(D) TOPOLOGIE: linear

(ii) ART DES MOLEKÜLS: Genom-DNA

(ix) MERKMAL:
(A) NAME/SCHLÜSSEL: exon
(B) LAGE:1..31

(xi) SEQUENZBESCHREIBUNG: SEQ ID NO: 43:

CCGGATCCGC CCAGGGCCAC CTAAGGAGCG G 31

(2) ANGABEN ZU SEQ ID NO: 44:

(i) SEQUENZKENNZEICHEN:
(A) LÄNGE: 29 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 44:

CTGAATTCAG GAGCCAGGGC ACAGGCATG 29